# EUROPEAN PATENT APPLICATION

(11) **EP 4 527 846 A1**
(43) Date of publication of application: **26.03.2025**
(21) Application number: 23807327.4
(22) Date of filing: 10.04.2023
(51) Int. Cl.: C07K 1/06, C07K 5/08, C07K 7/06

(54) **TWO-RESIDUE-EXTENDED PEPTIDE AND METHOD FOR PRODUCING SAME**

(30) Priority: 16.05.2022 JP 2022080477
(71) Applicant: NATIONAL UNIVERSITY CORPORATION TOKAI NATIONAL HIGHER EDUCATION AND RESEARCH SYSTEM, Nagoya-shi, Aichi 464-8601 (JP)
(72) Inventor: FUSE, Shinichiro, Nagoya-shi, Aichi 464-8601 (JP); SUGISAWA, Naoto, Nagoya-shi, Aichi 464-8601 (JP)
(74) Representative: Hoffmann Eitle
(86) International application number: PCT/JP2023/014637
(87) International publication number: WO 2023/223714

(57) **Abstract**

Various two-residue-extended peptides can be produced by reacting a first amino acid or peptide represented by the formula: 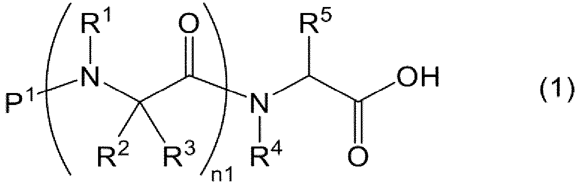 a halogenating agent, a tertiary amine represented by the formula: 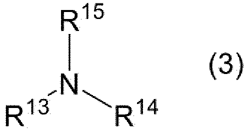 an amino acid N-carboxy anhydride (NCA) represented by the formula: 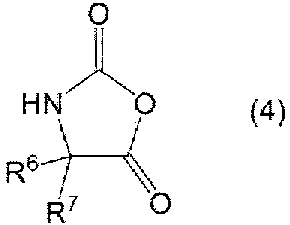 and a second amino acid or peptide represented by the formula:

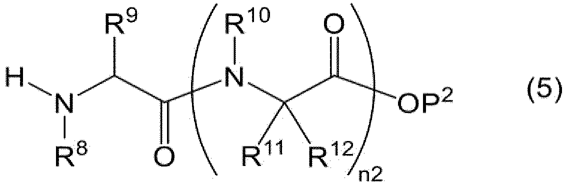

## Description

### Technical Field

The present invention relates to a two-residue-extended peptide and a method for producing the same.

### Background Art

Demand for peptides is increasing for use in pharmaceuticals, foods, cosmetics, and the like. However, conventional peptide synthesis methods require excessive amounts of expensive reactants and generate a large amount of waste derived from the reactants, which poses problems in terms of environmental load and increased production costs. Furthermore, each time a peptide is extended by one residue, a protecting group as large as or larger than the amino acid is used, and an additional deprotection step is required, which results in major problems in terms of a decrease in atom economy and an increase in the number of steps. For this reason, there is a demand for rapid and efficient methods for synthesizing peptides.

One-pot synthesis of tripeptides or oligopeptides is known as a method for reducing the number of steps in peptide synthesis. In particular, Shin et al. have reported one-pot tripeptide synthesis using dehydroamino acid N-carboxy anhydrides (dehydro-NCAs) (see, for example, NPL 1 and NPL 2). However, the methods of NPL 1 and NPL 2 merely improve the yield by using dehydro NCAs, which are more stable than normal amino acid N-carboxy anhydrides (NCAs). These methods cannot extend a wide range of amino acid residues using normal NCAs.

It has also been shown that urethane-protected NCAs are useful for one-pot tripeptide synthesis (see, for example, NPL 3). However, the method of NPL 3 requires the separate preparation of urethane-protected NCAs, and the need to remove the protecting groups remains unchanged. Moreover, there is no mention of epimerization of amino acid residues, which is a problem in ordinary peptide synthesis, especially in peptide synthesis under basic conditions.

On the other hand, there are reports of the one-pot synthesis of oligopeptides without using unstable NCAs (see, for example, NPL 4). However, the method of NPL 4 uses protecting groups and expensive reagents, requires a step of removing impurities derived from the protecting groups and the reagents, and has a long reaction time. For this reason, this method cannot be considered as an excellent peptide synthesis method that can be applied to a wide range of amino acids.

Thus, it was conventionally necessary to deactivate all of the extending amino acids by protection or dehydrogenation. In other words, there is still no method for rapidly obtaining various peptides using unprotected amino acids. Therefore, such a method is required.

### Citation List

### Non-patent Literature

NPL 1: Chem. Lett. 1994, 1909-1910.
NPL 2: Bull. Chem. Soc. Jpn. 1994, 67, 2151-2161.
NPL 3: Tetrahedron Lett. 1995, 36, 807-810.
NPL 4: Org. Biomol. Chem. 2017, 15, 7533-7542.

### Summary of Invention

### Technical Problem

The present invention was made in view of the current state of the prior art described above, and a primary object thereof is to provide a method for producing various two-residue-extended peptides using amino acid N-carboxy anhydrides (NCAs).

### Solution to Problem

The present inventors conducted intensive research to achieve the above object. As a result, they found that by reacting a first amino acid or peptide, a halogenating agent, a tertiary amine, an amino acid N-carboxy anhydride (NCA), and a second amino acid or peptide, the N-terminus of the first amino acid or peptide is extended by two residues, whereby various two-residue-extended peptides can be obtained in a short period of time. Upon further research based on this finding, the present inventors have completed the present invention. That is, the present invention includes the following configurations.

### Item 1.

A method for producing a two-residue-extended peptide represented by formula (6): 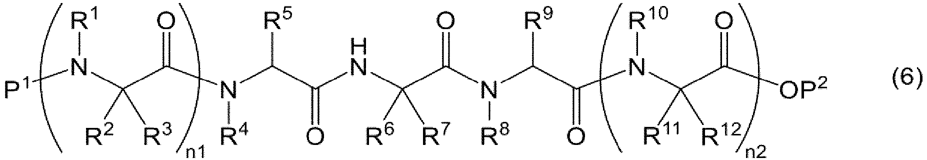 wherein R¹ represents a hydrogen atom or an alkyl group; R⁴, R⁸, and R¹⁰ are the same or different and each represents a hydrogen atom or a methyl group; R², R⁵, R⁶, R⁹, and R¹¹ are the same or different and each represents a side chain of an amino acid residue; R², R⁵, R⁶, R⁹, and R¹¹ each optionally form a ring together with the adjacent nitrogen atom through the carbon atom to which each is attached; R³, R⁷, and R¹² are the same or different and each represents a hydrogen atom or an optionally substituted monovalent organic group; P¹ represents a hydrogen atom, a protecting group for amino groups, a tag, or a solid-phase; P² represents a hydrogen atom, a protecting group for carboxy groups, a tag, or a solid-phase; n1 represents an integer of 0 or more, and when n1 is an integer of 2 or more, n1 R¹s, R²s, and R³s are optionally the same or different; and n2 represents an integer of 0 or more, and when n2 is an integer of 2 or more, n2 R¹⁰s, R¹¹s, and R¹²s are optionally the same or different;
the method comprising a reaction step of reacting a first amino acid or peptide represented by formula (1): 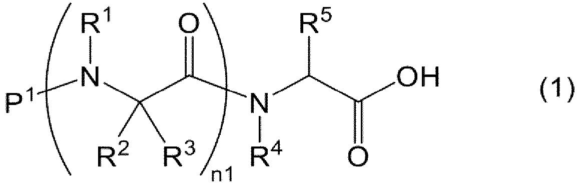
wherein R¹, R², R³, R⁴, R⁵, P¹, and n1 are as defined above,
a halogenating agent, a tertiary amine represented by formula (3) : 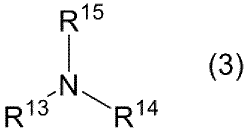
   wherein R¹³, R¹⁴, and R¹⁵ are the same or different and each represents an optionally substituted alkyl group, an optionally substituted aryl group, or an optionally substituted heteroaryl group, or two or more of R¹³, R¹⁴, and R¹⁵ are optionally linked to form a ring optionally having one or more heteroatoms or substituents,
   an amino acid N-carboxy anhydride (NCA) represented by formula (4): 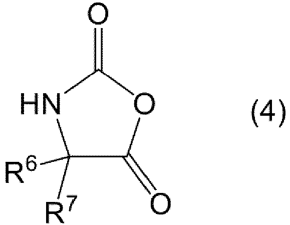
      wherein R⁶ and R⁷ are as defined above, and
      a second amino acid or peptide represented by formula (5): 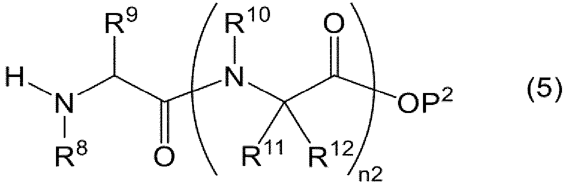 wherein R⁸, R⁹, R¹⁰, R¹¹, R¹², P², and n2 are as defined above.

### Item 2.

The production method according to Item 1, wherein the reaction step comprises:
(A) a reaction step of reacting the first amino acid or peptide represented by formula (1), the halogenating agent, the tertiary amine represented by formula (3), and the amino acid N-carboxy anhydride (NCA) represented by formula (4); and
(B) a reaction step of reacting a reaction mixture obtained in step (A) and the second amino acid or peptide represented by formula (5).

### Item 3.

The production method according to Item 1 or 2, wherein step (A) comprises:
(A1) a reaction step of reacting the first amino acid or peptide represented by formula (1), the halogenating agent, and the tertiary amine represented by formula (3); and
(A2) a reaction step of reacting a reaction mixture obtained in step (A1) and the amino acid N-carboxy anhydride (NCA) represented by formula (4).

Item 3-1. The production method according to any one of Items 1 to 3, wherein R², R⁵, R⁶, R⁹, and R¹¹ are side chains of natural amino acid residues.

Item 3-2. The production method according to any one of Items 1 to 3-1, wherein n1 is an integer of 0 to 100.

Item 3-3. The production method according to any one of Items 1 to 3-2, wherein n2 is an integer of 0 to 100.

### Item 4.

The production method according to any one of Items 1 to 3-3, wherein the halogenating agent is thionyl represented by formula (2): 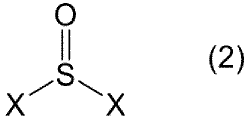 wherein Xs are the same or different and each represents a halogen atom.

### Item 5.

The production method according to any one of Items 1 to 4, wherein the tertiary amine comprises an aliphatic amine and/or a heterocyclic aromatic amine.

### Item 6.

The production method according to any one of Items 1 to 5, wherein the reaction temperature of the reaction step is - 80 to 100°C.

### Item 7.

The production method according to any one of Items 1 to 6, wherein the reaction time of the reaction step is less than 60 minutes.

### Item 8.

The production method according to any one of Items 1 to 7, wherein the reaction step is performed by a flow method.

### Item 9.

The production method according to any one of Items 1 to 8, wherein the reaction step is performed by a micro-flow method.

### Item 10.

An acylated NCA represented by formula (8): 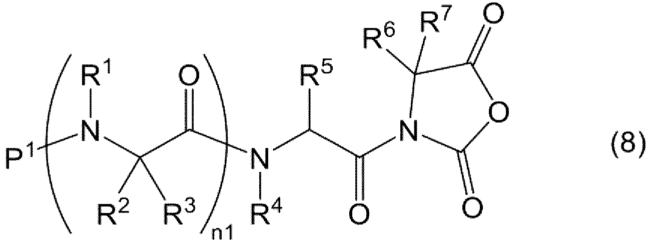 wherein R¹ represents a hydrogen atom or an optionally substituted monovalent organic group; R⁴ represents a hydrogen atom or a methyl group; R², R⁵, and R⁶ are the same or different and each represents a side chain of an amino acid residue; R², R⁵, and R⁶ each optionally form a ring together with the adjacent nitrogen atom through the carbon atom to which each is attached; R³ and R⁷ each represent a hydrogen atom or an optionally substituted monovalent organic group; P¹ represents a hydrogen atom, a protecting group for amino groups, a tag, or a solid-phase; and n1 represents an integer of 0 or more, and when n1 is an integer of 2 or more, n1 R¹s, R²s, and R³s are optionally the same or different.

### Item 11.

A peptide represented by formula (9): 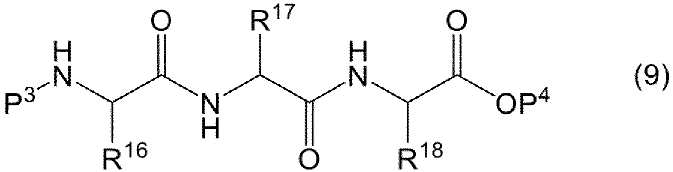 wherein R¹⁶, R¹⁷, and R¹⁸ are the same or different and each represents a side chain of an amino acid residue; R¹⁶, R¹⁷, and R¹⁸ each optionally form a ring together with the adjacent nitrogen atom through the carbon atom to which each is attached; P³ represents a hydrogen atom or a protecting group for amino groups; and P⁴ represents a hydrogen atom or a protecting group for carboxy groups.

### Item 12.

A peptide consisting of an amino acid sequence represented by SEQ ID NO: 1.

### Advantageous Effects of Invention

According to the present invention, by reacting a combination of a first amino acid or peptide, an amino acid N-carboxy anhydride (NCA), a specific halogenating agent, and a specific tertiary amine with a second amino acid or peptide, the N-terminus of the first amino acid or peptide is extended by two residues, whereby various two-residue-extended peptides can be obtained in a short period of time.

### Brief Description of Drawings

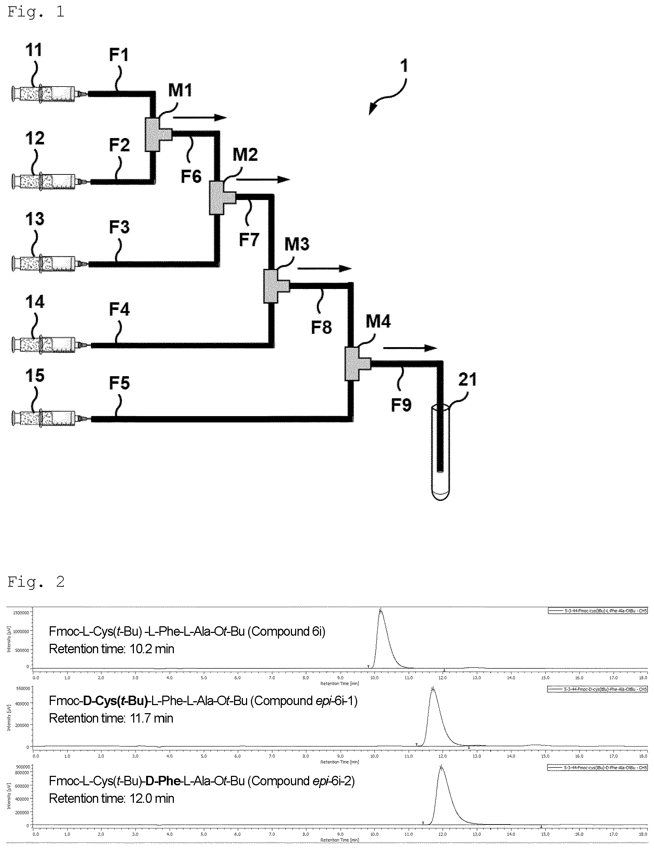

Fig. 1 is a schematic diagram showing the general configuration of a micro-flow reactor.
Fig. 2 is the chromatogram of a tripeptide represented by Fmoc-L-Cys(t-Bu)-L-Phe-L-Ala-Ot-Bu (compound 6i) obtained in Example 6 and its epimers, tripeptides (compounds epi-6i-1 and epi-6i-2) obtained in Example 9 (column: DAICEL CHIRALPAK IB 4.6 mm × 25 cm, solvent: 10% isopropyl alcohol in hexane, flow rate: 1.0 mL/min, detection wavelength: 254 nm).

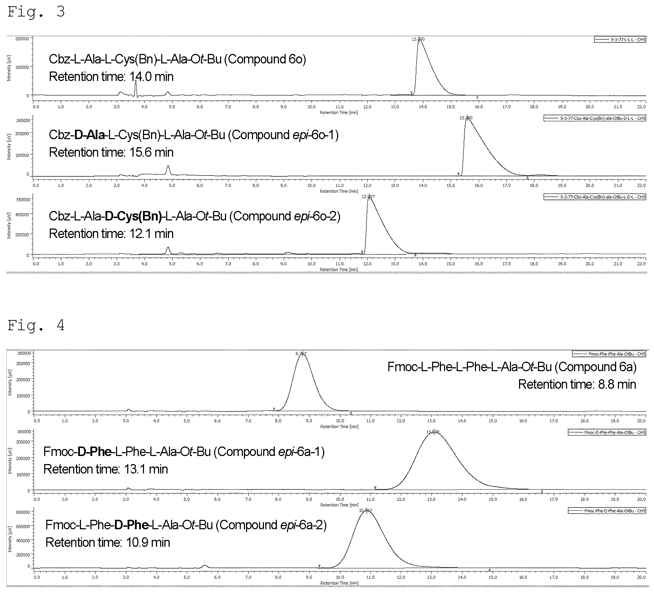

Fig. 3 is the chromatogram of a tripeptide represented by Fmoc-L-Ala-L-Cys(t-Bu)-L-Ala-Ot-Bu (compound 6o) obtained in Example 6 and its epimers, tripeptides (compounds epi-6o-1 and epi-6o-2) obtained in Example 9 (column: DAICEL CHIRALPAK IB 4.6 mm × 25 cm, solvent: 10% isopropyl alcohol in hexane, flow rate: 1.0 mL/min, detection wavelength: 254 nm).
Fig. 4 is the chromatogram of a tripeptide represented by Fmoc-L-Phe-L-Phe-L-Ala-Ot-Bu (compound 6a) obtained in Example 4 and its epimers, tripeptides (compounds epi-6a-1 and epi-6a-2) obtained in Comparative Examples 1 and 2 (column: DAICEL CHIRALPAK IH 4.6 mm × 25 cm, solvent: 10% isopropyl alcohol in hexane, flow rate: 1.0 mL/min, detection wavelength: 254 nm).

### Description of Embodiments

In the present specification, the term "comprise" or "contain" is a concept that encompasses all of the meanings of "comprise," "contain," "consist essentially of" and "consist of."

Further, in the present specification, the numerical range indicated by "A to B" means A or more and B or less.

The method for producing a two-residue-extended peptide of the present invention comprises reacting a first amino acid or peptide, a halogenating agent, a tertiary amine, an amino acid N-carboxy anhydride (NCA), and a second amino acid or peptide.

Moreover, in the method of the present invention, due to the use of an amino acid N-carboxy anhydride (NCA) with an unprotected N-terminus, the deprotection step required each time an amino acid is elongated in normal peptide synthesis can be omitted.

Furthermore, in the method of the present invention, even when an amino acid that is easily epimerized is contained, epimerization of the amino acid is unlikely to occur.

In addition, when a micro-flow method is used in the method of the present invention, by appropriately selecting a halogenating agent and a tertiary amine, it is possible to easily obtain a high-purity target peptide as long as the peptide is crystallizable.

### (1) First Amino Acid or Peptide

The first amino acid or peptide usable in the present invention is a compound represented by formula (1): 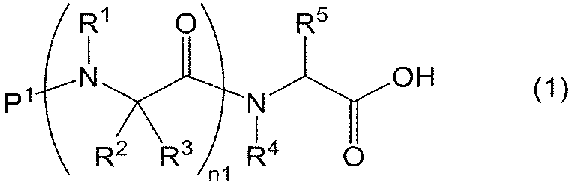 wherein R¹, R², R³, R⁴, R⁵, P¹, and n1 are as defined above.

In formula (1), the group represented by R¹ is a hydrogen atom or an alkyl group.

In formula (1), the alkyl group represented by R¹ is not particularly limited as long as it is a C₁₋₁₀ alkyl group, and examples include C₁₋₁₀ (particularly C₁₋₆) linear alkyl groups, such as methyl, ethyl, n-propyl, n-butyl, and n-pentyl groups; as well as C₃₋₁₀ (particularly C₃₋₆) branched alkyl groups, such as isopropyl, isobutyl, sec-butyl, and tert-butyl (t-Bu) groups; and C₃₋₁₀ (particularly C₅₋₈) cyclic alkyl groups, such as cyclopentyl and cyclohexyl groups.

In formula (1), the groups represented by R² and R⁵ are the same or different, and each represents a side chain of an amino acid residue. In the present specification, the side chain of an amino acid residue refers to a monovalent group obtained by removing a group represented by the following formula from an amino acid having an amino group and a carboxyl group in one molecule:

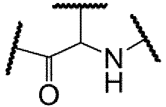

In formula (1), the side chains of the amino acid residues represented by R² and R⁵ are not limited to side chains of amino acid residues derived from α-amino acids, and side chains of amino acid residues derived from β- and γ-amino acids can also be used.

In formula (1), as the side chains of the amino acid residues represented by R² and R⁵, side chains of both natural and synthetic amino acid residues can be used. Examples include side chains of natural amino acid residues, such as glycine (Gly), alanine (Ala), valine (Val), leucine (Leu), isoleucine (Ile), phenylalanine (Phe), serine (Ser), threonine (Thr), lysine (Lys), 5-hydroxylysine (Hyl), arginine (Arg), aspartic acid (Asp), asparagine (Asn), glutamic acid (Glu), glutamine (Gln), cysteine (CySH), cystine (Cyss), cysteic acid (Cya), methionine (Met), tyrosine (Tyr), thyroxine (Thy), proline (Pro), hydroxyproline (Hyp), tryptophan (Trp), histidine (His), β-alanine, N-methyl-β-alanine, sarcosine, γ-aminobutyric acid, kainic acid, and derivatives thereof.

In formula (1), as the side chains of the amino acid residues represented by R² and R⁵, depending on the type of functional group to be protected, side chains of amino acid residues having protecting groups can be used. When the amino acid residue is an amino acid residue having an amino group or a carboxyl group in the side chain, a side chain of an amino acid residue having a protecting group is preferred from the viewpoint of avoiding side reactions. On the other hand, when the amino acid residue consists of an amino acid residue having a functional group other than an amino group and a carboxyl group in the side chain, a side chain of an amino acid residue without a protecting group is preferred from the viewpoint of eliminating the deprotection step, improving atom economy, and reducing waste. According to the production method of the present invention, even if the side chain of the amino acid residue does not have a protecting group, side reactions originating from the side chain are unlikely to occur.

As the protecting group present in the side chain of the amino acid residue, any protecting groups known as protecting groups present in side chains of amino acid residues can be used. Examples include alkyl-based protecting groups, such as tert-butyl (t-Bu), benzyl, allyl, methyl, and triphenylmethyl (Trt) groups; silyl-based protecting groups, such as a tert-butyldimethylsilyl (TBS) group; aryl-based protecting groups, such as p-methoxybenzyl (PMB) and p-methoxyphenyl (PMP) groups; amide-based protecting groups, such as formyl and acetyl (Ac) groups; phthalimide-based protecting groups, such as a phthaloyl (Phth) group; carbamate-based protecting groups, such as benzyloxycarbonyl (Cbz), tert-amyloxycarbonyl (Aoc), 9-fluorenylmethoxycarbonyl (Fmoc), tert-butyloxycarbonyl (Boc), allyloxycarbonyl (Alloc), and 2,2,2-triethoxycarbonyl (Troc) groups; sulfonamide-based protecting groups, such as 3-nitro-2-pyridinesulfenyl (Npys), 2-nitrobenzenesulfonyl (Ns), and (2-trimethylsilyl)-ethanesulfonyl (SES) groups; and the like.

In formula (1), the group represented by R³ is a hydrogen atom or an optionally substituted organic group.

In formula (1), the organic group represented by R³ is preferably a hydrocarbon group, and the hydrocarbon group is, for example, an optionally substituted alkyl group or an optionally substituted aryl group.

In formula (1), the alkyl group represented by R³ is not particularly limited, and examples include C₁₋₁₀ (particularly C₁₋₆) linear alkyl groups, such as methyl, ethyl, n-propyl, n-butyl, and n-pentyl groups; as well as C₃₋₁₀ (particularly C₃₋₆) branched alkyl groups, such as isopropyl, isobutyl, sec-butyl, and tert-butyl groups; and C₃₋₁₀ (particularly C₅₋₈) cyclic alkyl groups, such as cyclopentyl and cyclohexyl groups.

In formula (1), the aryl group represented by R³ is not particularly limited, and any of a monocyclic aryl group, a condensed aryl group, and a polycyclic aryl group can be used. Examples include C₆₋₁₈ (particularly C₆₋₁₄) aryl groups, such as phenyl, hydroxyphenyl, naphthyl, anthracenyl, phenanthrenyl, biphenyl, terphenyl, fluorenyl, indolyl, imidazolyl, pyrenyl, and triphenylenyl groups.

In formula (1), the hydrocarbon group represented by R³ may have substituents. Examples of substituents include, but are not particularly limited to, hydroxyl groups, halogen atoms (e.g., fluorine, chlorine, bromine, and iodine atoms), the above alkyl groups, the above aryl groups, alkoxy groups (e.g., methoxy, ethoxy, and propoxy groups), carboxyl groups, amino groups, and the like. When there are substituents, the number of substituents is not particularly limited, and is preferably 1 to 6, and more preferably 1 to 3.

In formula (1), the substituent present in the hydrocarbon group represented by R³ may have a protecting group. As the protecting group, any protecting groups known as protecting groups for the above substituents can be used. Examples include alkyl-based protecting groups, such as tert-butyl (t-Bu), benzyl, allyl, methyl, and triphenylmethyl (Trt) groups; silyl-based protecting groups, such as a tert-butyldimethylsilyl (TBS) group; aryl-based protecting groups, such as p-methoxybenzyl (PMB) and p-methoxyphenyl (PMP) groups; amide-based protecting groups, such as formyl and acetyl (Ac) groups; phthalimide-based protecting groups, such as a phthaloyl (Phth) group; carbamate-based protecting groups, such as benzyloxycarbonyl (Cbz), tert-amyloxycarbonyl (Aoc), 9-fluorenylmethoxycarbonyl (Fmoc), tert-butyloxycarbonyl (Boc), allyloxycarbonyl (Alloc), and 2,2,2-triethoxycarbonyl (Troc) groups; sulfonamide-based protecting groups, such as 3-nitro-2-pyridinesulfenyl (Npys), 2-nitrobenzenesulfonyl (Ns), and (2-trimethylsilyl)-ethanesulfonyl (SES) groups; and the like. When there are protecting groups, the number of protecting groups is not particularly limited, and is preferably 1 to 6, and more preferably 1 to 3.

In formula (1), the group represented by R⁴ is a hydrogen atom or a methyl group.

In formula (1), the group represented by P¹ is a hydrogen atom, a protecting group for amino groups, a tag, or a solid-phase. From the viewpoint of avoiding side reactions, a protecting group for amino groups is preferred. On the other hand, from the viewpoint of eliminating the deprotection step, improving atom economy, and reducing waste, the group represented by P¹ is preferably a hydrogen atom. On the other hand, when n1 is large in formula (1), that is, when the peptide represented by formula (1) is a long-chain peptide, the group represented by P¹ is preferably a tag or a solid-phase, from the viewpoint of enabling the application of the production method of the present invention.

As the protecting group for amino groups represented by P¹, any protecting groups known as protecting group for amino groups can be used. Examples include alkyl-based protecting groups, such as tert-butyl (t-Bu), benzyl, allyl, methyl, and triphenylmethyl (Trt) groups; silyl-based protecting groups, such as a tert-butyldimethylsilyl (TBS) group; aryl-based protecting groups, such as p-methoxybenzyl (PMB) and p-methoxyphenyl (PMP) groups; amide-based protecting groups, such as formyl and acetyl (Ac) groups; phthalimide-based protecting groups, such as a phthaloyl (Phth) group; carbamate-based protecting groups, such as benzyloxycarbonyl (Cbz), tert-amyloxycarbonyl (Aoc), 9-fluorenylmethoxycarbonyl (Fmoc), tert-butyloxycarbonyl (Boc), allyloxycarbonyl (Alloc), and 2,2,2-triethoxycarbonyl (Troc) groups; sulfonamide-based protecting groups, such as 3-nitro-2-pyridinesulfenyl (Npys), 2-nitrobenzenesulfonyl (Ns), and (2-trimethylsilyl)-ethanesulfonyl (SES) groups; and the like.

As the tag represented by P¹, tags used to improve peptide solubility problems can be widely used. Examples include hydrophobic tags composed of aromatic rings substituted with multiple long-chain alkoxy groups. This allows the direct application of reactions established in peptide solid-phase synthesis methods. Here, the term "solubility" means how easily a molecule dissolves in a solvent.

As the solid-phase represented by P¹, a solid-phase used in a conventional peptide solid-phase synthesis method can be used.

In formula (1), n1 is an integer of 0 or more, and generally an integer of 0 to 100. From the viewpoint of improving the yield and suppressing side reactions, n1 is preferably an integer of 0 to 50, more preferably an integer of 0 to 10, and even more preferably an integer of 0 to 2. When n1 is an integer of 2 or more, n1 R¹s, R²s, and R³s may be the same or different.

The amino acid constituting the first amino acid or peptide represented by formula (1) may be either the L- or D-enantiomer. In general, the most problematic issue in the amidation reaction in peptide synthesis is the epimerization of amino acid residues; however, in the production method of the present invention, the epimerization of amino acid residues can be suppressed.

From the above, specific examples of the first amino acid or peptide represented by formula (1) include: 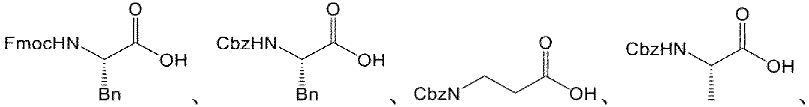 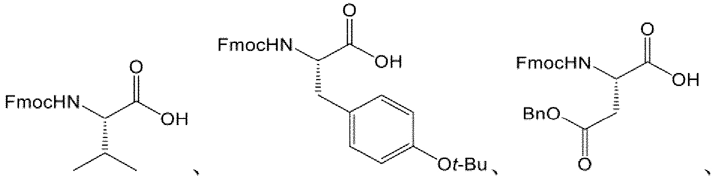 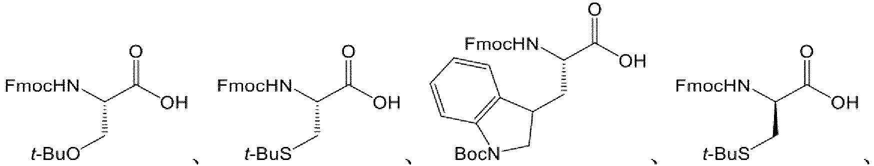 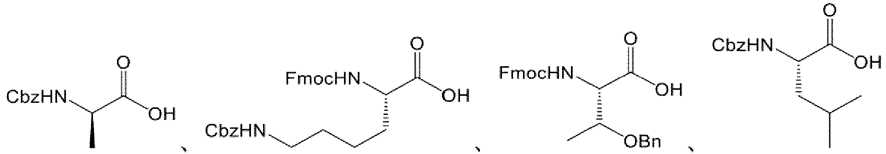 and the like.

### (2) Halogenating Agent

The halogenating agent usable in the present invention is an electrophilic halogenating agent.

Examples of such electrophilic halogenating agents include a phosphorus halide represented by PX₃ (wherein Xs are the same or different and each represents a halogen atom; the same applies hereinafter in the present specification) or PX₅, thionyl represented by SOX₂, sulfuryl represented by SO₂X₂, oxalyl halide represented by (COX)₂, and the like. Among these, thionyl is preferred from the viewpoint of high reactivity, cost, and the like.

The thionyl can also be represented by formula (2): 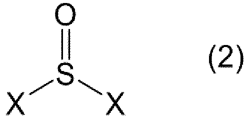 wherein Xs are the same or different and each represents a halogen atom.

The groups represented by Xs are the same or different and each is a halogen atom.

Examples of the halogen atom represented by X include fluorine, chlorine, bromine, and iodine atoms. From the viewpoint of economy, safety, and the like, a chlorine atom or a bromine atom is preferred, and a chlorine atom is more preferred.

From the above, specific examples of the thionyl represented by formula (2) used in the present invention include: 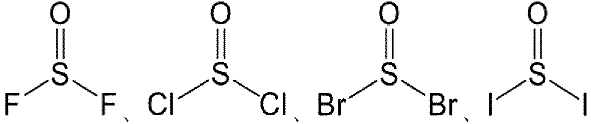 and the like.

The halogenating agents usable in the present invention can be used singly or in combination of two or more.

The amount of the halogenating agent used in the present invention is not particularly limited; however, from the viewpoint of suppressing the formation of impurities and improving the yield, the amount of the halogenating agent is generally preferably, for example, about 0.1 to 10 mol, more preferably about 0.2 to 5 mol, and even more preferably about 0.5 to 3 mol, per mol of the first amino acid or peptide represented by formula (1). When two or more halogenating agents usable in the present invention are used in combination, it is preferable to adjust the total amount thereof to be within the above range.

### (3) Tertiary Amine

The tertiary amine usable in the present invention is a compound represented by formula (3): 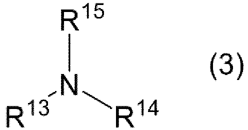 wherein R¹³, R¹⁴, and R¹⁵ are the same or different and each represents an optionally substituted alkyl group or an optionally substituted aryl group, or two or more of R¹³, R¹⁴, and R¹⁵ are optionally linked to form a ring optionally having one or more heteroatoms or substituents.

In formula (3), the alkyl groups represented by R¹³, R¹⁴, and R¹⁵ are not particularly limited, and examples include C₁₋₁₀ (particularly C₁₋₆) linear alkyl groups, such as methyl, ethyl, n-propyl, n-butyl, and n-pentyl groups; as well as C₃₋₁₀ (particularly C₃₋₆) branched alkyl groups, such as isopropyl, isobutyl, sec-butyl, and tert-butyl groups.

In formula (3), the alkyl groups represented by R¹³, R¹⁴, and R¹⁵ are not particularly limited, and any of a monocyclic aryl group, a condensed aryl group, and a polycyclic aryl group can be used. Examples include C₆₋₁₈ (particularly C₆₋₁₄) aryl groups, such as phenyl, naphthyl, anthracenyl, phenanthrenyl, biphenyl, terphenyl, fluorenyl, pyrenyl, and triphenylenyl groups.

In formula (3), the heteroaryl groups represented by R¹³, R¹⁴, and R¹⁵ are not particularly limited, and either of a monocyclic heteroaryl group and a polycyclic heteroaryl group can be used. Examples include pyrrolidyl, pyrrolyl, tetrahydrothienyl, thienyl, oxolanyl, furanyl, imidazolyl, N-methylimidazolyl, pyrazolyl, thiazolyl, oxazolyl, piperidyl, pyridyl, N,N-dimethyl-4-aminopyridyl, pyrazyl, indolyl, isoindolyl, benzimidazolyl, quinolyl, isoquinolyl, and quinoxalyl groups.

In formula (3), the alkyl group, aryl group, and heteroaryl group represented by R¹³, R¹⁴, and R¹⁵ may have substituents. Examples of substituents include, but are not particularly limited to, hydroxyl groups, halogen atoms (e.g., fluorine, chlorine, bromine, and iodine atoms), the above alkyl groups, the above aryl groups, alkoxy groups, amino groups, and the like. When there are substituents, the number of substituents is not particularly limited, and is preferably 1 to 6, and more preferably 1 to 3.

Two or more of R¹³, R¹⁴, and R¹⁵ may be bonded to form a ring optionally having one or more heteroatoms or substituents. The ring is not particularly limited, and examples include an aziridine ring, azirine ring, diaziridine ring, diazirine ring, azetidine ring, azeto ring, diazeto ring, pyrrolidine ring, pyrrole ring, imidazolidine ring, imidazole ring, pyridine ring, piperidine ring, piperazine ring, morpholine ring, and the like.

From the above, specific examples of the tertiary amine used in the present invention include: 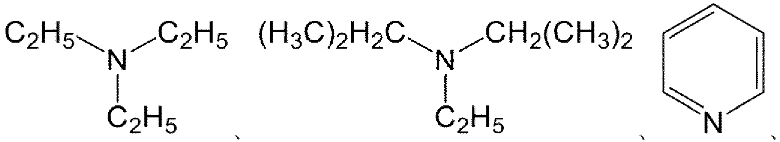 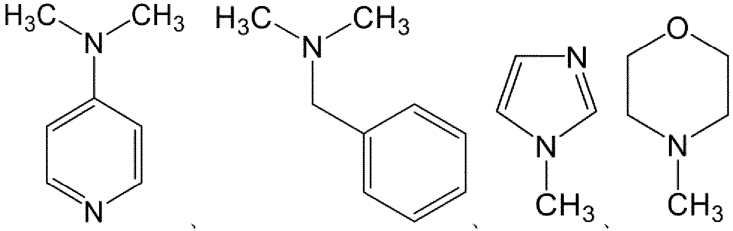 and the like.

The tertiary amines represented by formula (3) can be used singly or in combination of two or more. The tertiary amine may contain, for example, an aliphatic amine, such as triethylamine (Et₃N), diisopropylethylamine (i-Pr₂NEt), dimethylbenzylamine (Me₂NBn), or N-methylmorpholine (NMM), and/or a heterocyclic aromatic amine, such as pyridine, 4-dimethylaminopyridine (DMAP), or N-methylimidazole (NMI).

In particular, from the viewpoint of improving the yield and the like, the tertiary amine represented by formula (3) preferably contains an aliphatic amine and a heterocyclic aromatic amine; more preferably at least one aliphatic amine selected from the group consisting of triethylamine(Et₃N), diisopropylethylamine (i-Pr₂NEt), and dimethylbenzylamine (Me₂NBn), and at least one heterocyclic aromatic amine selected from the group consisting of pyridine, 4-dimethylaminopyridine (DMAP), and N-methylimidazole (NMI); and particularly preferably at least one aliphatic amine selected from the group consisting of triethylamine (Et₃N), diisopropylethylamine (i-Pr₂NEt), and dimethylbenzylamine (Me₂NBn), and N-methylimidazole (NMI) as a heterocyclic aromatic amine.

The amount of tertiary amine used is not particularly limited; however, from the viewpoint of yield improvement, epimerization suppression, economic efficiency, and the like, it is generally preferably, for example, about 0.1 to 10 mol, more preferably about 0.2 to 5 mol, and even more preferably about 0.5 to 3 mol, per mol of the first amino acid. When two or more tertiary amines represented by formula (3) are used in combination, it is preferable to adjust the total amount thereof to be within the above range. While the yield of the target two-residue-extended peptide increases with increasing amounts of tertiary amine, the use of an excess of tertiary amine hardly increases the yield of the target two-residue-extended peptide. Accordingly, it is preferable to appropriately adjust the amount while also taking into account economic efficiency.

### (4) Amino Acid N-Carboxy Anhydride (NCA)

The amino acid N-carboxy anhydride (NCA) usable in the present invention is a compound represented by formula (4): 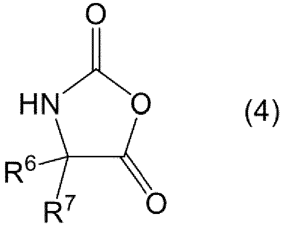 wherein R⁶ and R⁷ are as defined above.

In formula (4), the group represented by R⁶ is a side chain of an amino acid residue. In formula (4), the side chain of the amino acid residue represented by R⁶ is not limited to side chains of amino acid residues derived from α-amino acids, and side chains of amino acid residues derived from β- and γ-amino acids can also be used.

In formula (4), as the side chain of the amino acid residue represented by R⁶, side chains of both natural and synthetic amino acid residues can be used. Examples include side chains of natural amino acid residues, such as glycine (Gly), alanine (Ala), valine (Val), leucine (Leu), isoleucine (Ile), phenylalanine (Phe), serine (Ser), threonine (Thr), lysine (Lys), 5-hydroxylysine (Hyl), arginine (Arg), aspartic acid (Asp), asparagine (Asn), glutamic acid (Glu), glutamine (Gln), cysteine (CySH), cystine (Cyss), cysteic acid (Cya), methionine (Met), tyrosine (Tyr), thyroxine (Thy), proline (Pro), hydroxyproline (Hyp), tryptophan (Trp), histidine (His), β-alanine, N-methyl-β-alanine, sarcosine, γ-aminobutyric acid, kainic acid, and derivatives thereof.

In formula (4), as the side chain of the amino acid residue represented by R⁶, depending on the type of functional group to be protected, side chains of amino acid residues having protecting groups can be used. When the amino acid residue is an amino acid residue having an amino group or a carboxyl group in the side chain, a side chain of an amino acid residue having a protecting group is preferred from the viewpoint of avoiding side reactions. On the other hand, when the amino acid residue consists of an amino acid residue having a functional group other than an amino group and a carboxyl group in the side chain, a side chain of an amino acid residue without a protecting group is preferred from the viewpoint of eliminating the deprotection step, improving atom economy, and reducing waste. According to the production method of the present invention, even if the amino acid residue does not have a protecting group, side reactions originating from the side chain are unlikely to occur.

As the protecting group present in the side chain of the amino acid residue, any protecting groups known as protecting groups present in side chains of amino acid residues can be used. Examples include alkyl-based protecting groups, such as tert-butyl (t-Bu), benzyl, allyl, methyl, and triphenylmethyl (Trt) groups; silyl-based protecting groups, such as a tert-butyldimethylsilyl (TBS) group; aryl-based protecting groups, such as p-methoxybenzyl (PMB) and p-methoxyphenyl (PMP) groups; amide-based protecting groups, such as formyl and acetyl (Ac) groups; phthalimide-based protecting groups, such as a phthaloyl (Phth) group; carbamate-based protecting groups, such as benzyloxycarbonyl (Cbz), tert-amyloxycarbonyl (Aoc), 9-fluorenylmethoxycarbonyl (Fmoc), tert-butyloxycarbonyl (Boc), allyloxycarbonyl (Alloc), and 2,2,2-triethoxycarbonyl (Troc) groups; sulfonamide-based protecting groups, such as 3-nitro-2-pyridinesulfenyl (Npys), 2-nitrobenzenesulfonyl (Ns), and (2-trimethylsilyl)-ethanesulfonyl (SES) groups; and the like.

The amino acid N-carboxy anhydride (NCA) represented by formula (4) may be either the L- or D-enantiomer. In general, the problematic issue in the amidation reaction in peptide synthesis is the epimerization of amino acid residues; however, in the production method of the present invention, the epimerization of amino acid residues can be suppressed.

In formula (4), the group represented by R⁷ is a hydrogen atom or an optionally substituted organic group.

In formula (4), the organic group represented by R⁷ is preferably a hydrocarbon group, and the hydrocarbon group is, for example, an optionally substituted alkyl group or an optionally substituted aryl group.

In formula (4), the alkyl group represented by R⁷ is not particularly limited, and examples include C₁₋₁₀ (particularly C₁₋₆) linear alkyl groups, such as methyl, ethyl, n-propyl, n-butyl, and n-pentyl groups; as well as C₃₋₁₀ (particularly C₃₋₆) branched alkyl groups, such as isopropyl, isobutyl, sec-butyl, and tert-butyl groups; and C₃₋₁₀ (particularly C₅₋₈) cyclic alkyl groups, such as cyclopentyl and cyclohexyl groups.

In formula (4), the aryl group represented by R⁷ is not particularly limited, and any of a monocyclic aryl group, a condensed aryl group, and a polycyclic aryl group can be used. Examples include C₆₋₁₈ (particularly C₆₋₁₄) aryl groups, such as phenyl, hydroxyphenyl, indolyl, imidazolyl, naphthyl, anthracenyl, phenanthrenyl, biphenyl, terphenyl, fluorenyl, pyrenyl, and triphenylenyl groups.

In formula (4), the hydrocarbon group represented by R⁷ may have substituents. Examples of substituents include, but are not particularly limited to, hydroxyl groups, halogen atoms (e.g., fluorine, chlorine, bromine, and iodine atoms), the above alkyl groups, the above aryl groups, alkoxy groups (e.g., methoxy, ethoxy, and propoxy groups), carboxyl groups, amino groups, and the like. When there are substituents, the number of substituents is not particularly limited, and is preferably 1 to 6, and more preferably 1 to 3.

In formula (4), the substituent present in the hydrocarbon group represented by R⁷ may have a protecting group. As the protecting group, any protecting groups known as protecting groups for the above substituents can be used. Examples include alkyl-based protecting groups, such as tert-butyl (t-Bu), benzyl, allyl, methyl, and triphenylmethyl (Trt) groups; silyl-based protecting groups, such as a tert-butyldimethylsilyl (TBS) group; aryl-based protecting groups, such as p-methoxybenzyl (PMB) and p-methoxyphenyl (PMP) groups; amide-based protecting groups, such as formyl and acetyl (Ac) groups; phthalimide-based protecting groups, such as a phthaloyl (Phth) group; carbamate-based protecting groups, such as benzyloxycarbonyl (Cbz), tert-amyloxycarbonyl (Aoc), 9-fluorenylmethoxycarbonyl (Fmoc), tert-butyloxycarbonyl (Boc), allyloxycarbonyl (Alloc), and 2,2,2-triethoxycarbonyl (Troc) groups; sulfonamide-based protecting groups, such as 3-nitro-2-pyridinesulfenyl (Npys), 2-nitrobenzenesulfonyl (Ns), and (2-trimethylsilyl)-ethanesulfonyl (SES) groups; and the like. When there are protecting groups, the number of protecting groups is not particularly limited, and is preferably 1 to 6, and more preferably 1 to 3.

From the above, specific examples of the amino acid N-carboxy anhydride (NCA) represented by formula (4) include: 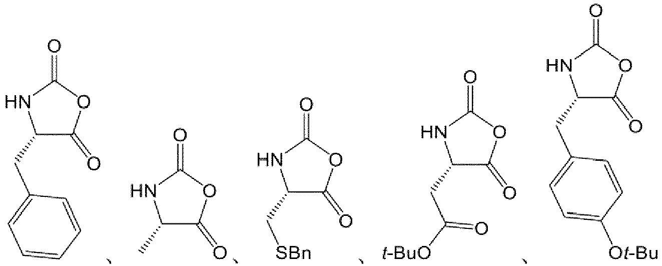 and the like.

The amount of NCA used is not particularly limited; however, from the viewpoint of yield improvement, epimerization suppression, economic efficiency, and the like, it is generally preferably, for example, about 0.1 to 10 mol, more preferably about 0.2 to 5 mol, and even more preferably about 0.5 to 3 mol, per mol of the first amino acid. When two or more NCAs represented by formula (4) are used in combination, it is preferable to adjust the total amount thereof to be within the above range.

### (5) Second Amino Acid or Peptide

The second amino acid or peptide usable in the present invention is a compound represented by formula (5): 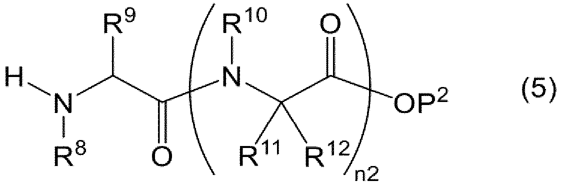 wherein R⁸, R⁹, R¹⁰, R¹¹, R¹², P², and n2 are as defined above.

In formula (5), the groups represented by R⁸ and R¹⁰ are the same or different and each represents a hydrogen atom or a methyl group.

In formula (5), the groups represented by R⁹ and R¹¹ are the same or different and each represents a side chain of an amino acid residue. In formula (5), the side chains of the amino acid residues represented by R⁹ and R¹¹ are not limited to side chains of amino acid residues derived from α-amino acids, and side chains of amino acid residues derived from β- and γ-amino acids can also be used.

In formula (5), as the side chains of the amino acid residues represented by R⁹ and R¹¹, side chains of both natural and synthetic amino acid residues can be used. Examples include side chains of natural amino acid residues, such as glycine (Gly), alanine (Ala), valine (Val), leucine (Leu), isoleucine (Ile), phenylalanine (Phe), serine (Ser), threonine (Thr), lysine (Lys), 5-hydroxylysine (Hyl), arginine (Arg), aspartic acid (Asp), asparagine (Asn), glutamic acid (Glu), glutamine (Gln), cysteine (CySH), cystine (Cyss), cysteic acid (Cya), methionine (Met), tyrosine (Tyr), thyroxine (Thy), proline (Pro), hydroxyproline (Hyp), tryptophan (Trp), histidine (His), β-alanine, N-methyl-β-alanine, sarcosine, γ-aminobutyric acid, kainic acid, and derivatives thereof.

In formula (5), as the side chains of the amino acid residues represented by R⁹ and R¹¹, depending on the type of functional group to be protected, side chains of amino acid residues having protecting groups can be used. When the amino acid residue is an amino acid residue having an amino group or a carboxyl group in the side chain, a side chain of an amino acid residue having a protecting group is preferred from the viewpoint of avoiding side reactions. On the other hand, when the amino acid residue consists of an amino acid residue having a functional group other than an amino group and a carboxyl group in the side chain, a side chain of an amino acid residue without a protecting group is preferred from the viewpoint of eliminating the deprotection step, improving atom economy, and reducing waste. According to the method of the present invention, even if the side chain of the amino acid residue does not have a protecting group, side reactions originating from the side chain are unlikely to occur.

As the protecting group present in the amino acid residue, any protecting groups known as protecting groups present in side chains of amino acid residues can be used. Examples include alkyl-based protecting groups, such as tert-butyl (t-Bu), benzyl, allyl, methyl, and triphenylmethyl (Trt) groups; silyl-based protecting groups, such as a tert-butyldimethylsilyl (TBS) group; aryl-based protecting groups, such as p-methoxybenzyl (PMB) and p-methoxyphenyl (PMP) groups; amide-based protecting groups, such as formyl and acetyl (Ac) groups; phthalimide-based protecting groups, such as a phthaloyl (Phth) group; carbamate-based protecting groups, such as benzyloxycarbonyl (Cbz), tert-amyloxycarbonyl (Aoc), 9-fluorenylmethoxycarbonyl (Fmoc), tert-butyloxycarbonyl (Boc), allyloxycarbonyl (Alloc), and 2,2,2-triethoxycarbonyl (Troc) groups; sulfonamide-based protecting groups, such as 3-nitro-2-pyridinesulfenyl (Npys), 2-nitrobenzenesulfonyl (Ns), and (2-trimethylsilyl)-ethanesulfonyl (SES) groups; and the like.

In formula (5), the group represented by R¹² is a hydrogen atom or an optionally substituted organic group.

In formula (5), the organic group represented by R¹² is preferably a hydrocarbon group, and the hydrocarbon group is, for example, an optionally substituted alkyl group or an optionally substituted aryl group.

In formula (5), the alkyl group represented by R¹² is not particularly limited, and examples include C₁₋₁₀ (particularly C₁₋₆) linear alkyl groups, such as methyl, ethyl, n-propyl, n-butyl, and n-pentyl groups; as well as C₃₋₁₀ (particularly C₃₋₆) branched alkyl groups, such as isopropyl, isobutyl, sec-butyl, and tert-butyl groups; and C₃₋₁₀ (particularly C₅₋₈) cyclic alkyl groups, such as cyclopentyl and cyclohexyl groups.

In formula (5), the aryl group represented by R¹² is not particularly limited, and any of a monocyclic aryl group, a condensed aryl group, and a polycyclic aryl group can be used. Examples include C₆₋₁₈ (particularly C₆₋₁₄) aryl groups, such as phenyl, hydroxyphenyl, indolyl, imidazolyl, naphthyl, anthracenyl, phenanthrenyl, biphenyl, terphenyl, fluorenyl, pyrenyl, and triphenylenyl groups.

In formula (5), the hydrocarbon group represented by R¹² may have substituents. Examples of substituents include, but are not particularly limited to, hydroxyl groups, halogen atoms (e.g., fluorine, chlorine, bromine, and iodine atoms), the above alkyl groups, the above aryl groups, alkoxy groups, carboxyl groups, amino groups, and the like. When there are substituents, the number of substituents is not particularly limited, and is preferably 1 to 6, and more preferably 1 to 3.

In formula (5), the substituent present in the hydrocarbon group represented by R¹² may have a protecting group. As the protecting group, any protecting groups known as protecting groups for the above substituents can be used. Examples include alkyl-based protecting groups, such as tert-butyl (t-Bu), benzyl, allyl, methyl, and triphenylmethyl (Trt) groups; silyl-based protecting groups, such as a tert-butyldimethylsilyl (TBS) group; aryl-based protecting groups, such as p-methoxybenzyl (PMB) and p-methoxyphenyl (PMP) groups; amide-based protecting groups, such as formyl and acetyl (Ac) groups; phthalimide-based protecting groups, such as a phthaloyl (Phth) group; carbamate-based protecting groups, such as benzyloxycarbonyl (Cbz), tert-amyloxycarbonyl (Aoc), 9-fluorenylmethoxycarbonyl (Fmoc), tert-butyloxycarbonyl (Boc), allyloxycarbonyl (Alloc), and 2,2,2-triethoxycarbonyl (Troc) groups; sulfonamide-based protecting groups, such as 3-nitro-2-pyridinesulfenyl (Npys), 2-nitrobenzenesulfonyl (Ns), and (2-trimethylsilyl)-ethanesulfonyl (SES) groups; and the like. When there are protecting groups, the number of protecting groups is not particularly limited, and is preferably 1 to 6, and more preferably 1 to 3.

In formula (5), the group represented by P² is a hydrogen atom, a protecting group for carboxy groups, a tag, or a solid-phase. From the viewpoint of avoiding side reactions, a protecting group for carboxy groups is preferred. On the other hand, from the viewpoint of eliminating the deprotection step, improving atom economy, and reducing waste, the group represented by P² is preferably a hydrogen atom. On the other hand, when n2 is large in formula (5), that is, when the peptide represented by formula (5) is a long-chain peptide, from the viewpoint of enabling the application of the production method of the present invention, the group represented by P² is preferably a tag or a solid-phase.

As the protecting group for carboxy groups represented by P², any protecting groups known as protecting groups for carboxy groups can be used. Examples include alkyl-based protecting groups, such as tert-butyl (t-Bu), benzyl, allyl, methyl, and triphenylmethyl (Trt) groups; silyl-based protecting groups, such as a tert-butyldimethylsilyl (TBS) group; aryl-based protecting groups, such as p-methoxybenzyl (PMB) and p-methoxyphenyl (PMP) groups; amide-based protecting groups, such as formyl and acetyl (Ac) groups; phthalimide-based protecting groups, such as a phthaloyl (Phth) group; carbamate-based protecting groups, such as benzyloxycarbonyl (Cbz), tert-amyloxycarbonyl (Aoc), 9-fluorenylmethoxycarbonyl (Fmoc), tert-butyloxycarbonyl (Boc), allyloxycarbonyl (Alloc), and 2,2,2-triethoxycarbonyl (Troc) groups; sulfonamide-based protecting groups, such as 3-nitro-2-pyridinesulfenyl (Npys), 2-nitrobenzenesulfonyl (Ns), and (2-trimethylsilyl)-ethanesulfonyl (SES) groups; and the like.

As the tag represented by P², tags for improving peptide solubility problems can be used. Examples include hydrophobic tags composed of aromatic rings substituted with multiple long-chain alkoxy groups. This allows the direct application of reactions established in peptide solid-phase synthesis methods. Here, the term "solubility" means how easily a molecule dissolves in a solvent.

As the solid-phase represented by P², a solid-phase used in a conventional peptide solid-phase synthesis method can be used.

In formula (5), n2 is an integer of 0 or more, and generally an integer of 0 to 100. From the viewpoint of improving the yield and suppressing side reactions, n2 is preferably an integer of 0 to 50, more preferably an integer of 0 to 10, and even more preferably an integer of 0 to 2. When n2 is an integer of 2 or more, n2 R¹⁰s, R¹¹s, and R¹²s may be the same or different.

The amino acids constituting the second amino acid or peptide represented by formula (5) each may be either the L- or D-enantiomer.

The peptide represented by formula (5) can also be a two-residue-extended peptide obtained by the production method of the present invention or a similar production method. By doing so, specifically, by repeating the production method of the present invention or a similar production method, oligopeptides or polypeptides of desired lengths can be obtained efficiently in a short period of time.

From the above, specific examples of the second amino acid or peptide represented by formula (5) include: 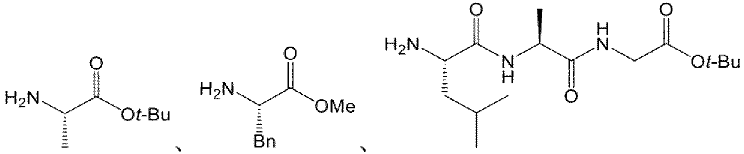 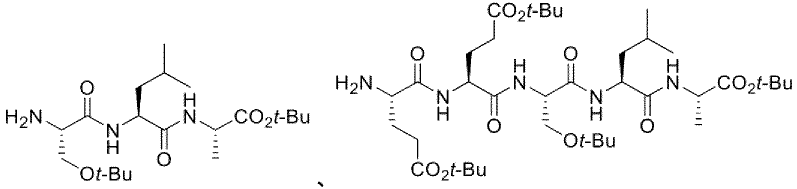 and the like.

The amount of second amino acid or peptide used is not particularly limited; however, from the viewpoint of yield improvement, epimerization suppression, economic efficiency, and the like, it is generally preferably, for example, about 0.1 to 10 mol, more preferably about 0.2 to 5 mol, and even more preferably about 0.5 to 3 mol, per mol of the first amino acid. When two or more second amino acids or peptides represented by formula (5) are used in combination, it is preferable to adjust the total amount thereof to be within the above range.

### 6. Two-Residue-Extended Peptide

The two-residue-extended peptide, which is the target product of the present invention, is a compound represented by formula (6):

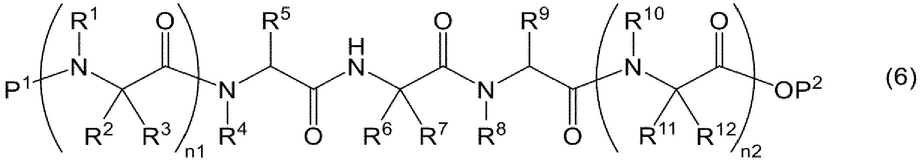

wherein R¹, R², R³, R⁴, R⁵, R⁶, R⁷, R⁸, R⁹, R¹⁰, R¹¹, R¹², P¹, P², n1, and n2 are as defined above.

Among compounds obtained by the production method of the present invention or a similar production method, those represented by the following formulas: 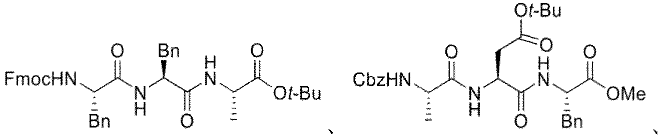 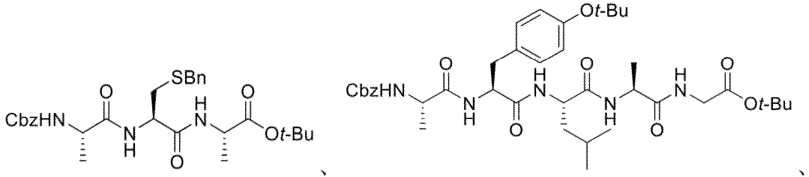 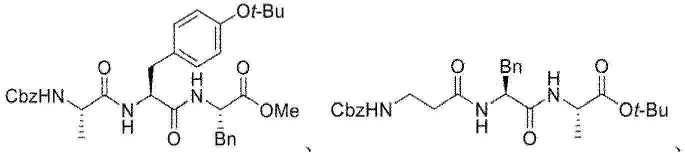 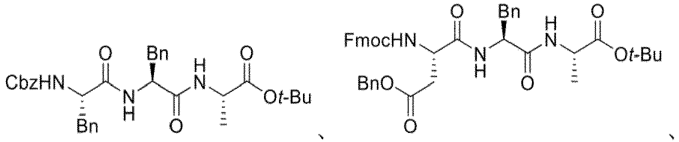 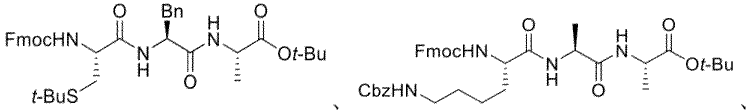 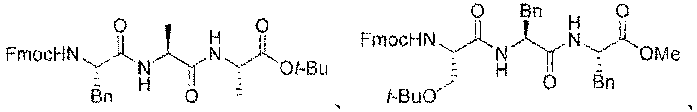 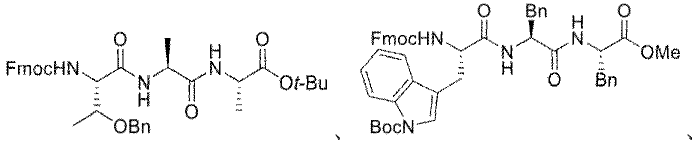 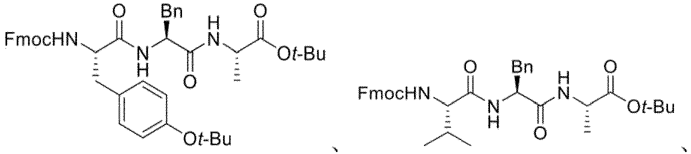 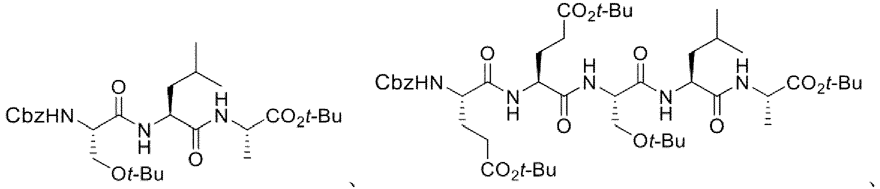 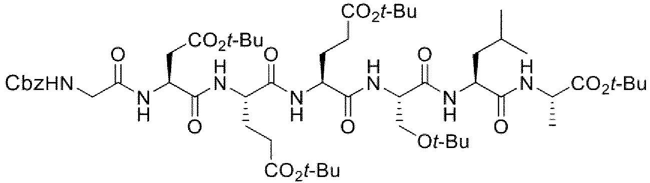 are novel compounds that have not been described in any documents.

### 7. Other Conditions

The solvent used in the reaction step of the present invention is not particularly limited, and any organic solvent that can dissolve the substrate, water, or a mixture thereof can be used. From the viewpoint of the solubility of the substrate, it is preferable to contain an aprotic polar solvent.

The aprotic polar solvent used in the reaction step of the present invention is not particularly limited, and examples include halogen-based solvents, such as dichloromethane and chloroform; ketone-based solvents, such as acetone and methyl ethyl ketone; ether-based solvents, such as 1,4-dioxane, tetrahydrofuran, and diethyl ether; amide-based solvents, such as N,N-dimethylformamide and N,N-diethylformamide; sulfoxide-based solvents, such as dimethyl sulfoxide; nitrile-based solvents, such as acetonitrile; and mixtures thereof. Among these, from the viewpoint of substrate solubility, low reactivity, ease of removal, and the like, preferred are halogen-based solvents and nitrile-based solvents, and more preferred are dichloromethane and acetonitrile. These solvents can be used singly or in combination of two or more.

In particular, as the solvent for dissolving peptides that are difficult to dissolve in organic solvents alone, it is preferable to use a mixed solvent of water and an aprotic polar solvent, and it is more preferable to use a mixed solvent of water and acetonitrile, water and 1,4-dioxane, or water and tetrahydrofuran. The mixing ratio of the mixed solvent is, by volume, preferably about 10:90 to 90:10, more preferably about 75:25 to 25:75, and even more preferably 50:50.

In the present invention, in addition to the above components, additives can be used as appropriate within the scope that does not impair the effects of the present invention. Examples of such additives include hydroxybenzotriazole or its related products, metal salts, and the like.

The reaction temperature in the reaction step of the present invention is preferably -80 to 100°C, more preferably 0 to 80°C, and even more preferably 0 to 60°C, from the viewpoint of suppressing epimerization, shortening the reaction time, and the like. The reaction step of the present invention includes reaction steps (A), (A1), (A2), and (B) described below. The reaction time can be the time required for two amide bond formation reactions to proceed, and from the viewpoint of epimerization suppression and the like, the reaction time is preferably 0.01 seconds to 12 hours, more preferably 1 second to 6 hours, and even more preferably 10 seconds to 1 hour. Preferably, when the reaction step of the present invention is carried out by a micro-flow method, the reaction time can be less than 10 minutes, more preferably less than 5 minutes, and even more preferably less than 1 minute.

### 8. Method for Producing Two-Residue-Extended Peptide

The two-residue-extended peptide, which is the target product of the present invention, can be produced by reacting an amino acid or peptide represented by formula (1), a halogenating agent, a tertiary amine represented by formula (3), an amino acid N-carboxy anhydride (NCA) represented by formula (4), and an amino acid or peptide represented by formula (5). The reaction is not particularly limited, and all components can be added simultaneously or sequentially.

In the production method of the present invention, the first amino acid or peptide represented by formula (1), the halogenating agent, and the tertiary amine represented by formula (3) can be reacted to produce an acid halide represented by formula (7): 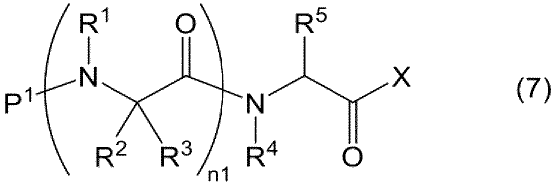 wherein R¹, R², R³, R⁴, R⁵, P¹, n1, and X are as defined above. Since the acid halide is unstable, in order to prevent side reactions, such as epimerization and self-polymerization, it is preferable to precisely control the reaction temperature and reaction time of this reaction.

In the production method of the present invention, the acid halide represented by formula (7) and the amino acid N-carboxy anhydride (NCA) represented by formula (4) can be reacted to produce an acylated NCA represented by formula (8): 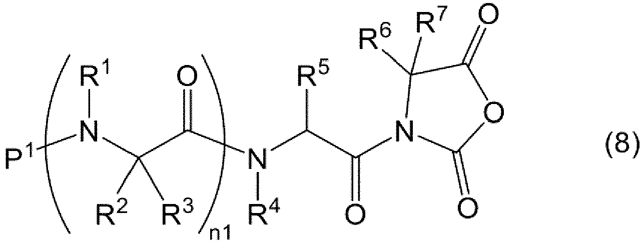 wherein R¹, R², R³, R⁴, R⁵, R⁶, R⁷, P¹, and n1 are as defined above. Since the acylated NCA is unstable, in order to prevent side reactions, such as epimerization and self-polymerization, it is preferable to precisely control the reaction temperature and reaction time of this reaction.

Conventionally, it has been considered that acylated NCAs derived from normal amino acid N-carboxy anhydrides (NCAs), not dehydro amino acid N-carboxy anhydrides (dehydro NCAs), are unstable and therefore impossible to synthesize (J. Synth. Org. Chem. Jpn. 1989, 47(9), 782-794). In contrast, in the production method of the present invention, acylated NCAs can be synthesized from normal NCAs and used for peptide synthesis. Furthermore, the production method of the present invention also makes it possible to isolate the acylated NCAs, which may be produced as intermediates. In the production method of the present invention, the acylated NCA represented by formula (8) and the second amino acid or peptide represented by formula (5) can be reacted to produce the two-residue-extended peptide.

Thus, in the production method of the present invention, since highly reactive reaction intermediates can be produced sequentially in a short period of time, it is easy to efficiently obtain two-residue-extended peptides and to suppress the epimerization of each amino acid residue.

Since highly reactive reaction intermediates can be produced more efficiently in this way, the production method of the present invention preferably employs the following:
(A) a reaction step of reacting the first amino acid or peptide represented by formula (1), the halogenating agent, the tertiary amine represented by formula (3), and the amino acid N-carboxy anhydride (NCA) represented by formula (4), and
(B) a reaction step of reacting a reaction mixture obtained in step (A), and the second amino acid or peptide represented by formula (5).
It is also preferable that step (A) comprises:
(A1) a reaction step of reacting the first amino acid or peptide represented by formula (1), the halogenating agent, and the tertiary amine represented by formula (3), and
(A2) a reaction step of reacting a reaction mixture obtained in step (A1) and the amino acid N-carboxy anhydride (NCA) represented by formula (4).

The reaction step of the present invention can be carried out by either a batch method or a flow method. From the viewpoint of high safety, ease of scale-up, and the like, the flow method is more preferred, and a micro-flow method is even more preferred from the viewpoint of being able to precisely control the reaction temperature and reaction time.

In the present specification, the batch method refers to a batch operation, specifically, a series of operations in which substances to be reacted are charged at once into a reaction vessel or reactor, reacted, and then removed after reaching equilibrium or a certain reaction rate.

In the present specification, the flow method refers to a continuous operation, specifically, an operation in which raw materials and substances required for processing are continuously fed into and discharged from a device at a certain rate (flow rate).

In the present specification, the micro-flow method refers to a flow method (continuous operation) using a micro-flow reactor.

In particular, the reaction step of the present invention can be carried out using a micro-flow reactor. The micro-flow reactor comprises, for example, a channel for transporting a fluid containing raw materials or intermediates to be used in the reaction, a pump for feeding the fluid into the channel, and a mixer for mixing the fluid.

The configuration of the micro-flow reactor and the method for producing the two-residue-extended peptide of the present invention using the same are described below with reference to Fig. 1. However, it goes without saying that the production method of the present invention is not limited to the embodiment of Fig. 1.

Fig. 1 is a schematic diagram showing the general configuration of a micro-flow reactor 1. The micro-flow reactor 1 may comprise a syringe pump 11 that accommodates a first liquid, a syringe pump 12 that accommodates a second liquid, a syringe pump 13 that accommodates a third liquid, a syringe pump 14 that accommodates a fourth liquid, and a syringe pump 15 that accommodates a fifth liquid.

As an example, the first liquid can contain a first amino acid or peptide, and a tertiary amine, the second liquid can contain a halogenating agent, the third liquid can contain an amino acid N-carboxy anhydride (NCA), the fourth liquid can contain a tertiary amine, and the fifth liquid can contain a second amino acid or peptide. As another example, in the above example, the first liquid can contain a first amino acid or peptide, and the second liquid can contain a halogenating agent and a tertiary amine; however, the present invention is not limited to either embodiment.

The micro-flow reactor 1 may comprise channels F1 to F9 for transporting fluids. The inner diameter of the channel may be, for example, 0.1 to 50 mm, or may be 0.3 to 1 mm. The micro-flow reactor 1 may comprise mixers M1 to M4 for mixing fluids. The mixers are not particularly limited, and examples include V-shaped mixers, T-shaped mixers, and the like in which the mixing of fluids is achieved by the inflow of the fluids. In such mixers, the entrainment flow occurring in the channel increases the interfacial area between two liquids, making it easy to mix two or more reaction liquids in a few milliseconds. The inner diameter of the channel inside the mixer may be, for example, 0.1 to 50 mm, or may be 0.2 to 1 mm.

The inner diameter of the channel can be the diameter of the inner part of the channel (the part through which the fluid passes) in the cross-section of the channel in a direction perpendicular to the longitudinal direction of the channel. If the shape of the inner part of the channel is not a perfect circle, the inner diameter of the channel can be a diameter calculated by converting the shape of the inner part of the channel into a perfect circle based on the area.

The syringe pumps 11 to 15 and the channels F1 to F9 may be formed, for example, from resins such as plastics and elastomers, glass materials, metals, ceramics, or the like.

By operating the syringe pump 11, the first liquid can move in the channel F1 and flow into the mixer M1. By operating the syringe pump 12, the second liquid can move in the channel F2 and flow into the mixer M1. Then, the first liquid and the second liquid can be mixed by the mixer M1 to form a first mixed liquid, which can be sent to the channel F6. On the other hand, by operating the syringe pump 13, the third liquid moves in the channel F3 and flows into the mixer M2, where it is mixed with the first mixed liquid to form a second mixed liquid, which can then be sent to the channel F7. Similarly, by operating the syringe pump 14, the fourth liquid moves in the channel F4 and flows into the mixer M3, where it is mixed with the second mixed liquid to form a third mixed liquid, which can then be sent to the channel F8; and by operating the syringe pump 15, the fifth liquid moves in the channel F5 and flows into the mixer M4, where it is mixed with the third mixed liquid to form a fourth mixed liquid, which can then be sent to the channel F9. The fourth mixed liquid can be stored, for example, in a test tube 21, or can be subjected to isolation and purification steps and in-line analysis.

The micro-flow reactor 1 makes it easy to increase the area for heat exchange per volume of the reaction solution. In addition, the reaction time is easily controlled by the flow rate and the length of the channel. This makes it easier to precisely control the reaction solution, thereby minimizing the progression of undesired side reactions and improving the yield of the target product.

As described above, the method for producing the two-residue-extended peptide of the present invention can be carried out by a liquid-phase method, and therefore can be easily scaled up.

Regarding the use of the micro-flow reactor, in the case of synthesizing the acid halide represented by formula (7), for example, the first liquid may contain the first amino acid or peptide represented by formula (1) and the tertiary amine represented by formula (3), and the second liquid may contain a halogenating agent. Alternatively, the first liquid may contain the first amino acid or peptide represented by formula (1), and the second liquid may contain a halogenating agent and the tertiary amine represented by formula (3). From the viewpoint of suppressing side reactions, it is preferable that the first liquid contains the first amino acid or peptide represented by formula (1) and the tertiary amine represented by formula (3), and that the second liquid contains a halogenating agent.

Regarding the use of the micro-flow reactor, in the case of synthesizing the acylated NCA represented by formula (8), in addition to the first mixed liquid containing the acid halide represented by formula (7) and the third liquid containing the amino acid N-carboxy anhydride (NCA) represented by formula (4), for example, in addition to the first or second liquid, the fourth liquid may contain the same tertiary amine represented by formula (3) as that in the first or second liquid, or the fourth liquid may contain a tertiary amine represented by formula (3) different from that in the first or second liquid. From the viewpoint of efficiently activating the acid halide represented by formula (7) and the amino acid N-carboxy anhydride (NCA) represented by formula (4), it is preferable that the fourth liquid contains a second tertiary amine represented by formula (3) different from the first tertiary amine contained in the first or second liquid.

The acid halide represented by formula (7) may be synthesized by a method other than the micro-flow method; however, it is preferable to use an acid halide synthesized by the micro-flow method, from the viewpoint of improving the reaction efficiency and suppressing side reactions.

The amino acid N-carboxy anhydride (NCA) represented by formula (4) may be synthesized by a method other than the micro-flow method; however, it is preferable to use an amino acid N-carboxy anhydride (NCA) synthesized by the micro-flow method, from the viewpoint of improving the reaction efficiency and suppressing side reactions. The present inventors have also reported the micro-flow synthesis of α- and β-amino acid N-carboxy anhydrides (α- and β-NCAs) (see, for example, Angew. Chem. Int. Ed., 2018, 57(35), 11389-11393, and Chem. Asian J., 2020, 15(1), 79-84).

The method for producing the two-residue-extended peptide of the present invention is not limited to being carried out using a micro-flow reactor. For example, a batch vessel having a small volume and capable of achieving a high stirring speed may also be used. The volume of the mixing portion of the batch vessel may be 1 to 100 mL, or may be 5 to 50 mL.

After the reaction using the micro-flow reactor is completed, the two-residue-extended peptide, which is the target compound, can be obtained through the usual isolation and purification steps, if necessary.

In addition, while conventional peptide synthesis requires complicated work for isolation and purification steps, the present invention uses reactants that produce fewer impurities after the reaction and are easy to remove, making it easy to obtain two-residue-extended peptides with a high yield. Therefore, these steps can be omitted or simplified, and the peptides can be directly subjected to crystallization and analysis.

### Examples

Examples and Comparative Examples are provided below to further clarify the features of the present invention; however, the present invention is not limited to the following Examples.

In order to simplify the description of the specification, the following abbreviations may be used in the Examples, as well as in the tables in the Examples. Abbreviations for substituents are as follows. Cbz: a benzyloxycarbonyl group, Boc: a tert-butoxycarbonyl group, Fmoc: a 9-fluorenylmethyloxycarbonyl group, NMI: N-methylimidazole, NMM: N-methylmorpholine, Me₂NBn: dimethylbenzylamine, Et₃N: triethylamine, i-Pr₂NEt: N,N-diisopropylethylamine, DMAP:4-dimethylaminopyridine, Bn: a benzyl group, Me: a methyl group, t-Bu: a tert-butyl group, Phe: phenylalanine, Ala: alanine, β-Ala: β-alanine, Val: valine, Tyr: tyrosine, Asp: aspartic acid, Ser: serine, Cys: cysteine, Trp: tryptophan, Lys: lysine, Thr: threonine.

### Example 1

### Examination of Range of Tertiary Amines

Acylated NCAs were synthesized using the various tertiary amines shown in Table 1 by the following method (Table 1). Unstable acylated NCAs were converted to stable amides, and the yield of the acylated NCAs was indirectly evaluated from the yield of the amides. In order to simplify the examination of the tertiary amines, stable pivaloyl chloride was used in place of unstable acid chlorides of amino acids; however, it goes without saying that it is not easy to use pivaloyl chloride as acid chlorides of amino acids from the viewpoint of side reactions, such as self-polymerization and dimerization.

A dichloromethane solution (CH₂Cl₂; flow rate: 1.20 mL/min) of an amino acid N-carboxy anhydride represented by L-Phe-NCA (compound 3a; 0.300 M, 1.00 molar equivalent) and pivaloyl chloride (0.300 M, 1.00 molar equivalent), and a dichloromethane solution (flow rate: 2.00 mL/min) of a tertiary amine (0.180 + 0.180 M, 1.00 + 1.00 molar equivalent) were introduced into a T-shaped mixer at 20°C using a syringe pump. The resulting mixture was introduced into a reaction tube 1 (inner diameter: 0.800 mm, length: 1061 mm, volume: 533 µL, reaction time: 10 sec) at the same temperature. Next, the resulting mixture and a dichloromethane solution (flow rate: 1.20 mL/min) of isopropylamine (3.00 M, 10.0 molar equivalent) were introduced into a T-shaped mixer at 20°C using a syringe pump. The resulting mixture was introduced into a reaction tube 2 (inner diameter: 0.800 mm, length: 1459 mm, volume: 733 µL, reaction time: 10 sec) at the same temperature. After reaching a steady state over 60 seconds, the reacted mixture was poured into 1M hydrochloric acid (1.5 mL) and dichloromethane (6 mL) at room temperature for 25 seconds. The aqueous layer was extracted with dichloromethane, and the organic layer was washed with saturated brine, dried over magnesium sulfate, filtered, and concentrated under vacuum at room temperature. The yield ratio of the resulting (S)-N-isopropyl-3-phenyl-2-pivalamide propanamide (compound S1) was determined by HPLC-UV analysis using a calibration curve (column: COSMOSIL5C₁₈-AR-II 4.6 mm I.D. × 150 mm, solvent: acetonitrile + 0.1% formic acid/H₂O + 0.1% formic acid (0-20 min: 30 to 100%, 20-25 min: 100%, 25-27 min: 30%, 27-30 min: 30%), flow rate: 1.0 mL/min, detection wavelength: 254 nm, temperature: 40°C, retention time: 8.7 min).

Since the desired compounds S1 can be obtained with various tertiary amines, it can be understood that the desired acylated NCAs are produced (Examples 1-1 to 1-7). Among them, compounds S1 were obtained with a high yield when two kinds of tertiary amines were combined (Examples 1-4 to 1-7). In particular, when N,N-diisopropylethylamine and N-methylimidazole were used as tertiary amines, compound S1 could be obtained quantitatively (>99%), and the reaction results could be analyzed quickly without the need for purification by silica gel column chromatography (Example 1-7).

**Table 1**

| 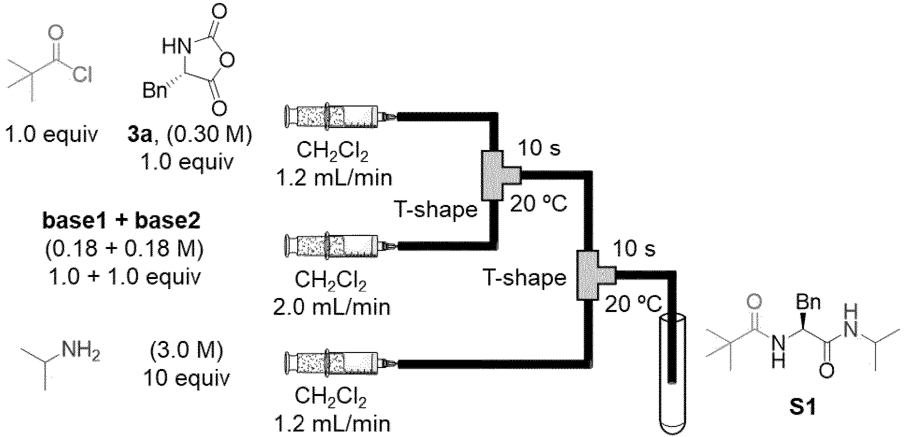 | | |
|---|---|---|
| Example | Tertiary amine (p*K*aH)^{e} | Yield (%) |
| Example 1-1 | NMI (7.0) | 35 |
| Example 1-2 | NMM (7.4) | 2 |
| Example 1-3 | Et₃N (10.9) | 8 |
| Example 1-4 | *i*-Pr₂NEt/pyridine (5.2) | 10 |
| Example 1-5 | *i*-Pr₂NEt/DMAP (9.7) | 99 |
| Example 1-6 | Me₂NBn/NMI | 89 |
| Example 1-7 | *i*-Pr₂NEt/NMI | >99 (>99)^{b} |

| | | |
|---|---|---|
| ^{a}pKa **of conjugate acid in water. ^{b}Isolated yield.** | | |

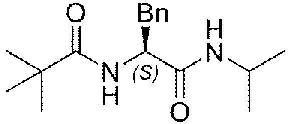

(S)-N-isopropyl-3-phenyl-2-pivalamide propanamide (compound S1): White solid, mp 168-171°C, IR (neat): 3285, 2969, 1633, 1556, 1216 cm⁻¹; [α]²³_{D} = -17.3 (c 2.05, CHCl₃); ¹H NMR (400 MHz, CDCl₃): δ 7.30-7.20 (m, 5H), 6.45 (d, J = 6.4 Hz, 1H), 5.82 (d, J = 7.2 Hz, 1H), 4.57 (ddd, J = 6.4, 6.4, 6.4 Hz, 1H), 4.00-3.91 (m, 1H), 3.10 (dd, J = 6.4, 13.2 Hz, 1H), 2.97 (dd, J = 6.4, 13.2 Hz, 1H), 1.15 (s, 9H), 1.04 (d, J = 6.4 Hz, 3H), 0.98 (d, J = 6.4 Hz, 3H) ppm; ¹³C NMR (100 MHz, CDCl₃): δ 178.4, 170.1, 137.0, 129.5, 128.6, 127.0, 54.5, 41.5, 38.9, 38.8, 27.5, 22.6, 22.5 ppm; HRMS (ESI): calcd for [C₁₇H₂₆N₂O₂+H]⁺ 291.2067, found 291.2066 and [C₁₇H₂₆N₂O₂+Na]⁺ 313.1886, found 313.1885.

### Example 2

### Examination of Amounts of Tertiary Amine and Halogenating Agent Used, and Reaction Time

Next, acid chlorides of amino acids were synthesized by the following method (Table 2). Unstable acid chlorides of amino acids were converted to stable amides, and the yield of the acid chlorides of amino acids was indirectly evaluated from the yield of the amides. The effects of the amounts of the tertiary amine and halogenating agent used, as well as the reaction time, on the yield of the target product were evaluated.

A solution (flow rate: 1.20 mL/min) of an N-terminal protected amino acid represented by Fmoc-L-Phe-OH (compound 1a; 0.300 M, 1.00 molar equivalent) and a tertiary amine (X molar equivalents), and a solution (flow rate: 2.00 mL/min) of thionyl chloride (SOCl₂; X molar equivalents) were each introduced into a T-shaped mixer at 20°C using a syringe pump. The resulting mixture was introduced into a reaction tube 1 (inner diameter: 0.800 mm, reaction time: Y sec) at the same temperature. Next, the resulting mixture and a solution (flow rate: 1.20 mL/min) of isopropylamine (3.00 M, 10.0 molar equivalents) were introduced into a T-shaped mixer at 20°C using a syringe pump. The resulting mixture was introduced into a reaction tube 2 (inner diameter: 0.800 mm, length: 1459 mm, volume: 733 µL, reaction time: 10 sec) at the same temperature. After reaching a steady state over 60 seconds, the reacted mixture was poured into 1M hydrochloric acid (1.5 mL) and dichloromethane (6 mL) at room temperature for 25 seconds. The aqueous layer was extracted with dichloromethane, and the organic layer was washed with saturated brine, dried over magnesium sulfate, filtered, and concentrated under vacuum at room temperature. The yield of (9H-fluoren-9-yl)methyl (S)-(1-(isopropylamino)-1-oxo-3-phenylpropan-2-yl)carbamate (compound S2) was determined by HPLC-UV analysis using a calibration curve (column: COSMOSIL5C₁₈-AR-II 4.6 mm I.D. × 150 mm, solvent: acetonitrile + 0.1% formic acid/H₂O + 0.1% formic acid (0-20 min: 30 to 100%, 20-25 min: 100%, 25-27 min: 30%, 27-30 min: 30%), flow rate: 1.0 mL/min, detection wavelength: 254 nm, temperature: 40°C, retention time: 14.3 min).

Desired compounds S2 can be obtained under the conditions of Examples 2-1 to 2-8, and it can be understood that the desired acid chlorides of amino acids are produced. In particular, compound S2 was obtained with an extremely high isolated yield when using 1.2 molar equivalents of thionyl chloride for a reaction time of 10 seconds (Example 2-5). Therefore, the conditions of Example 2-5 were used in the subsequent Examples.

**Table 2**

| 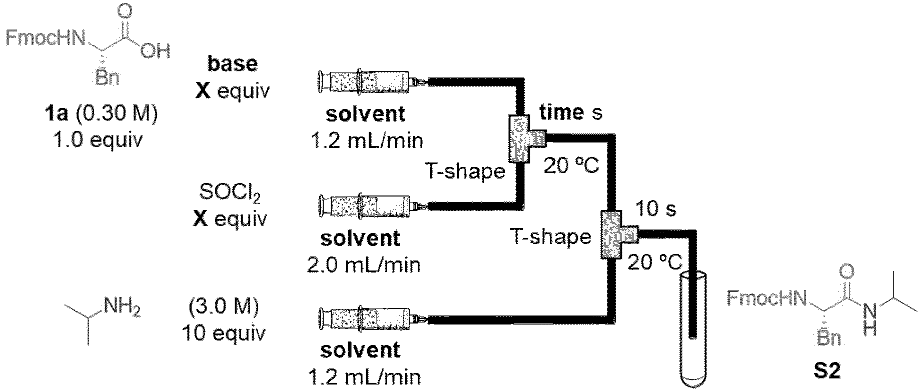 | | | | | |
|---|---|---|---|---|---|
| Example | Base | X (molar equivalents) | Solvent | Time (sec) | Yield (%) |
| Example 2-1 | NMI | 1.2 | CH₂Cl₂ | 10 | 98 |
| Example 2-2 | NMM | 1.2 | CH₂Cl₂ | 10 | >99 |
| Example 2-3 | Et₃N | 1.2 | CH₂Cl₂ | 10 | >99 |
| Example 2-4 | *i*-Pr₂NEt | 1.0 | CH₂Cl₂ | 10 | 96 |
| Example 2-5 | *i*-Pr₂NEt | 1.2 | CH₂Cl₂ | 10 | >99 (98)^{a} |
| Example 2-6 | *i*-Pr₂NEt | 1.2 | CH₃CN | 10 | 97 |
| Example 2-7 | *i*-Pr₂NEt | 1.2 | CH₂Cl₂ | 5 | >99 |
| Example 2-8 | *i*-Pr₂NEt | 1.2 | CH₂Cl₂ | 1 | 96 |

| | | | | | |
|---|---|---|---|---|---|
| ^{a} **Isolated** yield. | | | | | |

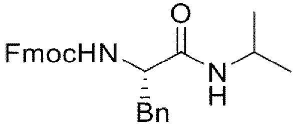

(9H-fluoren-9-yl)methyl (S)-(1-(isopropylamino)-1-oxo-3-phenylpropan-2-yl)carbamate (compound S2):
Chromatographic conditions: CH₂Cl₂ : MeOH = 98 : 2 to 80 : 20.63.3 mg, 0.15 mmol, 98%.

White solid, mp 188-191°C, IR (neat): 3295, 1697, 1651, 1541, 1261, 757, 740 cm⁻¹; [α]²⁴_{D} = -0.95 (c 2.05, CHCl₃); ¹H NMR (400 MHz, CDCl₃): δ 7.76 (d, J = 7.6 Hz, 2H), 7.56 (dd, J = 7.6 Hz, 2H), 7.40 (dd, J = 7.6, 7.6 Hz, 2H), 7.33-7.21 (m, 7H), 5.05 (d, J = 6.0 Hz, 1H), 5.25 (brs, 1H), 4.45-4.28 (m, 3H), 4.20 (t, J = 6.8 Hz, 1H), 4.00-3.91 (m, 1H), 3.16-3.14 (m, 1H), 2.97-2.92 (m, 1H), 1.03 (d, J = 6.4 Hz, 3H), 0.92 (d, J = 6.4 Hz, 3H) ppm; ¹³C NMR (100 MHz, CDCl₃): δ169.7, 156.0, 143.9, 141.4, 136.8, 129.5, 128.8, 127.9, 127.21, 127.17, 125.1, 120.1, 67.1, 56.7, 47.3, 41.6, 39.4, 22.6, 22.5 ppm; HRMS (ESI): calcd for [C₂₇H₂₈N₂O₃+H]⁺ 429.2173, found 429.2171 and [C₂₇H₂₈N₂O₃+Na]⁺ 451.1992, found 451.1992.

### Example 3

### Synthesis and Isolation of Intermediate

Next, based on the results of Examples 1 and 2, an acylated NCA was synthesized starting from the first amino acid and isolated by the following method.

A dichloromethane solution (flow rate: 1.20 mL/min) of an N-terminal protected amino acid represented by Fmoc-L-Phe-OH (compound 1a; 0.300 M, 1.00 molar equivalent) and N,N-diisopropylethylamine (0.360 M, 1.2 molar equivalents), and a dichloromethane solution (flow rate: 2.00 mL/min) of thionyl chloride (0.216 M, 1.20 molar equivalents) were introduced into a T-shaped mixer at 20°C using a syringe pump. The resulting mixture was introduced into a reaction tube 1 (inner diameter: 0.800 mm, length: 1061 mm, volume: 533 µL, reaction time: 10 sec) at the same temperature. Next, the resulting mixture and a dichloromethane solution (flow rate: 1.20 mL/min) of an amino acid N-carboxy anhydride represented by L-Phe-NCA (compound 3a; 0.300 M, 1.00 molar equivalent) were introduced into a T-shaped mixer at 20°C using a syringe pump. The resulting mixture was introduced into a reaction tube 2 (inner diameter: 0.800 mm, length: 72.9 mm, volume: 37 µL, reaction time: 0.50 sec) at the same temperature. The resulting mixture and a dichloromethane solution (flow rate: 2.00 mL/min) of N,N-diisopropylethylamine (0.216 M, 1.2 molar equivalents) and N-methylimidazole (0.180 M, 1.00 molar equivalent) were injected into a T-shaped mixer at 20°C using a syringe pump. The resulting mixture was introduced into a reaction tube 3 (inner diameter: 0.800 mm, length: 2122 mm, volume: 1067 µL, reaction time: 10 sec) at the same temperature. After reaching a steady state over 90 seconds, the reacted mixture was poured into 1M hydrochloric acid (1.5 mL) and dichloromethane (8 mL) at room temperature for 60 seconds. The aqueous layer was extracted with dichloromethane, and the organic layer was washed with saturated brine, dried over magnesium sulfate, filtered, and concentrated under vacuum at room temperature. The residue was purified by recrystallization from dichloromethane and hexane.

The yield of the resulting acylated NCA represented by Fmoc-L-Phe-L-Phe-NCA (compound 4a) was 189.6 mg (0.34 mmol), and the yield was 94%. The acylated NCA, which was previously considered impossible to synthesize, was successfully obtained with a high yield and high purity.

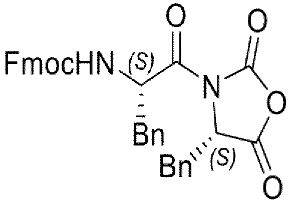

### Fmoc-L-Phe-L-Phe-NCA (compound 4a)

Colorless amorphous solid, ¹H NMR (400 MHz, CDCl₃): δ7.77-7.75 (m, 2H), 7.54 (d, J = 7.2 Hz, 2H), 7.42-7.38 (m, 2H), 7.31-7.25 (m, 8H), 7.21 (d, J = 7.2 Hz, 2H), 7.09-7.08 (m, 2H), 5.52 (ddd, J = 5.2, 8.0 Hz, 1H), 5.30 (d, J = 8.0 Hz, 1H), 4.97 (d, J = 2.8 Hz, 1H), 4.40 (d, J = 6.8 Hz, 2H), 4.21 (t, J = 6.8 Hz, 1H), 3.54 (dd, J = 5.2, 14.0 Hz, 1H), 3.27 (d, J = 2.8, 14.0 Hz, 1H), 3.20 (dd, J = 5.2, 14.0 Hz, 1H), 2.86 (dd, J = 8.0, 14.0 Hz, 1H) ppm; ¹³C NMR (100 MHz, CDCl₃): δ171.8, 165.1, 155.7, 147.5, 143.8, 141.4, 135.1, 131.9, 129.8, 129.4, 129.3, 128.9, 128.4, 127.9, 127.6, 127.2, 125.1, 120.1, 67.3, 60.9, 55.9, 47.2, 37.6, 34.9 ppm; HRMS (ESI): calcd for [C₃₄H₂₈N₂O₆+Na]⁺ 583.1840, found 583.1874.

### Example 4

### Examination of Amount of Amino Acid Used and Reaction Time

Next, a tripeptide (compound 6a) was synthesized by the following method (Table 3). Specifically, the following method is a method of reacting the acylated NCA (compound 4a) of Example 3 with a second amino acid. The effects of the amount of the second amino acid used and the reaction time on the yield of the target product were evaluated.

A dichloromethane solution (flow rate: 1.20 mL/min) of an N-terminal protected amino acid represented by Fmoc-L-Phe-OH (compound 1a; 0.300 M, 1.00 molar equivalent) and N,N-diisopropylethylamine (0.360 M, 1.2 molar equivalents), and a dichloromethane solution (flow rate: 2.00 mL/min) of thionyl chloride (0.216 M, 1.20 molar equivalents) were introduced into a T-shaped mixer at 20°C using a syringe pump. The resulting mixture was introduced into a reaction tube 1 (inner diameter: 0.800 mm, length: 1061 mm, volume: 533 µL, reaction time: 10 sec) at the same temperature. Next, the resulting mixture and a dichloromethane solution (flow rate: 1.20 mL/min) of an amino acid N-carboxy anhydride represented by L-Phe-NCA (compound 3a; 0.300 M, 1.00 molar equivalent) were introduced into a T-shaped mixer at 20°C using a syringe pump. The resulting mixture was introduced into a reaction tube 2 (inner diameter: 0.800 mm, length: 72.9 mm, volume: 37 µL, reaction time: 0.50 sec) at the same temperature. The resulting mixture and a dichloromethane solution (flow rate: 2.00 mL/min) of N,N-diisopropylethylamine (0.216 M, 1.2 molar equivalents) and N-methylimidazole (0.180 M, 1.00 molar equivalent) were injected into a T-shaped mixer at 20°C using a syringe pump. The resulting mixture was introduced into a reaction tube 3 (inner diameter: 0.800 mm, length: 2122 mm, volume: 1067 µL, reaction time: 10 sec) at the same temperature. The resulting mixture and a dichloromethane solution (flow rate: 1.20 mL/min) of a C-terminal protected amino acid represented by H-L-Ala-Ot-Bu (compound 5a; X molar equivalents) were injected into a T-shaped mixer at 20°C using a syringe pump. The resulting mixture was introduced into a reaction tube 4 (inner diameter: 0.800 mm, reaction time: Y sec) at the same temperature. After reaching a steady state over about 60 seconds, the reacted mixture was poured into 1M hydrochloric acid (1.5 mL) and dichloromethane (8 mL) at room temperature for 25 seconds. The aqueous layer was extracted with dichloromethane, and the organic layer was washed with saturated brine, dried over magnesium sulfate, filtered, and concentrated under vacuum at room temperature. The yield of the resulting tripeptide represented by Fmoc-L-Phe-L-Phe-L-Ala-Ot-Bu (compound 6a) was determined by HPLC-UV analysis using a calibration curve (column: COSMOSIL5C₁₈-AR-II 4.6 mm I.D. × 150 mm, solvent: acetonitrile + 0.1% formic acid/H₂O + 0.1% formic acid (0-20 min: 30 to 100%, 20-25 min: 100%, 25-27 min: 30%, 27-30 min: 30%), flow rate: 1.0 mL/min, detection wavelength: 254 nm, temperature: 40°C, retention time: 17.3 min).

Desired compounds 6a could be obtained in all conditions tested (Examples 4-1 to 3-4). In particular, compound 6a was obtained with a sufficiently high yield when using 1.2 molar equivalents of C-terminal protected amino acid for a reaction time of 10 seconds. Therefore, from the viewpoint of suppressing epimerization, shortening the reaction time, and the like, the conditions of Example 4-2 were used in the subsequent Examples.

**Table 3**

| 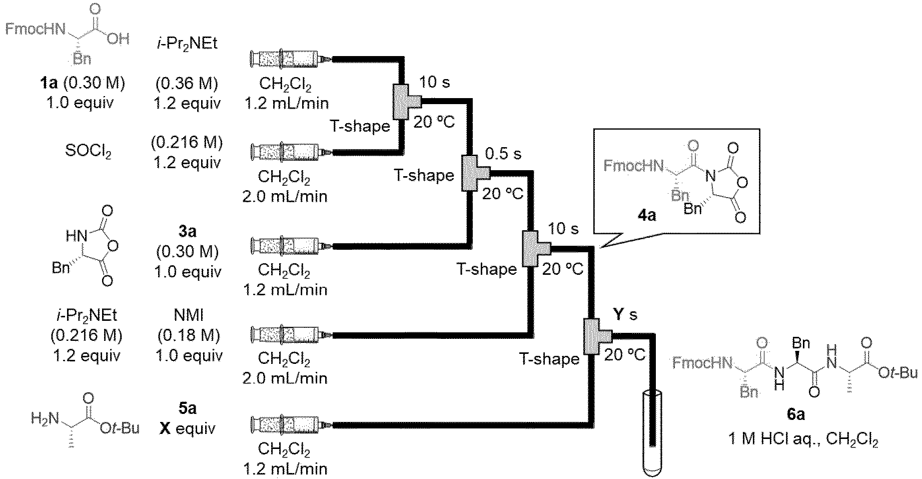 | | | |
|---|---|---|---|
| Example | X (molar equivalents) | Y (sec) | Yield (%) |
| Example 3-1 | 1.0 | 10 | 73 |
| Example 3-2 | 1.2 | 10 | 86, 88, 88 |
| Example 3-3 | 1.2 | 15 | 88 |
| Example 3-4 | 1.5 | 10 | 89 |

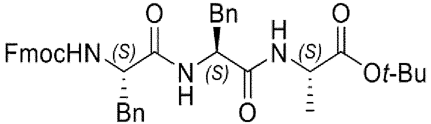

Fmoc-L-Phe-L-Phe-L-Ala-Ot-Bu (compound 6a):
Purification method: Recrystallization from CH₂Cl₂/hexane White solid, mp 162-164°C, IR (neat): 3286, 1732, 1704, 1644, 1539, 1451, 1254, 1146, 741 cm⁻¹; [α]²⁵_{D} = -21.7 (c 0.88, CHCl₃); ¹H NMR (400 MHz, CDCl₃): δ 7.75 (d, J = 7.6 Hz, 2H), 7.50 (dd, J = 7.6, 7.6 Hz, 2H), 7.39 (dd, J = 7.6, 7.6 Hz, 2H), 7.31-7.07 (m, 12H), 6.67 (brs, 1H), 6.54 (brs, 1H), 5.44 (d, J = 6.0 Hz, 1H), 4.68 (d, J = 6.4 Hz, 1H), 4.46 (brs, 1H), 4.42-4.38 (m, 1H), 4.34-4.31 (m, 1H), 4.24 (brs, 1H), 4.14 (t, J = 6.8 Hz, 1H), 3.01-2.99 (m, 4H), 1.43 (s, 9H), 1.28 (d, J = 6.0 Hz, 3H) ppm; ¹³C NMR (100 MHz, CDCl₃): δ171.6, 170.8, 169.8, 156.1, 143.8, 141.4, 136.3, 129.4, 128.8, 128.6, 127.9, 127.2, 127.1, 125.2, 125.1, 120.1, 82.1, 67.2, 56.2, 54.4, 48.9, 47.2, 38.5, 38.4, 28.0, 18.6 ppm; HRMS (ESI): calcd for [C₄₀H₄₃N₃O₆+Na]⁺ 684.3044, found 684.3041.

### Example 5

### Synthesis of Tripeptide 6a by Batch Method

Next, in order to evaluate the effect of the synthesis method, tripeptide 6a was synthesized by a batch method (Table 4). In the batch method, the reaction was carried out under the same conditions as in the flow method with respect to the amounts of the compound and solvent used, and the temperature.

A dichloromethane solution (0.50 mL) of an N-terminal protected amino acid represented by Fmoc-L-Phe-OH (compound 1a; 0.300 M, 1.00 molar equivalent) and N,N-diisopropylethylamine (0.360 M, 1.2 molar equivalents) was stirred vigorously (magnetic stirrer, 1000 rpm). To this was added a dichloromethane solution (0.83 mL) of thionyl chloride (0.216 M, 1.20 molar equivalents) in one portion at 20°C under an argon atmosphere. After stirring at the same temperature for 10 seconds, a dichloromethane solution (0.50 mL) of an amino acid N-carboxy anhydride represented by L-Phe-NCA (compound 3a; 0.300 M, 1.0 molar equivalent) was added in one portion at 20°C under an argon atmosphere. After stirring at the same temperature for 10 seconds, a dichloromethane solution (0.83 mL) of N,N-diisopropylethylamine (0.216 M, 1.2 molar equivalents) and N-methylimidazole (0.180 M, 1.0 molar equivalent) was added in one portion at 20°C under an argon atmosphere. After stirring at the same temperature for 10 seconds, a dichloromethane solution (0.50 mL) of a C-terminal protected amino acid represented by H-L-Ala-Ot-Bu (compound 5a; 0.360 M, 1.20 molar equivalents) was added in one portion at 20°C under an argon atmosphere. The resulting reaction mixture was washed with 1M hydrochloric acid and saturated saline, dried over magnesium sulfate, filtered, and concentrated under vacuum at room temperature. The yield of the resulting tripeptide represented by Fmoc-L-Phe-L-Phe-L-Ala-Ot-Bu (compound 6a) was determined by HPLC-UV analysis using a calibration curve (column: COSMOSIL5C₁₈-AR-II 4.6 mm I.D. × 150 mm, solvent: acetonitrile + 0.1% formic acid/H₂O + 0.1% formic acid (0-20 min: 30 to 100%, 20-25 min: 100%, 25-27 min: 30%, 27-30 min: 30%), flow rate: 1.0 mL/min, detection wavelength :254 nm, temperature: 40°C, retention time: 17.3 min), and the reaction results could be analyzed quickly without silica gel column chromatography. Although the yield was lower than that of the micro-flow method, the batch method also afforded the desired tripeptide (compound 6a) with high reproducibility (Table 4).

**Table 4**

| 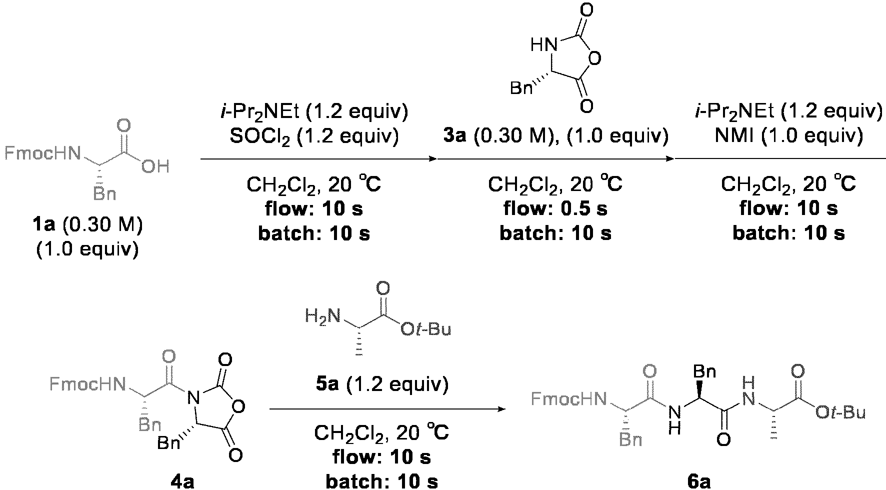 | | |
|---|---|---|
| Yield ( %) | | |
| | Micro-flow method | Batch method |
| N=1 | 86 | 69 |
| N=2 | 88 | 73 |
| N=3 | 88 | 75 |

### Example 6

### Examination of Substrate Range of Tripeptide 6

Next, in order to evaluate the substrate range of tripeptides, tripeptides were synthesized using amino acids having various amino acid residues (Table 5).

A dichloromethane solution (flow rate: 1.20 mL/min) of an N-terminal protected amino acid (compound 1; 0.300 M, 1.00 molar equivalent) and N,N-diisopropylethylamine (0.360 M, 1.2 molar equivalents), and a dichloromethane solution (flow rate: 2.00 mL/min) of thionyl chloride (0.216 M, 1.20 molar equivalents) were introduced into a T-shaped mixer at 20°C using a syringe pump. The resulting mixture was introduced into a reaction tube 1 (inner diameter: 0.800 mm, length: 1061 mm, volume: 533 µL, reaction time: 10 sec) at the same temperature. The resulting mixture and a dichloromethane solution (flow rate: 1.20 mL/min) of an amino acid N-carboxy anhydride (compound 3; 0.300 M, 1.00 molar equivalent) were introduced into a T-shaped mixer at 20°C using a syringe pump. The resulting mixture was introduced into a reaction tube 2 (inner diameter: 0.800 mm, length: 72.9 mm, volume: 37 µL, reaction time: 0.50 sec) at the same temperature. The resulting mixture and a dichloromethane solution (flow rate: 2.00 mL/min) of N,N-diisopropylethylamine (0.216 M, 1.2 molar equivalents) and N-methylimidazole (0.180 M, 1.00 molar equivalent) were introduced into a T-shaped mixer at 20°C using a syringe pump. The resulting mixture was introduced into a reaction tube 3 (inner diameter: 0.800 mm, reaction time: 10 sec or 15 sec) at the same temperature. The resulting mixture and a dichloromethane solution (flow rate: 1.20 mL/min) of a C-terminal protected amino acid (compound 5; 0.360 M, 1.20 molar equivalents) were introduced into a T-shaped mixer at 20°C using a syringe pump. The resulting mixture was introduced into a reaction tube 4 (inner diameter: 0.800 mm, length: 2519 mm, volume: 1267 µL, reaction time: 10 sec) at the same temperature. Then, as necessary, the mixture was mixed in batches at room temperature for 1 minute (compounds 6h, 6j, 6n, and 6p). After reaching a steady state over 90 seconds, the reacted mixture was poured into 1M hydrochloric acid (1.5 mL) and dichloromethane (8 mL) at room temperature for 25 seconds. The aqueous layer was extracted with dichloromethane, and the organic layer was washed with saturated aqueous sodium bicarbonate and saturated brine, dried over magnesium sulfate, filtered, and concentrated under vacuum at room temperature. The residue was purified by recrystallization. When compound 5, which required a long reaction time, was used, the resulting mixture was poured into a dichloromethane solution of MePhe-OMe (1.50 molar equivalents) at room temperature for 25 seconds. Then, the mixture was stirred at the same temperature for 3 hours (compound 6q). 1M hydrochloric acid was added thereto, the aqueous layer was extracted with dichloromethane, and the organic layer was washed with saturated brine, dried over magnesium sulfate, filtered, and concentrated under vacuum at room temperature. The residue was purified by silica gel column chromatography. The purification conditions, yield, and yield are as shown in Table 5.

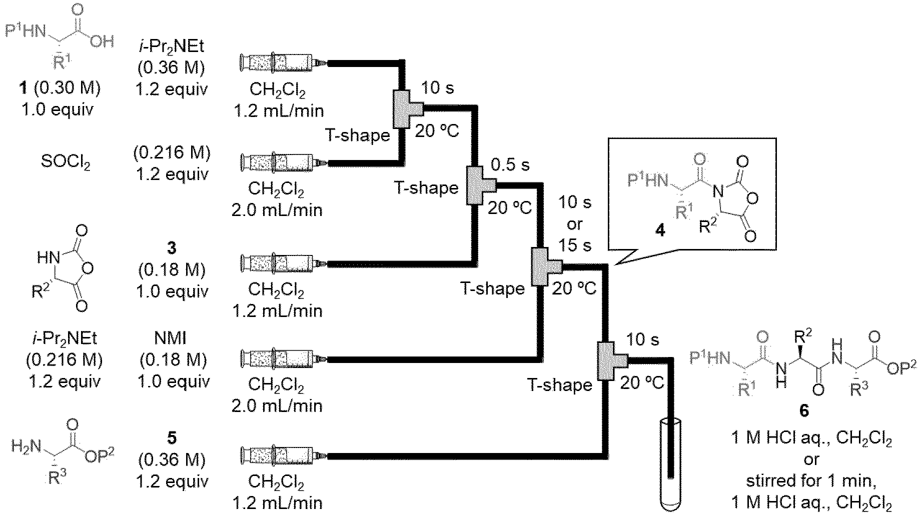

**Table 5**

| Tripeptide | Compound 1 | Compound 3 | Compound 5 | Recrystallization | Yield (mg, mmol) | Yield (%) |
|---|---|---|---|---|---|---|
| Compound 6b | Cbz-Phe | Phe-NCA | Ala-Ot-Bu | Dichloromethane/hexane | 76.3, 0.13 | 89 |
| Compound 6c | Cbz-β-Ala | Phe-NCA | Ala-Ot-Bu | Dichloromethane/hexane | 70.6, 0.14 | 95 |
| Compound 6d | Cbz-Ala | Phe-NCA | Ala-Ot-Bu | Dichloromethane/hexane | 60.9, 0.13 | 82 |
| Compound 6e | Fmoo-Val | Phe-NCA | Ala-Ot-Bu | Dichloromethane/methanolhexane | 79.4, 0.13 | 86 |
| Compound 6f | Fmoo-Tyr(t-Bu) | Phe-NCA | Ala-Ot-Bu | Dichloromethane/hexane | 97.2, 0.13 | 88 |
| Compound 6g | Fmoc-Asp(Bn) | Phe-NCA | Ala-Ot-Bu | Dichloromethane/methanolhexane | 89.3, 0.12 | 83 |
| Compound 6h | Fmoc-Ser(t-Bu) | Phe-NCA | Phe-OMe | Dichloromethane/hexane | 97.1, 0.14 | 94 |
| Compound 6i | Fmoo-Cys(t-Bu) | Phe-NCA | Ala-Ot-Bu | Dichloromethane/methanolhexane | 84.2, 0.13 | 83 |
| Compound 6j | Fmoo-Trp(Boc) | Phe-NCA | Phe-OMe | Dichloromethane/hexane | 108.8, 0.13 | 87 |
| Compound 6k | Fmoo-Lys(Cbz) | Ala-NCA | Ala-Ot-Bu | Dichloromethane/methanolhexane | 96.8, 0.14 | 92 |
| Compound 6l | Fmoc-Thr(Bn) | Ala-NCA | Ala-Ot-Bu | Dichloromethane/methanolhexane | 82.2, 0.13 | 87 |
| Compound 6m | Fmoo-Phe | Ala-NCA | Ala-Ot-Bu | Dichloromethane/hexane | 76.8, 0.13 | 87 |
| Compound 6n | Cbz-Ala | Asp(t-Bu)-NCA | Phe-OMe | Dichloromethane/methanolhexane | 66.3, 0.12 | 80 |
| Compound 6o | Cbz-Ala | Cys(Bn)-NCA | Ala-Ot-Bu | Dichloromethane/hexane | 66.6, 0.12 | 82 |
| Compound 6p | Cbz-Ala | Tyr(t-Bu)-NCA | Phe-OMe | Dichloromethane/hexane | 75.0, 0.12 | 83 |
| Compound 6q | Fmoo-Phe | Phe-NCA | MePhe-OMe | Hexanelethyl acetate (silica gel column chromatography) | 54.7. 0.077 | 51 |

| | | | | | | |
|---|---|---|---|---|---|---|
| *Unless otherwise specified, the amino acid residues contained in compounds 1, 3, and 5 are L-type amino acids. | | | | | | |

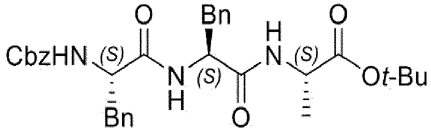

Cbz-L-Phe-L-Phe-L-Ala-Ot-Bu (compound 6b):
White solid, mp 112-115°C, IR (neat): 3290, 1734, 1703, 1644, 1538, 1148 cm⁻¹; [α] ²⁶_{D} = -17.7 (c 1.41, CHCl₃) ; ¹H NMR (400 MHz, CDCl₃): δ 7.36-7.07 (m, 15H), 6.55 (d, J = 7.2 Hz, 1H), 6.50 (d, J = 5.2 Hz, 1H), 5.35 (d, J = 6.4 Hz, 1H), 5.06 (d, J = 12.4 Hz, 1H), 5.01 (d, J = 12.4 Hz, 1H), 4.66 (ddd, J = 7.2, 7.2, 7.2 Hz, 1H), 4.44-4.42 (m, 1H), 4.35-4.28 (m, 1H), 3.06-2.94 (m, 4H), 1.44 (s, 9H), 1.28 (d, J = 7.2 Hz, 3H) ppm; ¹³C NMR (100 MHz, CDCl₃): δ171.6, 170.9, 170.0, 156.1, 136.4, 136.3, 129.4, 128.7, 128.6, 128.2, 128.1, 127.1, 127.0, 82.1, 67.1, 56.2, 54.3, 48.9, 38.6, 38.5, 28.0, 18.5 ppm; HRMS (ESI): calcd for [C₃₃H₃₉N₃O₆+Na]⁺ 596.2731, found 596.2732.

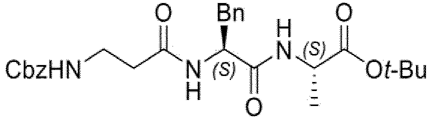

Cbz-β-Ala-L-Phe-L-Ala-Ot-Bu (compound 6c) :
White solid, mp 97-99°C, IR (neat): 3279, 1726, 1641, 1550, 1455, 1369, 1251, 1149 cm⁻¹; [α]²⁶_{D} = -0.77 (c 1.25, CHCl₃) ; ¹H NMR (400 MHz, CDCl₃): δ 7.32-7.09 (m, 10H), 6.71 (d, J = 6.8 Hz, 1H), 6.57 (d, J = 7.2 Hz, 1H), 5.56 (brs, 1H), 5.10-5.03 (m, 2H), 4.72 (ddd, J = 6.8, 6.8, 6.8 Hz, 1H), 4.39-4.32 (m, 1H), 3.41-3.40 (m, 2H), 3.07 (dd, J = 6.8, 13.6 Hz, 1H), 3.00 (dd, J = 6.8, 13.6 Hz, 1H), 2.40-2.35 (m, 2H), 1.43 (s, 9H), 1.29 (d, J = 7.2 Hz, 3H) ppm; ¹³C NMR (100 MHz, CDCl₃): δ171.8, 171.4, 170.4, 156.6, 136.6, 136.4, 129.4, 128.7, 128.6, 128.1, 127.1, 82.2, 66.7, 54.4, 48.9, 38.6, 37.2, 36.1, 28.0, 18.5 ppm; HRMS (ESI): calcd for [C₂₇H₃₅N₃O₆+Na]⁺ 520.2418, found 520.2419.

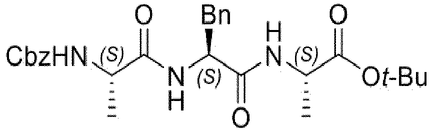

Cbz-L-Ala-L-Phe-L-Ala-Ot-Bu (compound 6d: Angew. Chem. Int. Ed. 2006, 45, 1248-1252.):
White solid, mp 129-133°C, IR (neat): 3310, 3272, 1733, 1698, 1645, 1539, 1239, 1150 cm⁻¹; [α] ²⁶_{D} = -31.0 (c 1.02, CHCl₃) ; ¹H NMR (400 MHz, CDCl₃): δ 7.38-7.30 (m, 5H), 7.29-7.09 (m, 5H), 6.82 (d, J = 6.8 Hz, 1H), 6.67 (d, J = 5.6 Hz, 1H), 5.46 (d, J = 6.8 Hz, 1H), 5.11 (d, J = 12.0 Hz, 1H), 5.05 (d, J = 12.0 Hz, 1H), 4.73 (ddd, J = 6.8, 6.8, 6.8 Hz, 1H), 4.38-4.31 (m, 1H), 4.27-4.24 (m, 1H), 3.06 (d, J = 6.8 Hz, 2H), 1.44 (s, 9H), 1.30 (d, J = 6.8 Hz, 3H), 1.30 (d, J = 7.2 Hz, 3H) ppm; ¹³C NMR (100 MHz, CDCl₃): δ172.2, 171.7, 170.0, 156.1, 136.4, 136.3, 129.5, 128.7, 128.4, 128.2, 127.1, 82.1, 67.2, 54.3, 50.8, 48.9, 38.5, 28.0, 18.7, 18.5 ppm; HRMS (ESI): calcd for [C₂₇H₃₅N₃O₆+Na]⁺520.2418, found 520.2419.

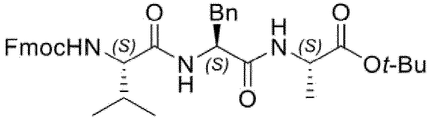

Fmoc-L-Val-L-Phe-L-Ala-Ot-Bu (compound 6e):
White solid, mp 192-196°C, IR (neat): 3289, 1734, 1696, 1644, 1541, 1247, 1147 cm⁻¹; [α]²⁴_{D} = -26.2 (c 1.41, CHCl₃) ; ¹H NMR (400 MHz, CDCl₃): δ 7.76 (d, J = 7.6 Hz, 2H), 7.59 (d, J = 7.6 Hz, 2H), 7.41-7.38 (m, 2H), 7.33-7.26 (m, 2H), 7.23-7.05 (m, 5H), 6.64 (d, J = 7.2 Hz, 1H), 6.55 (d, J = 6.8 Hz, 1H), 5.43 (d, J = 8.0 Hz, 1H), 4.74-4.70 (m, 1H), 4.45 (dd, J = 6.8 Hz, 10.0 Hz, 1H), 4.35-4.31 (m, 2H), 4.20 (t, J = 6.8 Hz, 1H), 4.04-4.01 (m, 1H), 3.07-3.01 (m, 2H), 2.10-2.07 (m, 1H), 1.43 (s, 9H), 1.29 (d, J = 6.4 Hz, 3H), 0.91 (d, J = 6.4 Hz, 3H), 0.84 (d, J = 6.4 Hz, 3H) ppm; ¹³C NMR (100 MHz, CDCl₃): δ171.6, 171.2, 170.0, 156.5, 144.0, 141.5, 136.4, 129.4, 128.7, 127.9, 127.2, 125.2, 120.1, 82.1, 67.2, 60.5, 54.3, 48.9, 47.3, 38.5, 31.1, 28.1, 19.3, 18.6, 17.8 ppm; HRMS (ESI): calcd for [C₃₆H₄₃N₃O₆+Na]⁺ 636.3044, found 636.3044.

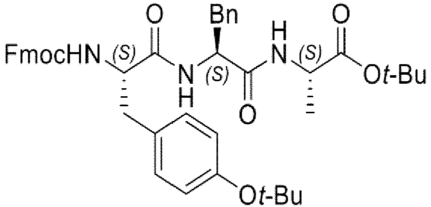

Fmoc-L-Tyr(t-Bu)-L-Phe-L-Ala-Ot-Bu (compound 6f):
White solid, mp 122-125°C, IR (neat): 3290, 1733, 1703, 1644, 1540, 1507, 1236, 1160 cm⁻¹; [α] ²⁴_{D} = -11.7 (c 1.70, CHCl₃) ; ¹H NMR (400 MHz, CDCl₃): δ 7.76 (d, J = 7.6 Hz, 2H), 7.53-7.51 (m, 2H), 7.40 (dd, J = 7.6, 7.6 Hz, 2H), 7.33-7.29 (m, 2H), 7.22-7.00 (m, 7H), 6.88 (d, J = 8.0 Hz, 2H), 6.49 (brs, 1H), 6.37 (brs, 1H), 5.23 (brs, 1H), 4.62 (ddd, J = 6.8, 6.8, 6.8 Hz, 1H), 4.41-4.30 (m, 4H), 4.15 (t, J = 6.8 Hz, 1H), 3.01-2.94 (m, 4H), 1.44 (s, 9H), 1.30 (s, 9H), 1.29 (d, J = 10.8 Hz, 3H) ppm; ¹³C NMR (100 MHz, CDCl₃): δ171.6, 170.7, 169.7, 154.7, 143.8, 141.4, 136.3, 130.9, 129.9, 129.4, 128.7, 127.9, 127.24, 127.18, 125.2, 125.1, 124.4, 120.1, 82.1, 78.5, 67.3, 56.3, 54.4, 48.9, 47.2, 38.4, 37.6, 29.0, 28.1, 18.5 ppm; HRMS (ESI): calcd for [C₄₄H₅₁N₃O₇+Na]⁺ 756.3619, found 756.3622.

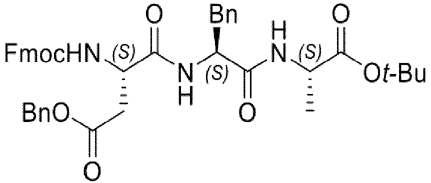

Fmoc-L-Asp(OBn)-L-Phe-L-Ala-Ot-Bu (compound 6g):
White solid, mp 112-115°C, IR (neat): 3294, 1736, 1697, 1646, 1541, 1227, 1148, 739 cm⁻¹; [α] ²⁴_{D} = -7.4 (c 1.82, CHCl₃) ; ¹H NMR (400 MHz, CDCl₃): δ 7.76 (d, J = 7.6 Hz, 2H), 7.56 (d, J = 7.6 Hz, 2H), 7.42-7.10 (m, 14H), 6.92 (d, J = 7.6 Hz, 1H), 6.41 (d, J = 6.0 Hz, 1H), 5.78 (d, J = 8.4 Hz, 1H), 5.12 (s, 2H), 4.63-4.54 (m, 2H), 4.43-4.32 (m, 3H), 4.19 (t, J = 6.8 Hz, 1H), 3.08-2.98 (m, 3H), 2.76 (dd, J = 6.0, 16.4 Hz, 1H), 1.43 (s, 9H), 1.29 (d, J = 6.8 Hz, 3H) ppm; ¹³C NMR (100 MHz, CDCl₃): δ171.7, 170.2, 169.7, 156.1, 143.8, 141.4, 136.4, 135.4, 129.4, 128.8, 128.6, 128.4, 127.9, 127.3, 127.2, 125.2, 120.2, 82.1, 67.5, 67.1, 54.7, 51.3, 48.9, 47.2, 38.0, 36.2, 28.0, 18.5 ppm; HRMS (ESI): calcd for [C₄₂H₄₅N₃O₈+Na]⁺ 742.3099, found 742.3098.

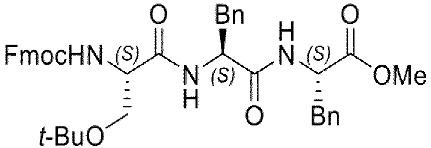

Fmoc-L-Ser(t-Bu)-L-Phe-L-Phe-OMe (compound 6h): White solid, mp 131-135°C, IR (neat): 3294, 2974, 1745, 1698, 1647, 1541, 1241, 1195, 738 cm⁻¹; [α]²⁵_{D} = +11.4 (c 1.75, CHCl₃); ¹H NMR (400 MHz, CDCl₃): δ 7.76 (d, J = 7.2 Hz, 2H), 7.58 (d, J = 7.2 Hz, 2H), 7.39 (dd, J = 7.2, 7.2 Hz, 2H), 7.31 (dd, J = 7.2, 7.2 Hz, 2H), 7.29-7.15 (m, 10H), 7.01 (d, J = 6.0 Hz, 2H), 6.40 (d, J = 5.2 Hz, 1H), 5.63 (d, J = 5.2 Hz, 1H), 4.75-4.71 (m, 1H), 4.63 (ddd, J = 6.8, 6.8, 6.8 Hz, 1H), 4.39-4.33 (m, 2H), 4.21 (t, J = 7.6 Hz, 1H), 4.14 (brs, 1H), 3.74-3.62 (m, 4H), 3.26 (dd, J = 8.0 Hz, 1H), 3.09-2.95 (m, 4H), 1.12 (s, 9H) ppm; ¹³C NMR (100 MHz, CDCl₃): δ171.5, 170.3, 156.2, 143.9, 143.8, 141.4, 136.4, 135.9, 129.4, 129.3, 128.8, 128.7, 127.9, 127.2, 125.2, 120.1, 74.6, 67.3, 61.6, 54.5, 53.6, 52.4, 47.2, 37.9, 27.4 ppm; HRMS (ESI): calcd for [C₄₁H₄₅N₃O₇+Na]⁺ 714.3150, found 714.3150.

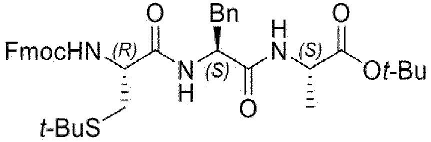

Fmoc-L-Cys(t-Bu)-L-Phe-L-Ala-Ot-Bu (compound 6i):
White solid, mp 126-128°C, IR (neat): 3290, 1732, 1708, 1645, 1538, 1452, 1245, 1150, 737 cm⁻¹; [α]²⁴_{D} = -17.0 (c 1.81, CHCl₃) ; ¹H NMR (400 MHz, CDCl₃): δ 7.77 (d, J = 7.6 Hz, 2H), 7.58 (d, J = 7.6 Hz, 2H), 7.40 (dd, J = 7.6, 7.6 Hz, 2H), 7.31 (dd, J = 7.6, 7.6 Hz, 2H), 7.26-7.15 (m, 5H), 6.80 (d, J = 7.2 Hz, 1H), 6.53 (d, J = 5.2 Hz, 1H), 5.67 (brs, 1H), 4.69 (ddd, J = 7.2, 7.2, 7.2 Hz, 1H), 4.43-4.28 (m, 4H), 4.21 (t, J = 7.2 Hz, 1H), 3.15-3.06 (m, 2H), 2.93 (dd, J = 5.6, 13.2 Hz, 1H), 2.80 (dd, J = 6.8, 13.2 Hz, 1H), 1.44 (s, 9H), 1.30 (s, 12H) ppm; ¹³C NMR (100 MHz, CDCl₃): δ171.6, 170.0, 169.7, 156.1, 143.8, 143.8, 141.4, 136.3, 129.5, 128.8, 127.9, 127.2, 125.2, 120.1, 82.0, 67.4, 55.0, 54.5, 48.9, 47.2, 43.4, 38.0, 31.0, 30.8, 28.0, 18.5 ppm; HRMS (ESI): calcd for [C₃₈H₄₇N₃O₆S₁+Na]⁺ 696.3078, found 696.3078.

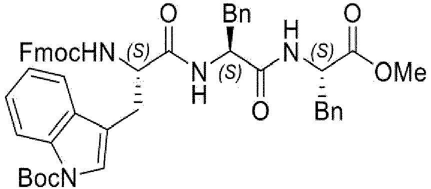

Fmoc-L-Trp(Boc)-L-Phe-L-Phe-OMe (compound 6j):
White solid, mp 184-188°C, IR (neat): 3304, 1732, 1706, 1672, 1643, 1539, 1453, 1369, 1270, 1160, 739 cm⁻¹; [α] ²⁴_{D} = -10.0 (c 1.30, CHCl₃) ; ¹H NMR (400 MHz, CDCl₃): δ 8.11 (d, J = 8.0 Hz, 1H), 7.73 (d, J = 7.2 Hz, 2H), 7.57 (d, J = 7.6 Hz, 1H), 7.51-7.46 (m, 3H), 7.39-7.35 (m, 2H), 7.32-7.15 (m, 7H), 7.13-7.07 (m, 3H), 6.99-6.96 (m, 4H), 6.68 (d, J = 4.8 Hz, 1H), 6.37 (brs, 1H), 5.52 (brs, 1H), 4.73-4.67 (m, 1H), 4.60-4.51 (m, 2H), 4.34 (dd, J = 7.6, 10.4 Hz, 1H), 4.25-4.23 (m, 1H), 4.14 (t, J = 7.2 Hz, 1H), 3.61 (s, 3H), 3.13-2.89 (m, 6H), 1.61 (s, 9H) ppm; ¹³C NMR (100 MHz, CDCl₃): δ171.4, 171.0, 170.0, 156.2, 149.6, 143.8, 141.4, 136.2, 135.8, 135.6, 130.4, 129.3, 129.2, 128.7, 128.6, 127.8, 127.2, 127.1, 125.2, 124.8, 124.6, 122.9, 120.1, 119.1, 115.5, 115.3, 83.9, 67.4, 55.0, 54.4, 53.6, 52.3, 47.1, 38.2, 38.0, 28.3, 27.9 ppm; HRMS (ESI): calcd for [C₅₀H₅₀N₄O₈+Na]⁺ 857.3521, found 857.3523.

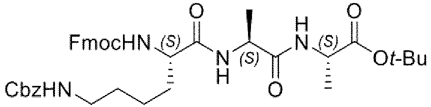

Fmoc-L-Lys(Cbz)-L-Ala-L-Ala-Ot-Bu (compound 6k):
White solid, mp 156-159°C, IR (neat): 3287, 1732, 1716, 1684, 1638, 1541, 1261, 1151, 739 cm⁻¹; [α] ²⁴_{D} = -8.1 (c 1.73, CHCl₃) ¹H NMR (400 MHz, CDCl₃): δ 7.75 (d, J = 7.2 Hz, 2H), 7.57 (d, J = 7.2 Hz, 2H), 7.40-7.26 (m, 9H), 6.71 (d, J = 7.2 Hz, 1H), 6.66 (d, J = 6.8 Hz, 1H), 5.68 (d, J = 6.0 Hz, 1H), 5.10-5.07 (m, 3H), 4.51-4.37 (m, 4H), 4.19 (t, J = 6.8 Hz, 1H), 3.20-3.15 (m, 1H), 1.84 (brs, 1H), 1.68-1.67 (m, 1H), 1.51-1.31 (m, 19H) ppm; ¹³C NMR (100 MHz, DMSO-d₆): δ172.0, 171.63, 171.56, 156.1, 156.0, 143.9, 143.8, 140.7, 137.3, 128.3, 127.7, 127.6, 127.1, 125.3, 120.1, 80.3, 65.6, 65.1, 54.5, 48.3, 47.6, 46.7, 31.6, 29.1, 27.6, 22.8, 18.4, 16.8 ppm; HRMS (ESI): calcd for [C₃₉H₄₈N₄O₈+H]⁺701.3544, found 701.3545, [C₃₉H₄₈N₄O₈+Na]⁺723.3364, found 723.3363.

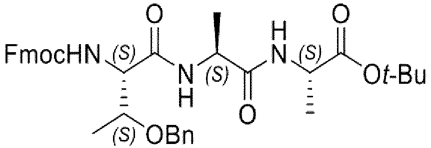

Fmoc-L-Thr(Bn)-L-Ala-L-Ala-Ot-Bu (compound 6l):
White solid, mp 182-185°C, IR (neat): 3294, 1732, 1705, 1641, 1537, 1451, 1245, 1154, 739 cm⁻¹; [α] ²⁴_{D} = -9.6 (c 1.28, CHCl₃) ; ¹H NMR (400 MHz, CDCl₃): δ 7.76 (d, J = 7.6 Hz, 2H), 7.60 (d, J = 7.2 Hz, 2H), 7.41-7.29 (m, 9H), 7.02 (d, J = 7.2 Hz, 1H), 6.69 (d, J = 6.8 Hz, 1H), 5.82 (d, J = 6.4 Hz, 1H), 4.68 (d, J = 11.6 Hz, 1H), 4.57-4.36 (m, 6H), 4.22 (t, J = 6.8 Hz, 1H), 4.16-4.15 (m, 1H), 1.46 (s, 9H), 1.34 (d, J = 7.6 Hz, 3H), 1.32 (d, J = 6.8 Hz, 3H), 1.17 (d, J = 6.0 Hz, 3H) ppm; ¹³C NMR (100 MHz, CDCl₃): δ171.9, 171.1, 169.4, 156.4, 144.0, 143.8, 141.4, 137.9, 128.6, 128.1, 127.9, 127.2, 125.2, 120.1, 82.1, 74.8, 71.7, 67.3, 58.0, 49.1, 48.9, 47.3, 28.1, 18.5, 18.2, 15.2 ppm; HRMS (ESI): calcd for [C₃₆H₄₃N₃O₇+Na]⁺652.2993, found 652.2990.

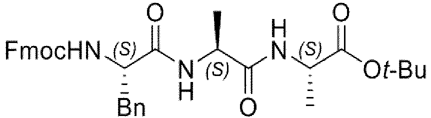

Fmoc-L-Phe-L-Ala-L-Ala-Ot-Bu (compound 6m):
White solid, mp 153-156°C, IR (neat): 3296, 1731, 1707, 1643, 1539, 1451, 1254, 1154, 739 cm⁻¹; [α]²⁵_{D} = -16.5 (c 1.53, CHCl₃); ¹H NMR (400 MHz, CDCl₃): δ 7.74 (d, J = 7.6 Hz, 2H), 7.51 (dd, J = 7.6, 7.6 Hz, 2H), 7.38 (dd, J = 7.6, 7.6 Hz, 2H), 7.29-7.18 (m, 7H), 6.92 (brs, 2H), 5.73 (d, J = 7.2 Hz, 1H), 4.59-4.52 (m, 2H), 4.43-4.37 (m, 2H), 4.27-4.23 (m, 1H), 4.15 (t, J = 6.8 Hz, 1H), 3.07 (d, J = 5.6 Hz, 2H), 1.44 (s, 9H), 1.34 (d, J = 6.8 Hz, 6H) ppm; ¹³C NMR (100 MHz, CDCl₃): δ172.0, 171.4, 170.9, 156.1, 143.9, 141.4, 136.4, 129.5, 128.7, 127.8, 127.2, 125.3, 125.2, 120.1, 82.2, 67.2, 56.1, 49.0, 48.9, 47.2, 38.8, 28.1, 18.9, 18.6 ppm; HRMS (ESI) : calcd for [C₃₄H₃₉N₃O₆+Na]⁺ 608.2731, found 608.2729.

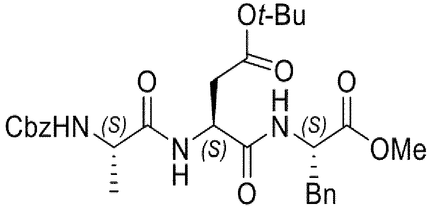

Cbz-L-Ala-L-Asp(Ot-Bu)-L-Phe-OMe (compound 6n):
White solid, mp 139-142°C, IR (neat): 3299, 1731, 1653, 1523, 1217, 1155, cm⁻¹; [α]²³_{D} = +19.1 (c 1.1, CHCl₃) ; ¹H NMR (400 MHz, CDCl₃): δ 7.38-7.13 (m, 11H), 7.04 (d, J = 7.2 Hz, 1H), 5.26 (brs, 1H), 5.11 (d, J = 12.4 Hz, 1H), 5.07 (d, J = 12.4 Hz, 1H), 4.79-4.74 (m, 2H), 4.21-4.18 (m, 1H), 3.69 (s, 3H), 3.13 (dd, J = 6.0, 13.6 Hz, 1H), 3.05 (dd, J = 6.0, 13.6 Hz, 1H), 2.91-2.88 (m, 1H), 2.53 (dd, J = 6.4, 16.4 Hz, 1H), 1.43 (s, 9H), 1.31 (d, J = 7.2 Hz, 3H) ppm; ¹³C NMR (100 MHz, CDCl₃): δ172.2, 171.5, 170.1, 156.1, 136.3, 135.9, 129.4, 128.71, 128.68, 128.3, 128.2, 127.2, 82.1, 67.2, 53.8, 52.4, 50.8, 49.2, 37.7, 36.8, 28.1, 18.7 ppm; HRMS (ESI) : calcd for [C₂₉H₃₇N₃O₈+Na]⁺578.2473, found 578.2473.

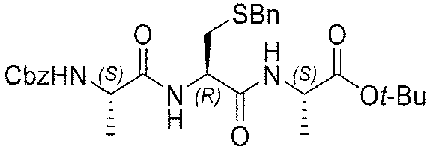

Cbz-L-Ala-L-Cys(Bn)-L-Ala-Ot-Bu (compound 6o):
Yellow amorphous solid, IR (neat): 3301, 1731, 1692, 1641, 1538, 1239, 1146, 698 cm⁻¹; [α]²³_{D} = -23.4 (c 1.08, CHCl₃) ; ¹H NMR (400 MHz, CDCl₃): δ 7.34-7.21 (m, 10H), 6.97 (d, J = 5.6 Hz, 1H), 6.90 (d, J = 6.4 Hz, 1H), 5.42 (d, J = 6.4 Hz, 1H), 5.11 (s, 2H), 4.56-4.54 (m, 1H), 4.43-4.38 (m, 1H), 4.28-4.26 (m, 1H), 3.74 (s, 2H), 2.91 (dd, J = 5.2, 13.6 Hz, 1H), 2.73 (dd, J = 6.4, 13.6 Hz, 1H), 1.45 (s, 9H), 1.37 (d, J = 7.6 Hz, 3H), 1.35 (d, J = 7.2 Hz, 3H) ppm; ¹³C NMR (100 MHz, CDCl₃): δ172.3, 171.6, 169.4, 156.1, 138.2, 136.2, 129.2, 128.8, 128.7, 128.4, 128.2, 127.4, 82.2, 67.2, 52.3, 50.9, 49.2, 36.6, 33.7, 28.1, 18.8, 18.4 ppm; HRMS (ESI): calcd for [C₂₈H₃₇N₃O₆S₁+Na]⁺566.2295, found 556.2297.

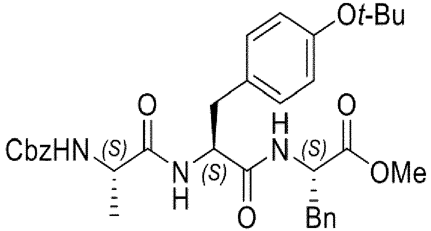

Cbz-L-Ala-L-Tyr(t-Bu)-L-Phe-OMe (compound 6p):
White solid, mp 163-166°C, IR (neat): 3296, 1742, 1698, 1646, 1538, 1507, 1236, 1163 cm⁻¹; [α]²⁴_{D} = -3.3 (c 1.85, CHCl₃) ; ¹H NMR (400 MHz, CDCl₃): δ 7.36-7.18 (m, 8H), 7.06-7.00 (m, 4H), 6.86 (d, J = 8.4 Hz, 2H), 6.68 (d, J = 6.8 Hz, 1H), 6.41 (d, J = 5.6 Hz, 1H), 5.30 (d, J = 6.8 Hz, 1H), 5.11 (d, J = 12.4 Hz, 1H), 5.03 (d, J = 12.4 Hz, 1H), 4.75 (ddd, J = 5.6 Hz, 1H), 4.60 (ddd, J = 6.8, 6.8, 6.8 Hz, 1H), 4.21-4.17 (m, J = 6.4 Hz, 1H), 3.66 (s, 3H), 3.07 (dd, J = 5.6, 13.2 Hz, 1H), 3.00-2.95 (m, 3H), 1.30 (s, 9H), 1.26 (d, J = 6.8 Hz, 3H) ppm; ¹³C NMR (100 MHz, CDCl₃): δ172.2, 171.4, 170.3, 154.6, 136.2, 135.8, 131.1, 129.9, 129.3, 128.7, 128.4, 128.3, 127.2, 124.4, 78.5, 67.3, 54.4, 53.5, 52.4, 50.7, 37.9, 37.5, 28.9, 18.5 ppm; HRMS (ESI): calcd for [C₃₄H₄₁N₃O₇+H]⁺604.3017, found 604.3016, [C₃₄H₄₁N₃O₇+Na]⁺ 626.2837, found 626.2837.

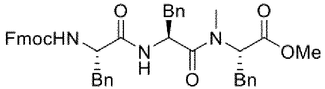

Fmoc-L-Phe-L-Phe-L-MePhe-OMe (compound 6q):
White solid, mp 80-84°C, IR (neat): 3296, 1739, 1724, 1637, 1538, 1496, 1452, 1256, 741 cm⁻¹; [α]²¹_{D} = -55.9 (c 0.77, CHCl₃) ; ¹H NMR (400 MHz, CDCl₃): (¹H NMR spectrum was observed as conformational isomers. Only NMR signals of major isomer were shown) δ 7.76 (d, J = 7.6 Hz, 2H), 7.55-7.50 (m, 2H), 7.40 (dd, J = 7.6, 7.6 Hz, 2H), 7.31 (dd, J = 7.6, 7.6 Hz, 2H), 7.25-7.10 (m, 15H), 6.51 (d, J = 7.6 Hz, 1H), 5.26-5.24 (m, 1H), 5.13 (d, J = 7.6 Hz, 1H), 5.01-4.95 (m, 1H), 4.46-4.42 (m, 1H), 4.40-4.35 (m, 1H), 4.30-4.26 (m, 1H), 4.19-4.12 (m, 1H), 3.68 (s, 3H), 3.32 (dd, J = 4.8, 14.0 Hz, 1H), 3.02-2.80 (m, 5H), 2.68 (s, 3H) ppm; ¹³C NMR (100 MHz, CDCl₃): δ 171.3, 170.8, 169.9, 155.8, 143.9, 141.4, 136.7, 136.3, 135.9, 129.7, 129.5, 129.0, 128.8, 128.6, 128.5, 127.9, 127.2, 127.1, 127.0, 125.3, 125.2, 120.1, 67.1, 58.7, 55.8, 52.4, 50.5, 47.2, 38.9, 38.3, 34.8, 32.7 ppm; HRMS (ESI): calcd for [C₄₄H₄₃N₃O₆+Na]⁺732.3044, found 732.3046.

### Example 7

### Synthesis of Protected Stellarin G Precursor (Compound 7a)

A protected Stellarin G precursor (compound 7a; SEQ ID NO: 1), which is a pentapeptide, was synthesized by the following method. Specifically, a tripeptide was first synthesized by the method of the present invention, and then the tripeptide was extended by two residues to obtain a pentapeptide.

### Step 1: Synthesis of Tripeptide (Compound 6r)

A dichloromethane solution (flow rate: 1.20 mL/min) of an N-terminal protected amino acid represented by Cbz-L-Leu-OH (compound 1b; 0.300 M, 1.0 molar equivalent) and N,N-diisopropylethylamine (0.360 M, 1.20 molar equivalents), and a dichloromethane solution (flow rate: 2.00 mL/min) of thionyl chloride (0.216 M, 1.2 molar equivalents) were introduced into a T-shaped mixer at 20°C using a syringe pump. The resulting mixture was introduced into a reaction tube 1 (inner diameter: 0.800 mm, length: 1061 mm, volume: 533 µL, reaction time: 10 sec) at the same temperature. The resulting mixture and a dichloromethane solution (flow rate: 1.20 mL/min) of an amino acid N-carboxy anhydride represented by L-Ala-NCA (compound 3b; 0.300 M, 1.0 molar equivalent) were introduced into a T-shaped mixer at 20°C using a syringe pump. The resulting mixture was introduced into a reaction tube 2 (inner diameter: 0.800 mm, length: 72.9 mm, volume: 37 µL, reaction time: 0.50 sec) at the same temperature. The resulting mixture and a dichloromethane solution (flow rate: 2.00 mL/min) of N,N-diisopropylethylamine (0.216 M, 1.2 molar equivalents) and N-methylimidazole (0.180 M, 1.00 molar equivalent) were introduced into a T-shaped mixer at 20°C using a syringe pump. The resulting mixture was introduced into a reaction tube 3 (inner diameter: 0.800 mm, length: 3133 mm, volume: 1600 µL, reaction time: 15 sec) at the same temperature. The resulting mixture and a dichloromethane solution (flow rate: 1.20 mL/min) of a C-terminal protected amino acid represented by H-Gly-Ot-Bu (compound 5b; 0.360 M, 1.2 molar equivalents) were introduced into a T-shaped mixer at 20°C using a syringe pump. The resulting mixture was introduced into a reaction tube 4 (inner diameter: 0.800 mm, length: 2519 mm, volume: 1267 µL, reaction time: 10 sec) at the same temperature. After reaching a steady state over 90 seconds, the reacted mixture was poured into 1M hydrochloric acid (1.5 mL) and dichloromethane (8 mL) at room temperature for 60 seconds. The aqueous layer was extracted with dichloromethane, and the organic layer was washed with a saturated aqueous NaHCO₃ solution and saturated brine, dried over magnesium sulfate, filtered, and concentrated under vacuum at room temperature. Palladium carbon (10 wt%) was added at room temperature to a methanol solution containing the resulting semi-pure tripeptide represented by Cbz-L-Leu-L-Ala-Gly-Ot-Bu (compound 6q; 1.0 molar equivalent). The resulting mixture was stirred at room temperature under a hydrogen atmosphere. After stirring for 4 hours, the resulting mixture was filtered through Celite and concentrated under vacuum at room temperature to obtain a semi-pure tripeptide (compound 6r).

### Step 2: Synthesis of Pentapeptide (Compound 7a)

A dichloromethane solution (flow rate: 1.20 mL/min) of an N-terminal protected amino acid represented by Cbz-L-Ala-OH (compound 1c; 0.300 M, 1.0 molar equivalent) and N,N-diisopropylethylamine (0.360 M, 1.2 molar equivalents), and a dichloromethane solution (flow rate: 2.00 mL/min) of thionyl chloride (0.216 M, 1.2 molar equivalents) were introduced into a T-shaped mixer at 20°C using a syringe pump. The resulting mixture was introduced into a reaction tube 1 (inner diameter: 0.800 mm, length: 1061 mm, volume: 533 µL, reaction time: 10 sec) at the same temperature. The resulting mixture and a dichloromethane solution (flow rate: 1.20 mL/min) of an amino acid N-carboxy anhydride represented by L-Tyr(t-Bu)-NCA (compound 3c; 0.300 M, 1.0 molar equivalent) were introduced into a T-shaped mixer at 20°C using a syringe pump. The resulting mixture was introduced into a reaction tube 2 (inner diameter: 0.800 mm, length: 72.9 mm, volume: 37 µL, reaction time: 0.50 sec) at the same temperature. The resulting mixture and a dichloromethane solution (flow rate: 2.00 mL/min) of N,N-diisopropylethylamine (0.216 M, 1.2 molar equivalents) and N-methylimidazole (0.180 M, 1.0 molar equivalent) were introduced into a T-shaped mixer at 20°C using a syringe pump. The resulting mixture was introduced into a reaction tube 3 (inner diameter: 0.800 mm, length: 2122 mm, volume: 1067 µL, reaction time: 10 sec) at the same temperature. After reaching a steady state over 70 seconds, the resulting mixture was poured into a test tube containing a dichloromethane solution (1.2 molar equivalents) of the semi-pure tripeptide represented by H-L-Leu-L-Ala-Gly-Ot-Bu (compound 6r) at room temperature for 50 seconds. After stirring at room temperature for 10 minutes, 1M hydrochloric acid (1.5 mL) and dichloromethane (8 mL) were added. The aqueous layer was extracted with dichloromethane, and the organic layer was washed with a saturated aqueous NaHCO₃ solution and saturated brine, dried over magnesium sulfate, filtered, and concentrated under vacuum at room temperature. The crude product was recrystallized from dichloromethane/hexane. Without the need for silica gel column chromatography, a protected Stellarin G precursor consisting of the amino acid sequence represented by SEQ ID NO: 1 (compound 7a) was obtained with a high yield of 80%.

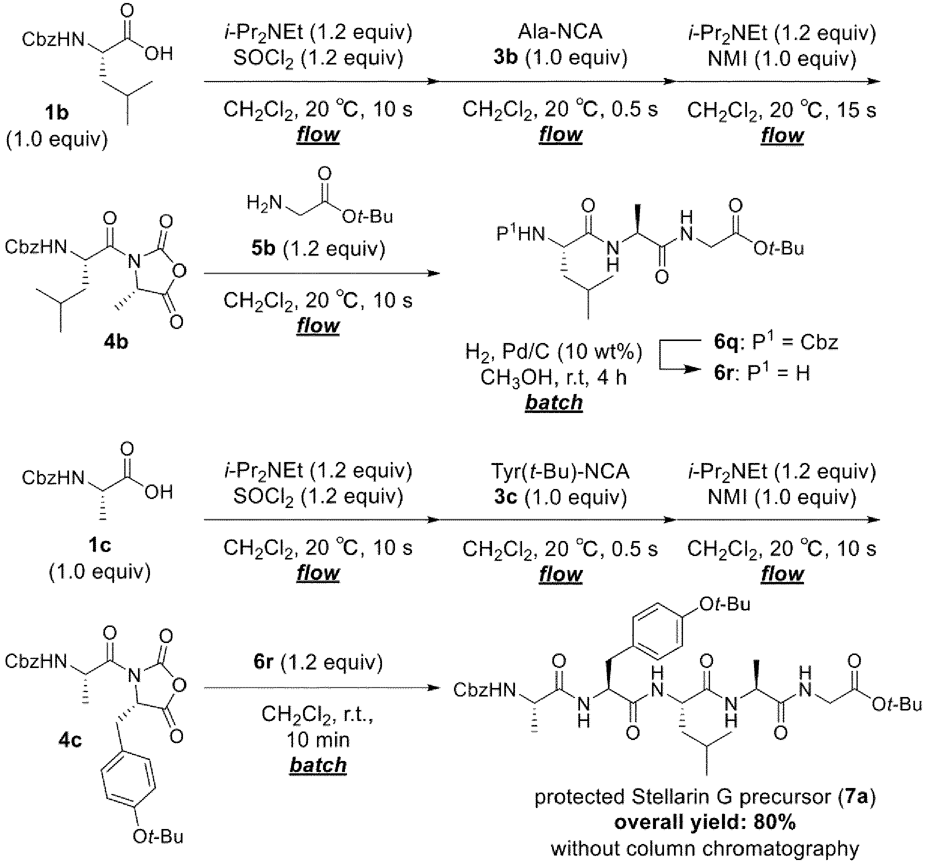

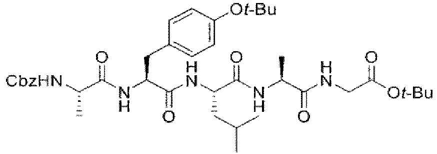

Protected Stellarin G precursor (compound 7a):
Colorless amorphous solid, IR (neat): 3282, 2977, 1734, 1715, 1705, 1682, 1630, 1540, 1407, 1455, 1366, 1237, 1161 cm⁻¹; [α]²³_{D} = -26.1 (c 2.09, CHCl₃) ; ¹H NMR (400 MHz, CD₃OD) : δ7.36-7.30 (m, 5H), 7.13 (d, J = 8.4 Hz, 2H), 6.87 (d, J = 8.4 Hz, 2H), 5.11 (d, J = 12.4 Hz, 1H), 5.00 (d, J = 12.4 Hz, 1H), 4.65 (q, J = 4.8 Hz, 1H), 4.45-4.40 (m, 2H), 4.11 (q, J = 7.2 Hz, 1H), 3.89-3.77 (m, 2H), 3.12 (dd, J = 4.8, 13.6 Hz, 1H), 2.95 (dd, J = 8.4, 13.6 Hz, 1H), 1.63-1.62 (m, 3H), 1.45 (s, 9H), 1.39 (d, J = 7.6 Hz, 3H), 1.28 (s, 9H), 1.24 (d, J = 7.2 Hz, 3H), 0.92-0.87 (m, 6H) ppm; ¹³C NMR (100 MHz, CD₃OD): δ175.6, 175.1, 174.2, 173.6, 170.1, 158.4, 155.4, 137.9, 133.1, 130.9, 129.5, 129.1, 128.8, 125.1, 82.7, 79.4, 67.8, 56.0, 53.4, 52.4, 50.2, 42.8, 41.6, 37.5, 29.2, 28.3, 25.7, 23.6, 22.0, 18.2, 18.0 ppm; HRMS (ESI): calcd for [C₃₉H₅₇N₅O₉+Na]⁺ 762.4048, found 762.4049.

### Example 8

### Synthesis of Beefy Meaty Peptide (Compound 9b)

Next, a beefy meaty peptide (compound 9b; SEQ ID NO: 7), which is an octapeptide, was synthesized by the following method. Specifically, a tripeptide was first synthesized by the method of the present invention, and then the tripeptide was extended by two residues to obtain a pentapeptide. Then, the pentapeptide was extended by two residues to obtain a heptapeptide, and the heptapeptide was extended by one residue to obtain an octapeptide.

### Step 1: Synthesis of Tripeptide (Compound 6t)

A dichloromethane solution (flow rate: 1.20 mL/min) of an N-terminal protected amino acid represented by Cbz-L-Ser(t-Bu)-OH (compound 1d; 0.324 M, 1.1 molar equivalents) and N,N-diisopropylethylamine (0.389 M, 1.3 molar equivalents), and a dichloromethane solution (flow rate: 2.00 mL/min) of thionyl chloride (0.233 M, 1.3 molar equivalents) were introduced into a T-shaped mixer at 20°C using a syringe pump. The resulting mixture was introduced into a reaction tube 1 (inner diameter: 0.800 mm, length: 1061 mm, volume: 533 µL, reaction time: 10 sec) at the same temperature. The resulting mixture and a dichloromethane solution (flow rate: 1.20 mL/min) of an amino acid N-carboxy anhydride represented by L-Leu-NCA (compound 3d; 0.324 M, 1.1 molar equivalents) were introduced into a T-shaped mixer at 20°C using a syringe pump. The resulting mixture was introduced into a reaction tube 2 (inner diameter: 0.800 mm, length: 72.9 mm, volume: 37 µL, reaction time: 0.50 sec) at the same temperature. The resulting mixture and a dichloromethane solution (flow rate: 2.00 mL/min) of N,N-diisopropylethylamine (0.233 M, 1.3 molar equivalents) and N-methylimidazole (0.194 M, 1.1 molar equivalents) were introduced into a T-shaped mixer at 20°C using a syringe pump. The resulting mixture was introduced into a reaction tube 3 (inner diameter: 0.800 mm, length: 2122 mm, volume: 1067 µL, reaction time: 10 sec) at the same temperature. After reaching a steady state over about 90 seconds, the resulting mixture was poured into a dichloromethane solution of a C-terminal protected amino acid represented by H-Ala-Ot-Bu·HCl (compound 5a; 1.0 molar equivalent) and N,N-diisopropylethylamine (1.0 molar equivalent) at room temperature for 265 seconds. After stirring at room temperature for 1 minute, 1M hydrochloric acid (5 mL) was added for quenching. The aqueous layer was extracted with dichloromethane, and the organic layer was washed with a saturated aqueous NaHCO₃ solution, water, and saturated brine, dried over magnesium sulfate, filtered, and concentrated under vacuum at room temperature. The crude product was recrystallized from hexane. 10 wt% palladium carbon (25 wt%) was added at room temperature to a methanol solution containing the resulting tripeptide represented by Cbz-L-Ser(t-Bu)-L-Leu-L-Ala-Ot-Bu (compound 6s; 1.0 molar equivalent). The resulting mixture was stirred at room temperature under a hydrogen atmosphere. After stirring for 3 hours, the resulting mixture was filtered through Celite and concentrated under vacuum at room temperature. The crude product was dissolved in ethyl acetate, washed with 10% aqueous K₂CO₃ solution, water, and saturated brine, dried over sodium sulfate, filtered, and concentrated under vacuum at room temperature. A semi-pure tripeptide represented by H-L-Ser(t-Bu)-L-Leu-L-Ala-Ot-Bu (compound 6t) was obtained.

### Step 2: Synthesis of Pentapeptide (Compound 7c)

A dichloromethane solution (flow rate: 1.20 mL/min) of an N-terminal protected amino acid represented by Cbz-L-Glu(Ot-Bu)-OH (compound 1e; 0.324 M, 1.1 molar equivalents) and N,N-diisopropylethylamine (0.389 M, 1.3 molar equivalents), and a dichloromethane solution (flow rate: 2.00 mL/min) of thionyl chloride (0.233 M, 1.3 molar equivalents) were introduced into a T-shaped mixer at 20°C using a syringe pump. The resulting mixture was introduced into a reaction tube 1 (inner diameter: 0.800 mm, length: 1061 mm, volume: 533 µL, reaction time: 10 sec) at the same temperature. The resulting mixture and a dichloromethane solution (flow rate: 1.20 mL/min) of an amino acid N-carboxy anhydride represented by L-Glu(Ot-Bu)-NCA (compound 3e; 0.324 M, 1.1 molar equivalents) were introduced into a T-shaped mixer at 20°C using a syringe pump. The resulting mixture was introduced into a reaction tube 2 (inner diameter: 0.800 mm, length: 72.9 mm, volume: 37 µL, reaction time: 0.50 sec) at the same temperature. The resulting mixture and a dichloromethane solution (flow rate: 2.00 mL/min) of N,N-diisopropylethylamine (0.233 M, 1.3 molar equivalents) and N-methylimidazole (0.194 M, 1.1 molar equivalents) were introduced into a T-shaped mixer at 20°C using a syringe pump. The resulting mixture was introduced into a reaction tube 3 (inner diameter: 0.800 mm, length: 2122 mm, volume: 1067 µL, reaction time: 10 sec) at the same temperature. After reaching a steady state over about 90 seconds, the resulting mixture was poured into a dichloromethane solution of the semi-pure tripeptide (compound 6t; 1.0 molar equivalent) at room temperature for 106 seconds. After stirring at room temperature for 10 minutes, 1M hydrochloric acid (3.5 mL) was added for quenching. Water was further added, the aqueous layer was extracted with dichloromethane, and the organic layer was washed with a saturated aqueous NaHCO₃ solution and saturated brine, dried over sodium sulfate, filtered, and concentrated under vacuum at room temperature to obtain a semi-pure pentapeptide (compound 7b; SEQ ID NO: 2). The crude product was recrystallized from ethyl acetate. 10 wt% palladium carbon (25 wt%) was added at room temperature to a methanol solution containing the resulting pentapeptide represented by Cbz-L-Glu(Ot-Bu)-L-Glu(Ot-Bu)-L-Ser(t-Bu)-L-Leu-L-Ala-Ot-Bu (compound 7b; 1.0 molar equivalent). The resulting mixture was stirred at room temperature under a hydrogen atmosphere. After stirring for 3 hours, the resulting mixture was filtered through Celite and concentrated under vacuum at room temperature. The crude product was dissolved in ethyl acetate, washed with 1M aqueous NaOH solution, water, and saturated brine, dried over sodium sulfate, filtered, and concentrated under vacuum at room temperature. A semi-pure pentapeptide represented by H-L-Glu(Ot-Bu)-L-Glu(Ot-Bu)-L-Ser(t-Bu)-L-Leu-L-Ala-Ot-Bu (compound 7c; SEQ ID NO: 3) was obtained.

### Step 3: Synthesis of Heptapeptide (Compound 8b)

A dichloromethane solution (flow rate: 1.20 mL/min) of an N-terminal protected amino acid represented by Cbz-Gly-OH (compound 1f; 0.324 M, 1.1 molar equivalents) and N,N-diisopropylethylamine (0.389 M, 1.3 molar equivalents), and a dichloromethane solution (flow rate: 2.00 mL/min) of thionyl chloride (0.233M, 1.3 molar equivalents) were introduced into a T-shaped mixer at 20°C using a syringe pump. The resulting mixture was introduced into a reaction tube 1 (inner diameter: 0.800 mm, length: 1061 mm, volume: 533 µL, reaction time: 10 sec) at the same temperature. The resulting mixture and a dichloromethane solution (flow rate: 1.20 mL/min) of an amino acid N-carboxy anhydride represented by L-Asp(Ot-Bu)-NCA (compound 3e; 0.324 M, 1.1 molar equivalents) were introduced into a T-shaped mixer at 20°C using a syringe pump. The resulting mixture was introduced into a reaction tube 2 (inner diameter: 0.800 mm, length: 72.9 mm, volume: 37 µL, reaction time: 0.50 sec) at the same temperature. The resulting mixture and a dichloromethane solution (flow rate: 2.00 mL/min) of N,N-diisopropylethylamine (0.233 M, 1.3 molar equivalents) and N-methylimidazole (0.194 M, 1.1 molar equivalents) were introduced into a T-shaped mixer at 20°C using a syringe pump. The resulting mixture was introduced into a reaction tube 3 (inner diameter: 0.800 mm, length: 2122 mm, volume: 1067 µL, reaction time: 10 sec) at the same temperature. After reaching a steady state over about 90 seconds, the resulting mixture was poured into a dichloromethane solution of the semi-pure pentapeptide (compound 7c; 1.0 molar equivalent) at room temperature for 33 seconds. After stirring at room temperature for 10 minutes, 1M hydrochloric acid (2 mL) was added for quenching. Water was further added, the aqueous layer was extracted with dichloromethane, and the organic layer was washed with a saturated aqueous NaHCO₃ solution and saturated brine, dried over sodium sulfate, filtered, and concentrated under vacuum at room temperature to obtain a semi-pure heptapeptide (compound 8a; SEQ ID NO: 4). The crude product was recrystallized from ethyl acetate and methanol. 10 wt% palladium carbon (25 wt%) was added at room temperature to a methanol solution containing the resulting semi-pure heptapeptide represented by Cbz-Gly-L-Asp(Ot-Bu)-L-Glu(Ot-Bu)-L-Glu(Ot-Bu)-L-Ser(t-Bu)-L-Leu-L-Ala-Ot-Bu (compound 8a; 1.0 molar equivalent). The resulting mixture was stirred at room temperature under a hydrogen atmosphere. After stirring for 2 hours, the resulting mixture was filtered through Celite and concentrated under vacuum at room temperature. The crude product was dissolved in a mixed solution of ethyl acetate and methanol, washed with 1M aqueous NaOH solution, water, and saturated brine, dried over sodium sulfate, filtered, and concentrated under vacuum at room temperature. A semi-pure heptapeptide represented by H-Gly-L-Asp(Ot-Bu)-L-Glu(Ot-Bu)-L-Glu(Ot-Bu)-L-Ser(t-Bu)-L-Leu-L-Ala-Ot-Bu (compound 8b; SEQ ID NO: 5) was obtained.

### Step 4: Synthesis of Octapeptide (Compound 9b)

An acetonitrile solution (flow rate: 1.20 mL/min) of an N-terminal protected amino acid represented by Boc-L-Lys(Boc)-OH (compound 1g; 0.333 M, 1.3 molar equivalents), N,N-diisopropylethylamine (0.333 M, 1.3 molar equivalents), and N-methylmorpholine (0.333 M, 1.3 molar equivalents), and an acetonitrile solution (flow rate: 2.00 mL/min) of methyl chloroformate (0.240 M, 1.5 molar equivalents) were introduced into a T-shaped mixer at 20°C using a syringe pump. The resulting mixture was introduced into a reaction tube 1 (inner diameter: 0.800 mm, length: 1061 mm, volume: 533 µL, reaction time: 10 sec) at the same temperature. After reaching a steady state over about 60 seconds, the resulting mixture was poured into an acetonitrile/dimethylformamide solution of the semi-pure heptapeptide (compound 8b) at room temperature for 20 seconds. After stirring at room temperature for 20 minutes, the reacted mixture was quenched with 1M hydrochloric acid (2 mL). Water was further added, the aqueous layer was extracted with dichloromethane, and the organic layer was washed with a saturated aqueous NaHCO₃ solution and saturated brine, dried over sodium sulfate, filtered, and concentrated under vacuum at room temperature to obtain a semi-pure protected octapeptide (compound 9a; SEQ ID NO: 6). The crude product was recrystallized from methanol. Trifluoroacetic acid was added at room temperature to an acetonitrile/dimethylformamide solution containing the resulting semi-pure octapeptide represented by Boc-L-Lys(Boc)-Gly-L-Asp(t-Bu)-L-Glu(t-Bu)-L-Glu(t-Bu)-L-Ser(t-Bu)-L-Leu-L-Ala-Ot-Bu (compound 9a; 1.0 molar equivalent), followed by stirring at room temperature. After stirring for 2.5 hours, the resulting mixture was filtered through Celite and concentrated under vacuum at room temperature to obtain a semi-pure octapeptide (compound 9b; SEQ ID NO: 7). By using just one silica gel column chromatography, a beefy meaty peptide (compound 9b), which is an octapeptide represented by H-L-Lys-Gly-L-Asp-L-Glu-L-Glu-L-Ser-L-Leu-L-Ala-OH, was obtained with a high purity of 98%.

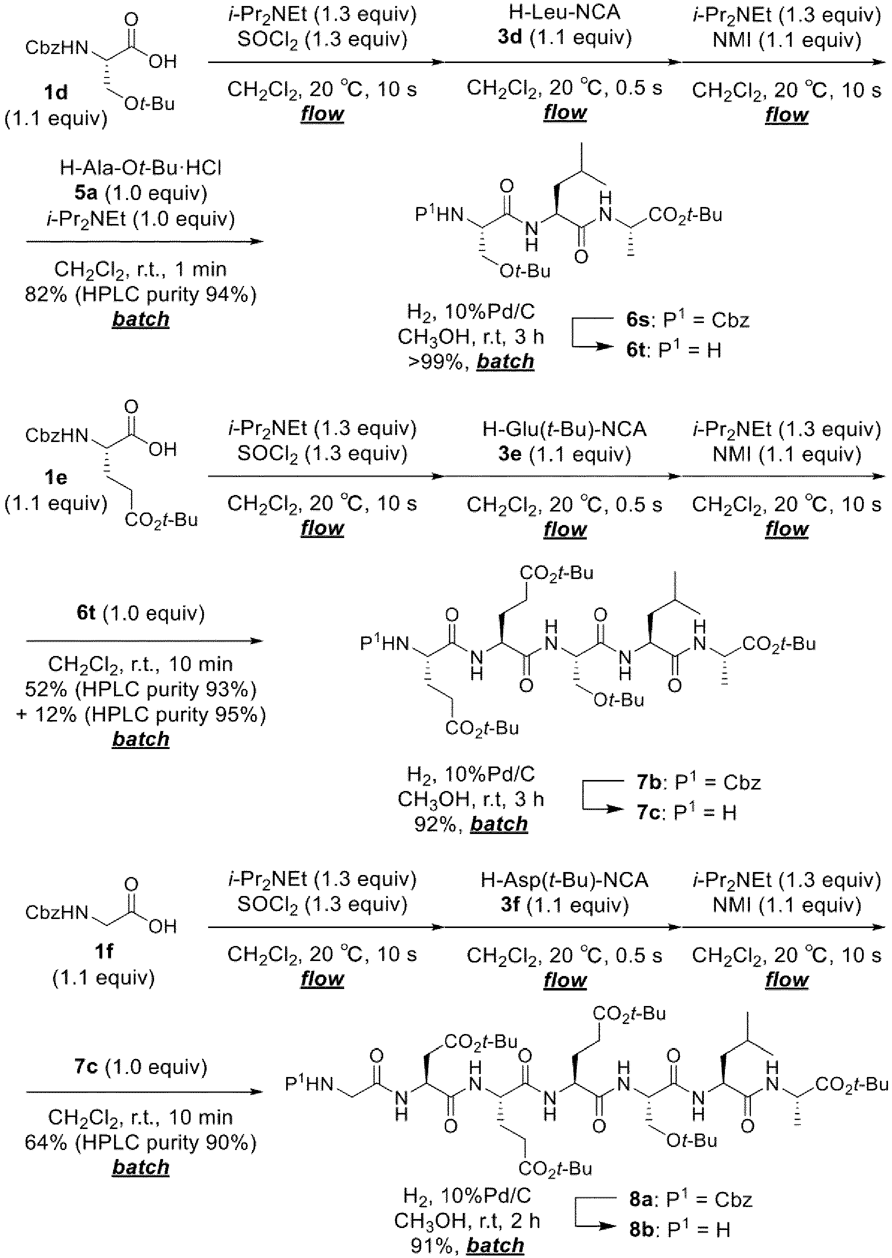

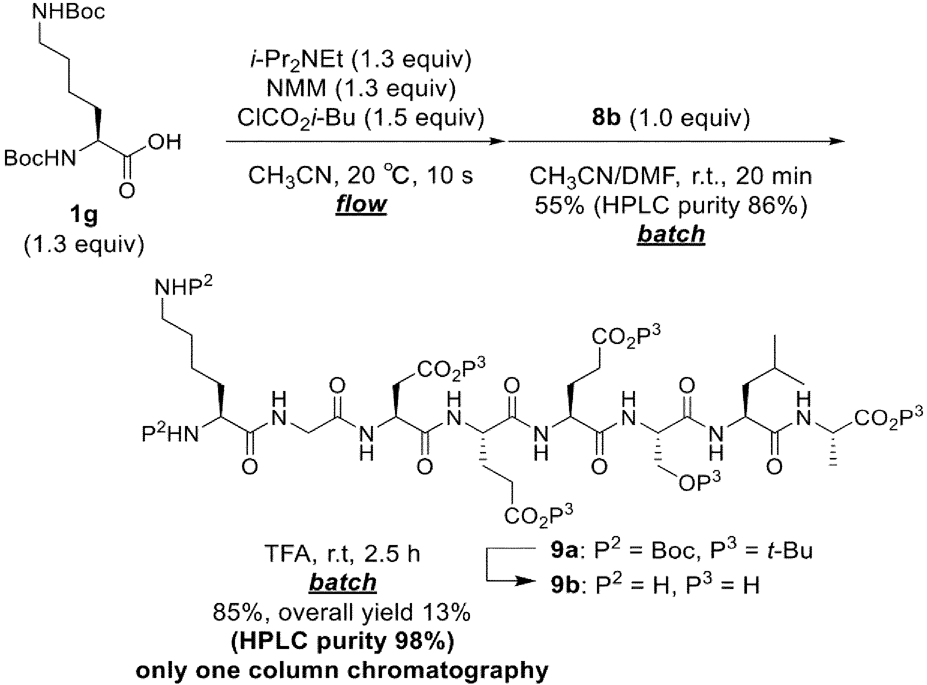

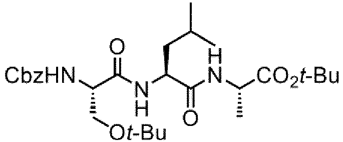

Cbz-L-Ser(t-Bu)-L-Leu-L-Ala-Ot-Bu (compound 6s):
HPLC conditions; COSMOSIL 5C₁₈-AR-II 4.6 mm I.D. × 150 mm, CH₃CN+0.1% formic acid/H₂O+0.1% formic acid, 0-20 min: 30 to 100%, 20-25 min: 100%, 25-27 min: 100 to 30%, 27-30 min: 30%, flow rate 1.0 mL/min, detection wavelength 254 nm, temperature 40°C, retention time: 14.7 min.

685.3 mg, 1.28 mmol, 82%, HPLC purity: 94%.

Colorless amorphous solid, ¹H NMR (400 MHz, CDCl₃): δ 7.38-7.33 (m, 5H), 6.93 (d, J = 7.8 Hz, 1H), 6.55 (d, J = 6.4 Hz, 1H), 5.96 (d, J = 4.8 Hz, 1H), 5.12 (s, 2H), 4.49-4.39 (m, 2H), 4.25 (brs, 1H), 3.85-3.83 (m, 1H), 3.43 (dd, J = 7.2, 8.8 Hz, 1H), 1.70-1.51 (m, 3H), 1.46 (s, 9H), 1.35 (d, J = 7.2 Hz, 3H), 1.18 (s, 9H), 0.93 (d, J = 6.8 Hz, 3H), 0.92 (d, J = 6.0 Hz, 3H) ppm; HRMS (ESI): calcd for [C₂₈H₄₅N₃O₇+H]⁺536.3330, found 536.3330, [C₂₈H₄₅N₃O₇+Na]⁺558.3150, found 558.3152.

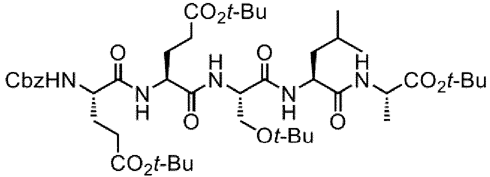

Cbz-L-Glu(Ot-Bu)-L-Glu(Ot-Bu)-L-Ser(t-Bu)-L-Leu-L-Ala-Ot-Bu (compound 7b):
HPLC conditions; COSMOSIL 5C₁₈-AR-II 4.6 mm I.D. × 150 mm, CH₃CN+0.1% formic acid/H₂O+0.1% formic acid, 0-20 min: 50 to 100%, 20-25 min: 100%, 25-30 min: 50%, flow rate 1.0 mL/min, detection wavelength 254 nm, temperature 40°C, retention time: 14.5 min. 295.1 mg, 0.326 mmol, 52%, HPLC purity: 93%, 69.5 mg, 0.076 mmol, 12%, HPLC purity: 95%.

Colorless amorphous solid, ¹H NMR (400 MHz, CDCl₃): δ 7.82 (d, J = 4.4 Hz, 1H), 7.36-7.31 (m, 5H), 7.11 (d, J = 8.0 Hz, 2H), 6.81 (d, J = 7.6 Hz, 1H), 5.79 (d, J = 5.6 Hz, 1H), 5.16 (d, J = 12.4 Hz, 1H), 5.08 (d, J = 12.4 Hz, 1H), 4.53-4.47 (m, 1H), 4.43-4.34 (m, 2H), 4.21-4.13 (m, 2H), 3.85 (dd, J = 3.7, 8.8 Hz, 1H), 3.52 (dd, J = 4.8, 8.8 Hz, 1H), 2.53-2.29 (m, 4H), 2.19-1.91 (m, 4H), 1.78-1.61 (m, 3H), 1.45 (s, 9H), 1.44 (s, 9H), 1.43 (s, 9H), 1.35 (d, J = 7.3 Hz, 3H), 1.17 (s, 9H), 0.92 (d, J = 6.4 Hz, 3H), 0.90 (d, J = 6.4 Hz, 3H) ppm; HRMS (ESI): calcd for [C₄₆H₇₅N₅O₁₃+H]⁺ 906.5434, found 906.5432, [C₄₆H₇₅N₅O₁₃+Na]⁺ 928.5254, found 928.5254.

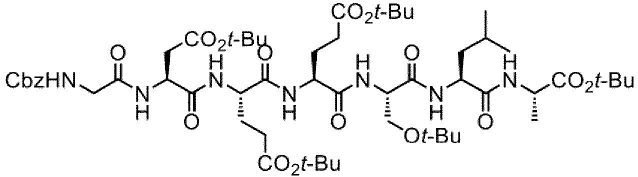

Cbz-L-Gly-L-Asp(Ot-Bu)-L-Glu(Ot-Bu)-L-Glu(Ot-Bu)-L-Ser(t-Bu)-L-Leu-L-Ala-Ot-Bu (compound 8a):
HPLC conditions; COSMOSIL 5C₁₈-AR-II 4.6 mm I.D. × 150 mm, CH₃CN+0.1% formic acid/H₂O+0.1% formic acid, 0-20 min: 50 to 100%, 20-25 min: 100%, 25-30 min: 50%, flow rate 1.0 mL/min, detection wavelength 254 nm, temperature 40°C, retention time: 14.5 min. 135.6 mg, 0.120 mmol, 64%, HPLC purity: 90%.

Colorless amorphous solid, ¹H NMR (400 MHz, DMSO-d₆): δ 8.18 (d, J = 7.6 Hz, 1H), 8.11 (d, J = 6.8 Hz, 1H), 7.97-7.94 (m, 2H), 7.87 (d, J = 7.6 Hz, 1H), 7.74 (d, J = 8.8 Hz, 1H), 7.48 (brt, J = 6.0 Hz, 1H), 7.35-7.32 (m, 5H), 5.02 (s, 2H), 4.63-4.58 (m, 1H), 4.40-4.21 (m, 4H), 4.11-4.08 (m, 1H), 3.64 (d, J = 6.0 Hz, 2H), 3.46-3.41 (m, 1H), 3.33 (overlapped with H₂O, 1H), 2.64 (dd, J = 4.8, 16.0 Hz, 1H), 2.50 (overlapped with DMSO, 1H), 2.28-2.14 (m, 4H), 1.87 (brs, 2H), 1.78-1.70 (m, 2H), 1.66-1.59 (m, 1H), 1.47-1.34 (m, 38H), 1.23 (d, J = 7.2 Hz, 3H), 1.09 (s, 9H), 0.87 (d, J = 6.8 Hz, 3H), 0.84 (d, J = 6.4 Hz, 3H) ppm; HRMS (ESI): calcd for [C₅₆H₉₁N₇O₁₇+H]⁺ 1134.6544, found 1134.6542, [C₅₆H₉₁N₇O₁₇+Na]⁺ 1156.6364, found 1156.6363.

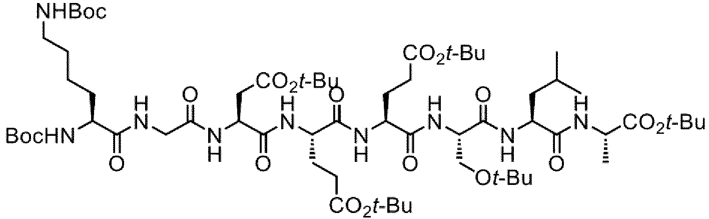

Boc-L-Lys(Boc)-Gly-L-Asp(Ot-Bu)-L-Glu(Ot-Bu)-L-Glu(Ot-Bu)-L-Ser(t-Bu)-L-Leu-L-Ala-Ot-Bu (compound 9a):
HPLC conditions; COSMOSIL 5C₁₈-AR-II 4.6 mm I.D. × 150 mm, CH₃CN+0.1% formic acid/H₂O+0.1% formic acid, 0-20 min: 50 to 100%, 20-25 min: 100%, 25-30 min: 50%, flow rate 1.0 mL/min, detection wavelength 254 nm, temperature 40°C, retention time: 15.9 min. 76.3 mg, 0.058 mmol, 55%, HPLC purity: 86%.

Colorless amorphous solid, ¹H NMR (400 MHz, DMSO-d₆): δ 8.11 (d, J = 6.8 Hz, 1H), 8.10 (d, J = 8.4 Hz, 1H), 8.04 (brt, J = 5.2 Hz, 1H), 7.95 (d, J = 6.8 Hz, 1H), 7.94 (d, J = 6.8 Hz, 1H), 7.86 (d, J = 8.0 Hz, 1H), 7.74 (d, J = 8.8 Hz, 1H), 6.91 (d, J = 7.2 Hz, 1H), 6.75 (brt, J = 4.8 Hz, 1H), 4.65-4.59 (m, 1H), 4.38-4.21 (m, 4H), 4.11-4.07 (m, 1H), 3.88-3.83 (m, 1H), 3.77-3.65 (m, 2H), 3.51-3.40 (m, 2H), 2.90-2.85 (m, 2H), 2.66 (dd, J = 5.2, 16.0 Hz, 1H), 2.43 (dd, J = 8.2, 16.0 Hz, overlapped with DMSO, 1H), 2.28-2.13 (m, 4H), 1.92-1.83 (m, 2H), 1.77-1.69 (m, 2H), 1.66-1.58 (m, 1H), 1.47-1.33 (m, 62H), 1.24 (d, J = 7.2 Hz, 3H), 1.09 (s, 9H), 0.87 (d, J = 6.4 Hz, 3H), 0.84 (d, J = 6.8 Hz, 3H) ppm; HRMS (ESI) : calcd for [C₆₄H₁₁₃N₉O₂₀+Na]⁺ 1350.7994, found 1350.7993.

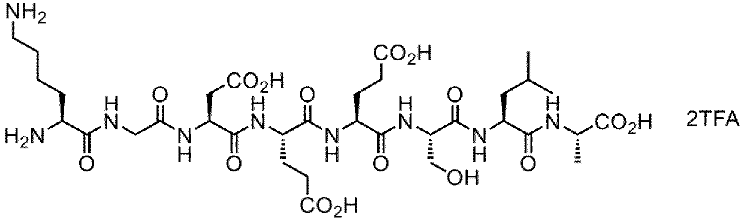

H-L-Lys-L-Gly-L-Asp-L-Glu-L-Glu-L-Ser-L-Leu-L-Ala-OH·TFA (compound 9b): Chromatographic conditions (reverse phase): Biotage Sfar Bio C18 Duo 25 g, 0.1% TFA in H₂O : 0.1% TFA in CH₃CN = 100 : 0 to 70 : 30, flow rate 5 mL/min, detection wavelength 215 nm and Biotage Sfar Bio C18 Duo 10 g, 0.1% TFA in H₂O : 0.1% TFA in CH₃CN = 100 : 0 to 70 : 30, flow rate 2 mL/min, detection wavelength 215 nm.

HPLC conditions; COSMOSIL 5C₁₈-AR-II 4.6 mm I.D. × 150 mm, CH₃CN+0.1% TFA/H₂O+0.1% TFA, 0-15 min: 0 to 20%, 15-20 min: 20%, 20-25 min: 0%, flow rate 1.0 mL/min, detection wavelength 215 nm, temperature 40°C, retention time: 13.1 min.

36.4 mg, 0.034 mmol, 85% (total yield 13%), HPLC purity: 98%. Colorless amorphous solid, ¹H NMR (400 MHz, D₂O): δ 4.79 (overlapped with H₂O, 1H), 4.44 (t, J = 6.0 Hz, 1H), 4.41-4.33 (m, 4H), 4.08 (t, J = 6.8 Hz, 1H), 4.04 (s, 2H), 3.85 (d, J = 6.0 Hz, 2H), 3.01 (t, J = 8.0 Hz, 2H), 2.98-2.94 (m, 1H), 2.89 (dd, J = 6.8, 17.2 Hz, 1H), 2.51-2.48 (m, 4H), 2.18-2.11 (m, 2H), 2.06-1.98 (m, 2H), 1.96-1.92 (m, 2H), 1.76-1.63 (m, 5H), 1.53-1.47 (m, 2H), 1.42 (d, J = 7.2 Hz, 3H), 0.93 (d, J = 5.6 Hz, 3H), 0.88 (d, J = 6.0 Hz, 3H) ppm; ¹³C NMR (100 MHz, D₂O, CH₃CN was used as internal standard): δ 176.1, 176.0, 175.2, 173.2, 173.1, 172.32, 172.27, 171.5, 170.5, 169.8, 169.4, 161.9 (q, JC-_{F} = 35.2 Hz), 115.5 (q, JC-_{F} = 290.8 Hz), 60.0, 54.6, 52.5, 52.3, 52.1, 51.3, 49.1, 47.7, 41.5, 38.8, 38.1, 34.4, 29.4, 29.0, 25.4, 25.0, 24.9, 23.3, 21.3, 20.3, 19.7, 15.2 ppm; HRMS (ESI): calcd for [C₃₄H₅₇N₉O₁₆+H]⁺ 848.3996, found 848.3998, [C₃₄H₅₇N₉O₁₆+Na]⁺ 870.3815, found 870.3815.

### Example 9

### Synthesis of Tripeptides (Compounds epi-6i-1, epi-6i-2, epi-6o-1, and epi-6i-2)

Next, epimers of the respective tripeptides (compounds 6i and 6o) obtained in Example 6 were synthesized by the following method (Table 6).

An N-terminal protected amino acid represented by Fmoc-Cys(t-Bu)-OH (compound 1h) or an N-terminal protected amino acid represented by Cbz-Ala-OH (compound 1i) (0.300 M, 1.00 molar equivalent), and N,N-diisopropylethylamine (0.360 M, 1.2 molar equivalents) in dichloromethane (flow rate: 1.20 mL/min), and a dichloromethane solution (flow rate: 2.00 mL/min) of thionyl chloride (0.216 M, 1.20 molar equivalents) were introduced into a T-shaped mixer at 20°C using a syringe pump. The resulting mixture 1 was introduced into a reaction tube 1 (inner diameter: 0.800 mm, length: 1061 mm, volume: 533 µL, reaction time: 10 sec) at the same temperature. The resulting mixture 1 and a dichloromethane solution (flow rate: 1.20 mL/min) of an amino acid N-carboxy anhydride represented by Phe-NCA (compound 3g) or an amino acid N-carboxy anhydride represented by Cys(Bn)-NCA (compound 3h) (0.300 M, 1.00 molar equivalent) were introduced into a T-shaped mixer at 20°C using a syringe pump. The resulting mixture 2 was introduced into a reaction tube 2 (inner diameter: 0.800 mm, length: 72.9 mm, volume: 37 µL, reaction time: 0.50 sec) at the same temperature. The resulting mixture 2 and a dichloromethane solution (flow rate: 2.00 mL/min) of N,N-diisopropylethylamine (0.216 M, 1.2 molar equivalents) and N-methylimidazole (0.180 M, 1.00 molar equivalent) were introduced into a T-shaped mixer at 20°C using a syringe pump. The resulting mixture 3 was introduced into a reaction tube 3 (inner diameter: 0.800 mm, reaction time: 10 sec or 15 sec) at the same temperature. The resulting mixture 3 and a dichloromethane solution (flow rate: 1.20 mL/min) of a C-terminal protected amino acid represented by H-L-Ala-Ot-Bu (compound 5c; 0.360 M, 1.20 molar equivalents) were introduced into a T-shaped mixer at 20°C using a syringe pump. The resulting mixture 4 was introduced into a reaction tube 4 (inner diameter: 0.800 mm, length: 2519 mm, volume: 1267 µL, reaction time: 10 sec) at the same temperature. After reaching a steady state over 90 seconds, the reacted mixture 4 was poured into 1M hydrochloric acid (1.5 mL) and dichloromethane (8 mL) at room temperature for 25 seconds. The aqueous layer was extracted with dichloromethane, and the organic layer was washed with a saturated aqueous NaHCO₃ solution and saturated brine, dried over magnesium sulfate, filtered, and concentrated under vacuum at room temperature. The residues were purified by recrystallization or washing, and each was identified by HPLC-UV analysis using a chiral column. The results are shown in Figs. 2 and 3. The purification conditions, yield, and yield are as shown in Table 6 (Examples 9-1 to 9-4). The results of Figs. 2 and 3 reveal that the compounds obtained by the method of the present invention do not contain separately synthesized epimers, and that epimerization is very unlikely to occur in the method of the present invention. In addition, since cysteine is known to be an amino acid that is easily epimerized, it is clear that the method of the present invention can also be applied to amino acids that are easily epimerized.

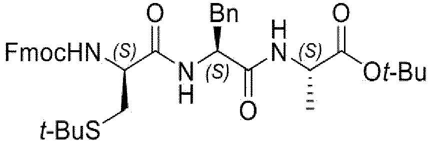

Fmoc-D-Cys(t-Bu)-L-Phe-L-Ala-Ot-Bu (compound epi-6i-1):
White solid, mp 152-156°C, IR (neat): 3283, 1732, 1644, 1550, 1226, 1149, 758, 741 cm⁻¹; [α]²⁴_{D} = -0.061 (c 0.95, CHCl₃) ; ¹H NMR (400 MHz, CDCl₃): δ 7.76 (d, J = 7.2 Hz, 2H), 7.58 (d, J = 7.2 Hz, 2H), 7.39 (dd, J = 7.2, 7.2 Hz, 2H), 7.30 (dd, J = 7.2, 7.2 Hz, 2H), 7.26-7.13 (m, 5H), 6.86 (d, J = 6.4 Hz, 1H), 6.47 (d, J = 7.2 Hz, 1H), 5.72 (brs, 1H), 4.72-4.67 (m, 1H), 4.40-4.28 (m, 4H), 4.20 (t, J = 6.8 Hz, 1H), 3.15 (dd, J = 6.4, 13.6 Hz, 1H), 3.07 (dd, J = 6.8, 13.6 Hz, 1H), 2.94 (dd, J = 6.0, 12.8 Hz, 1H), 2.76 (dd, J = 6.8, 12.8 Hz, 1H), 1.42 (s, 9H), 1.31 (d, J = 8.4 Hz, 12H) ppm; ¹³C NMR (100 MHz, CDCl₃): δ171.6, 170.2, 169.8, 156.1, 143.9, 143.8, 141.4, 136.3, 129.5, 128.7, 127.8, 127.2, 125.3, 120.1, 82.1, 67.5, 55.2, 54.5, 49.0, 47.2, 43.3, 38.2, 31.0, 30.9, 28.0, 18.5 ppm; HRMS (ESI): calcd for [C₃₈H₄₇N₃O₆S₁+Na]⁺ 696.3078, found 696.3080.

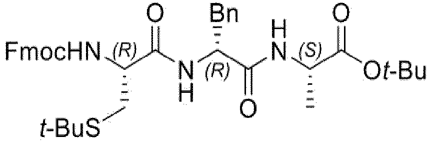

Fmoc-L-Cys(t-Bu)-D-Phe-L-Ala-Ot-Bu (compound epi-6i-2):
White solid, mp 125-127°C, IR (neat): 3352, 1731, 1657, 1524, 1235, 1151, 758, 741 cm⁻¹; [α]²⁴_{D} = -7.5 (c 0.84, CHCl₃) ; ¹H NMR (400 MHz, CDCl₃): δ 7.76 (d, J = 7.2 Hz, 2H), 7.58 (d, J = 7.2 Hz, 2H), 7.40 (dd, J = 7.2, 7.2 Hz, 2H), 7.31-7.19 (m, 7H), 6.86 (d, J = 6.8 Hz, 1H), 6.39 (d, J = 6.4 Hz, 1H), 5.73 (brs, 1H), 4.72-4.69 (m, 1H), 4.42-4.19 (m, 5H), 3.15 (dd, J = 6.4, 13.2 Hz, 1H), 3.07 (dd, J = 7.6, 13.2 Hz, 1H), 2.97 (dd, J = 6.0, 12.8 Hz, 1H), 2.78 (dd, J = 7.2, 12.8 Hz, 1H), 1.41 (s, 9H), 1.32 (s, 9H), 1.21 (d, J = 7.2 Hz, 3H) ppm; ¹³C NMR (100 MHz, CDCl₃): δ171.9, 170.2, 169.6, 156.1, 143.9, 143.8, 141.4, 136.5, 129.5, 128.8, 127.9, 127.2, 125.3, 120.1, 82.1, 67.5, 55.3, 54.6, 48.8, 47.2, 43.3, 38.3, 31.0, 30.8, 18.3 ppm; HRMS (ESI) : calcd for [C₃₈H₄₇N₃O₆S₁+Na]⁺ 696.3078, found 696.3077.

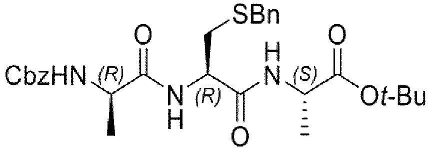

Cbz-D-Ala-L-Cys(Bn)-L-Ala-Ot-Bu (compound epi-6o-1):
Yellow oil, IR (neat): 3287, 1732, 1645, 1541, 1508, 1234, 1148, 698 cm⁻¹; [α]²⁴_{D} = +11.1 (c 1.16, CHCl₃) ; ¹H NMR (400 MHz, CDCl₃) : δ 7.32-7.22 (m, 10H), 7.05 (d, J = 6.4 Hz, 1H), 6.95 (d, J = 7.6 Hz, 1H), 5.52 (d, J = 7.6 Hz, 1H), 5.13 (d, J = 12.4 Hz, 1H), 5.05 (d, J = 12.4 Hz, 1H) 4.57 (d, J = 6.0 Hz, 1H), 4.42-4.36 (m, 1H), 4.25-4.22 (m, 1H), 3.74 (s, 2H), 2.89 (dd, J = 4.0, 13.2 Hz, 1H), 2.73 (dd, J = 6.4, 13.2 Hz, 1H), 1.44 (s, 9H), 1.37 (d, J = 7.2 Hz, 3H), 1.35 (d, J = 7.6 Hz, 3H) ppm; ¹³C NMR (100 MHz, CDCl₃): δ172.5, 171.6, 169.5, 156.1, 138.1, 136.2, 129.2, 128.7, 128.6, 128.3, 128.2, 127.3, 82.1, 67.2, 52.2, 50.9, 49.1, 36.6, 33.7, 28.0, 18.7, 18.3 ppm; HRMS (ESI): calcd for [C₂₈H₃₇N₃O₆S₁+Na]⁺ 566.2295, found 566.2295.

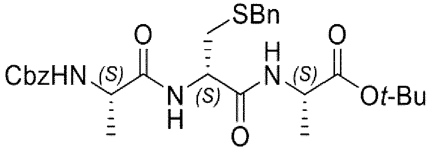

Cbz-L-Ala-D-Cys(Bn)-L-Ala-Ot-Bu (compound epi-6o-2):
Yellow oil, IR (neat): 3296, 1732, 1717, 1647, 1540, 1523, 1241, 1150, 698 cm⁻¹; [α]²⁴_{D} = 1.25 (c 1.41, CHCl₃); ¹H NMR (400 MHz, CDCl₃): δ 7.33-7.22 (m, 10H), 7.06 (d, J = 6.4 Hz, 1H), 6.90 (d, J = 7.6 Hz, 1H), 5.46 (d, J = 6.4 Hz, 1H), 5.43 (d, J = 12.4 Hz, 1H), 5.04 (d, J = 12.4 Hz, 1H), 4.60 (d, J = 6.4 Hz, 1H), 4.44-4.37 (m, 1H), 4.21-4.14 (m, 1H), 3.73 (s, 2H), 2.95 (dd, J = 5.2, 14.0 Hz, 1H), 2.74 (dd, J = 6.4, 14.0 Hz, 1H), 1.44 (s, 9H), 1.38 (d, J = 6.8 Hz, 3H), 1.35 (d, J = 7.2 Hz, 3H) ppm; ¹³C NMR (100 MHz, CDCl₃): δ172.6, 171.9, 169.5, 156.2, 138.2, 136.2, 129.2, 128.74, 128.67, 128.4, 128.2, 127.3, 82.1, 67.2, 52.1, 51.1, 49.1, 36.5, 33.3, 28.1, 18.3, 18.2 ppm; HRMS (ESI): calcd for [C₂₈H₃₇N₃O₆S₁+Na]⁺ 566.2295, found 566.2292.

### Comparative Example 1

### Sequential Synthesis of Tripeptide (Compound epi-6a-1)

Next, using a conventional technique, a tripeptide (compound epi-6a-1) was synthesized by sequentially extending amino acid residues one by one. Compound epi-6a-1 is an epimer of the tripeptide (compound 6a) obtained in Example 4.

To a dichloromethane solution of an N-terminal protected amino acid represented by Fmoc-L-Phe-OH (compound 1a; 1.0 molar equivalent) and N-methylmorpholine (1.1 molar equivalent), isobutyl chloroformate (1.0 molar equivalent) was added at -20°C. After stirring at -20°C for 1 minute, a C-terminal protected amino acid represented by HL-Ala-Ot-Bu (1.1 molar equivalents) and N,N-diisopropylethylamine (1.1 molar equivalents) were added at -20°C. After stirring at room temperature for 15 minutes, the resulting mixture was poured into dichloromethane and 1M hydrochloric acid at the same temperature. The aqueous layer was extracted with dichloromethane, and the organic layer was washed with a saturated aqueous NaHCO₃ solution and saturated brine, dried over magnesium sulfate, filtered, and concentrated under vacuum at room temperature. A step of purifying the residue by silica gel column chromatography was required. An N-terminal protected dipeptide represented by Fmoc-L-Phe-L-Ala-Ot-Bu (compound S3) was obtained. The purification conditions, yield, and yield are as shown in Table 6 (Comparative Example 1-1).

Diazabicycloundecene (1.0 molar equivalent) was added to a dichloromethane solution of compound S3 (1.0 molar equivalent) at 0°C. The reaction was monitored by thin-layer chromatography. After the reaction was completed, the resulting solution was purified by silica gel column chromatography. An N-terminal unprotected dipeptide represented by H-L-Phe-L-Ala-Ot-Bu (compound S5) was obtained. The purification conditions, yield, and yield are as shown in Table 6 (Comparative Example 1-2).

Isobutyl chloroformate (1.1 molar equivalents) was added at -20°C to a dichloromethane solution of an N-terminal protected amino acid represented by Fmoc-D-Phe-OH (1.0 molar equivalent) and N-methylmorpholine (1.1 molar equivalents). After stirring at -20°C for 1 minute, the N-terminal unprotected dipeptide represented by H-L-Phe-L-Ala-Ot-Bu (compound S5; 1.1 molar equivalents) and N,N-diisopropylethylamine (1.1 molar equivalents) were added at -20°C. After stirring at room temperature for 15 minutes, the resulting mixture was poured into dichloromethane and 1M hydrochloric acid at the same temperature. The aqueous layer was extracted with dichloromethane, and the organic layer was washed with a saturated aqueous NaHCO₃ solution and saturated brine, dried over magnesium sulfate, filtered, and concentrated under vacuum at room temperature. A step of purifying the residue by silica gel column chromatography was required. The resulting tripeptide represented by Fmoc-D-Phe-L-Phe-L-Ala-Ot-Bu (compound epi-6a-1) was isolated by silica gel column chromatography, and the crude products were purified by recrystallization and each identified by HPLC-UV analysis using a chiral column. The results are shown in Fig. 4. The purification conditions, yield, and yield are as shown in Table 6 (Comparative Example 1-3).

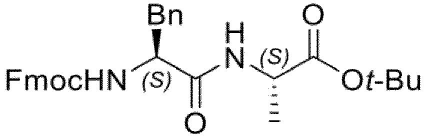

Fmoc-L-Phe-L-Ala-Ot-Bu (compound S3: Org. Biomol. Chem. 2022, 20, 3303-3310.):
White solid, ¹H NMR (400 MHz, CDCl₃): δ 7.69 (d, J = 7.2 Hz, 2H), 7.51-7.46 (m, 2H), 7.35-7.31 (m, 2H), 7.24-7.12 (m, 7H), 7.02 (d, J = 6.8 Hz, 1H), 5.96 (d, J = 8.0 Hz, 1H), 4.64-4.60 (m, 1H), 4.43-4.34 (m, 2H), 4.20-4.15 (m, 1H), 4.10 (t, J = 6.8 Hz, 1H), 3.12-3.02 (m, 2H), 1.42 (s, 9H), 1.29 (d, J = 7.2 Hz, 3H) ppm; ¹³C NMR (100 MHz, CDCl₃): δ171.7, 170.8, 156.0, 143.8, 143.7, 141.2, 136.5, 129.4, 128.5, 127.6, 127.0, 126.9, 125.15, 125.07, 119.9, 81.8, 67.1, 56.0, 48.7, 47.0, 38.8, 27.9, 18.3 ppm.

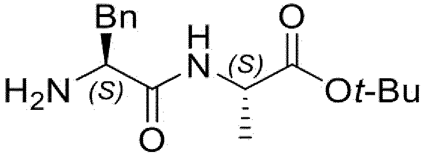

H-L-Phe-L-Ala-Ot-Bu (compound S5: ACS Catal. 2018, 8, 2181-2187.) :
Colorless oil, ¹H NMR (400 MHz, CDCl₃): δ 7.78 (d, J = 7.6 Hz, 1H), 7.33-7.29 (m, 2H), 7.25-7.21 (m, 3H), 4.50-4.42 (m, 1H), 3.62 (dd, J = 3.6, 9.6 Hz, 1H), 3.24 (dd, J = 3.6, 14.0 Hz, 1H), 2.72 (dd, J = 9.2, 14.0 Hz, 1H), 1.46 (s, 11H), 1.36 (d, J = 7.6 Hz, 3H) ppm; ¹³C NMR (100 MHz, CDCl₃): δ173.8, 172.2, 137.8, 129.4, 128.7, 126.8, 81.8, 56.3, 48.3, 41.0, 28.0, 18.6 ppm.

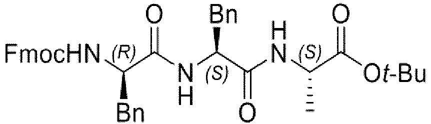

Fmoc-D-Phe-L-Phe-L-Ala-Ot-Bu (compound epi-6a-1):
White solid, mp 97-101°C, IR (neat): 3283, 1732, 1644, 1541, 1245, 1224, 1148, 757, 741 cm⁻¹; [α]²⁴_{D} = +5.20 (c 2.92, CHCl₃) ; ¹H NMR (400 MHz, CDCl₃): δ 7.72-7.70 (m, 2H), 7.48 (dd, J = 8.0, 8.0 Hz, 2H), 7.34 (dd, J = 7.2, 8.0 Hz, 2H), 7.25-6.96 (m, 14H), 5.94 (d, J = 8.4 Hz, 1H), 4.84-4.81 (m, 1H), 4.52 (d, J = 7.2 Hz, 1H), 4.38-4.31 (m, 2H), 4.19-4.08 (m, 2H), 3.05-2.87 (m, 4H), 1.39 (s, 9H), 1.26 (d, J = 7.2 Hz, 3H) ppm; ¹³C NMR (100 MHz, CDCl₃): δ171.6, 171.2, 170.3, 156.0, 143.9, 143.8, 141.3, 136.5, 136.4, 129.5, 128.6, 127.7, 127.1, 127.0, 125.2, 125.1, 120.0, 81.9, 67.2, 56.4, 54.2, 48.9, 47.1, 38.8, 38.3, 28.0, 18.3 ppm; HRMS (ESI): calcd for [C₄₀H₄₃N₃O₆+Na]⁺684.3044, found 684.3047.

### Comparative Example 2

### Sequential Synthesis of Tripeptide (Compound epi-6a-2)

Amino acid residues were sequentially extended one by one in the same manner as in Comparative Example 1 to synthesize a tripeptide (epi-6a-2). Compound epi-6a-2 is an epimer of the tripeptide (compound 6a) obtained in Example 4.

Isobutyl chloroformate (1.0 molar equivalent) was added at -20°C to a dichloromethane solution of an N-terminal protected amino acid represented by Fmoc-D-Phe-OH (1.0 molar equivalent) and N-methylmorpholine (1.1 molar equivalents). After stirring at -20°C for 1 minute, a C-terminal protected amino acid represented by H-L-Ala-Ot-Bu (1.1 molar equivalents) and N,N-diisopropylethylamine (1.1 molar equivalents) were added at -20°C. After stirring at room temperature for 15 minutes, the resulting mixture was poured into dichloromethane and 1M hydrochloric acid at the same temperature. The aqueous layer was extracted with dichloromethane, and the organic layer was washed with a saturated aqueous NaHCO₃ solution and saturated brine, dried over magnesium sulfate, filtered, and concentrated under vacuum at room temperature. A step of purifying the residue by silica gel column chromatography was required. An N-terminal protected dipeptide represented by Fmoc-D-Phe-L-Ala-Ot-Bu (compound S4) was obtained. The purification conditions, yield, and yield are as shown in Table 6 (Comparative Example 2-1).

Diazabicycloundecene (1.0 molar equivalent) was added at 0°C to a dichloromethane solution of compound S4 (1.0 molar equivalent). The reaction was monitored by thin-layer chromatography. After the reaction was completed, the resulting solution was purified by silica gel column chromatography. An N-terminal unprotected dipeptide represented by H-D-Phe-L-Ala-Ot-Bu (compound S6) was obtained. The purification conditions, yield, and yield are as shown in Table 6 (Comparative Example 2-2).

Isobutyl chloroformate (1.1 molar equivalents) was added at -20°C to a dichloromethane solution of an N-terminal protected amino acid represented by Fmoc-L-Phe-OH (1.0 molar equivalent) and N-methylmorpholine (1.1 molar equivalents). After stirring at -20°C for 1 minute, an N-terminal unprotected dipeptide represented by H-D-Phe-L-Ala-Ot-Bu (1.1 molar equivalents) and N,N-diisopropylethylamine (1.1 molar equivalents) were added at -20°C. After stirring at room temperature for 15 minutes, the resulting mixture was poured into dichloromethane and 1M hydrochloric acid at the same temperature. The aqueous layer was extracted with dichloromethane, and the organic layer was washed with a saturated aqueous NaHCO₃ solution and saturated brine, dried over magnesium sulfate, filtered, and concentrated under vacuum at room temperature. A step of purifying the residue by silica gel column chromatography was required. The resulting tripeptide represented by Fmoc-L-Phe-D-Phe-L-Ala-Ot-Bu (compound epi-6a-2) was isolated by silica gel column chromatography, and the crude products were purified by recrystallization and each identified by HPLC-UV analysis using a chiral column. The results are shown in Fig. 4. The purification conditions, yield, and yield are as shown in Table 6 (Comparative Example 2-3).

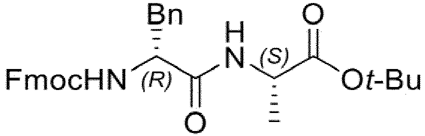

Fmoc-D-Phe-L-Ala-Ot-Bu (compound S4):
White solid, mp 133-135°C, IR (neat): 3299, 1732, 1716, 1655, 1540, 1450, 1253, 1147, 757, 741 cm⁻¹; [α]²⁴_{D} = +18.5 (c 2.38, CHCl₃); ¹H NMR (400 MHz, CDCl₃): δ 7.75 (d, J = 7.6 Hz, 2H), 7.53 (dd, J = 7.2, 7.2 Hz, 2H), 7.38 (dd, J = 7.2, 7.2 Hz, 2H), 7.31-7.20 (m, 7H), 6.32 (d, J = 4.8 Hz, 1H), 5.55 (d, J = 7.2 Hz, 1H), 4.47-4.31 (m, 4H), 4.18 (t, J = 7.2 Hz, 1H), 3.15-3.01 (m, 2H), 1.41 (s, 9H), 1.20 (d, J = 7.2 Hz, 3H) ppm; ¹³C NMR (100 MHz, CDCl₃): δ171.8, 170.1, 155.9, 143.9, 143.8, 141.4, 136.5, 129.5, 128.8, 127.8, 127.2, 125.2, 120.1, 82.2, 67.2, 56.3, 48.7, 47.2, 39.1, 28.0, 18.4 ppm; HRMS (ESI): calcd for [C₃₁H₃₄N₂O₅+Na]⁺ 537.2360, found 537.2363.

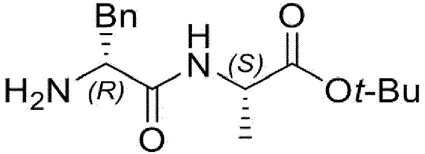

H-D-Phe-L-Ala-Ot-Bu (compound S6: ACS Catal. 2018, 8, 2181-2187.) :
Colorless oil, ¹H NMR (400 MHz, CDCl₃): δ 7.66 (d, J = 7.6 Hz, 1H), 7.34-7.30 (m, 2H), 7.26-7.22 (m, 3H), 4.50-4.43 (m, 1H), 3.59 (dd, J = 4.4, 10.0 Hz, 1H), 3.30 (dd, J = 4.0, 13.6 Hz, 1H), 2.66 (dd, J = 9.6, 13.6 Hz, 1H), 1.47 (s, 11H), 1.36 (d, J = 7.2 Hz, 3H) ppm; ¹³C NMR (100 MHz, CDCl₃): δ173.9, 172.3, 138.3, 129.4, 128.8, 126.9, 81.9, 56.8, 48.4, 41.2, 28.1, 18.7 ppm.

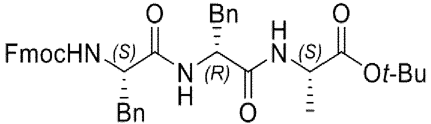

Fmoc-L-Phe-D-Phe-L-Ala-Ot-Bu (compound epi-6a-2):
Colorless solid, mp 138-142°C, IR (neat): 3299, 1732, 1716, 1655, 1540, 1450, 1253, 1147, 757, 741 cm⁻¹; [α]²⁴_{D} = +1.85 (c 3.19, CHCl₃); ¹H NMR (400 MHz, CDCl₃): δ 7.74 (d, J = 7.6 Hz, 2H), 7.51 (dd, J = 7.6, 7.6 Hz, 2H), 7.38 (dd, J = 7.6, 7.6 Hz, 2H), 7.29-7.13 (m, 10H), 7.24 (d, J = 6.8 Hz, 2H), 6.65-6.60 (m, 2H), 5.67 (d, J = 7.6 Hz, 1H), 4.71 (d, J = 6.4 Hz, 1H), 4.40-4.30 (m, 3H), 4.24-4.12 (m, 2H), 3.04-3.02 (m, 3H), 2.80 (dd, J = 8.0, 13.2 Hz, 1H), 1.38 (s, 9H), 1.15 (d, J = 7.2 Hz, 3H) ppm; ¹³C NMR (100 MHz, CDCl₃): δ171.8, 170.9, 169.7, 156.1, 143.8, 141.4, 136.4, 129.5, 128.8, 128.7, 127.8, 127.2, 127.1, 125.3, 125.2, 120.1, 82.0, 67.3, 56.7, 54.2, 48.7, 47.2, 38.6, 38.2, 28.0, 18.2 ppm; HRMS (ESI): calcd for [C₄₀H₄₃N₃O₆+Na]⁺ 684.3044, found 684.3046.

**Table 6**

| Example | Compound | Silica gel chromatography | Recrystallization | Washing | Retention time (s) | Yield (mg, mmol) | Yield (%) |
|---|---|---|---|---|---|---|---|
| Example 9-1 | Compound epi-6i-1 | - | Dichloromethane/hexane | - | 11.7^{*1} | 89.7, 0.13 | 89 |
| Example 9-2 | Compound | - | Dichloromethane/hexane | - | 12.0^{*1} | 75.5, 0.11 | 74 |
| Example 9-3 | Compound epi-6o-1 | - | - | Hexane | 15.6^{*1} | 73.0, 0.13 | 90 |
| Example 9-4 | Compound epi-6o-2 | - | - | Hexane | 12.1^{*1} | 70.3, 0.13 | 86 |
| Comparative Example 1-1 | Compound S3 | Hexane:ethyl acetate = 90:10-20:80 | - | - | - | 557.4, 1.08 | >99 |
| Comparative Example 1-2 | Compound S5 | Dichloromethane:methanol = 98:2-80:20 | - | - | - | 229.2, 0.78 | 85 |
| Comparative Example 1-3 | Compound epi-6a-1 | Dichloromethane:methanol = 99:1-00:10 | Dichloromethane/hexane | - | 13.1^{*2} | 377.3, 0.57 | 83 |
| Comparative Example 2-1 | Compound S4 | Hexane:ethyl acetate = 90:10-20:80 | - | - | - | 557.4, 1.08 | >99 |
| Comparative Example 2-2 | Compound S6 | Dichloromethane:methanol = 98:2-80:20 | - | - | - | 229.2, 0.78 | 85 |
| Comparative Example 2-3 | Compound epi-6a-2 | Dichloromethane:methanol = 99:1-00:10 | - | - | 10.9^{*2} | 453.8, 0.69 | 97 |

| | | | | | | | |
|---|---|---|---|---|---|---|---|
| ^{*1} Column: DAICEL CHIRALPAK IB 4.6 mm x 25 cm, solvent 10% isopropyl alcohol in hexane, flow rate: 1.0 mL/min, detection wavelength: 254 nm. ^{*2} Column: DAICEL CHIRALPAK IH 4.6 mm x 25 cm, solvent 10% isopropyl alcohol in hexane, flow rate: 1.0 mL/min, detection wavelength: 254 nm. | | | | | | | |

Sequence Listing

## Claims

1. A method for producing a two-residue-extended peptide represented by formula (6): 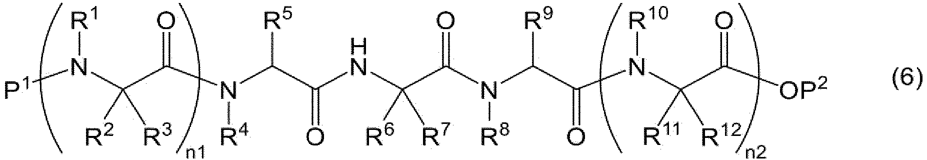 wherein R¹ represents a hydrogen atom or an alkyl group; R⁴, R⁸, and R¹⁰ are the same or different and each represents a hydrogen atom or a methyl group; R², R⁵, R⁶, R⁹, and R¹¹ are the same or different and each represents a side chain of an amino acid residue; R², R⁵, R⁶, R⁹, and R¹¹ each optionally form a ring together with the adjacent nitrogen atom through the carbon atom to which each is attached; R³, R⁷, and R¹² are the same or different and each represents a hydrogen atom or an optionally substituted monovalent organic group; P¹ represents a hydrogen atom, a protecting group for amino groups, a tag, or a solid-phase; P² represents a hydrogen atom, a protecting group for carboxy groups, a tag, or a solid-phase; n1 represents an integer of 0 or more, and when n1 is an integer of 2 or more, n1 R¹s, R²s, and R³s are optionally the same or different; and n2 represents an integer of 0 or more, and when n2 is an integer of 2 or more, n2 R¹⁰s, R¹¹s, and R¹²s are optionally the same or different;
the method comprising a reaction step of reacting a first amino acid or peptide represented by formula (1): 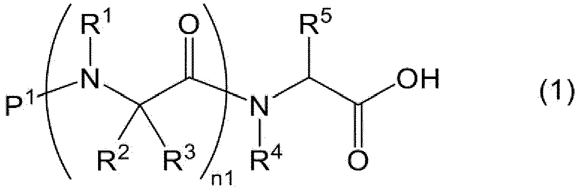
wherein R¹, R², R³, R⁴, R⁵, P¹, and n1 are as defined above,
a halogenating agent, a tertiary amine represented by formula (3) : 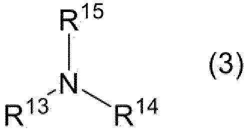
wherein R¹³, R¹⁴, and R¹⁵ are the same or different and each represents an optionally substituted alkyl group, an optionally substituted aryl group, or an optionally substituted heteroaryl group, or two or more of R¹³, R¹⁴, and R¹⁵ are optionally linked to form a ring optionally having one or more heteroatoms or substituents,
an amino acid N-carboxy anhydride (NCA) represented by formula (4): 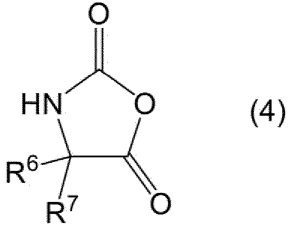
wherein R⁶ and R⁷ are as defined above, and
a second amino acid or peptide represented by formula (5): 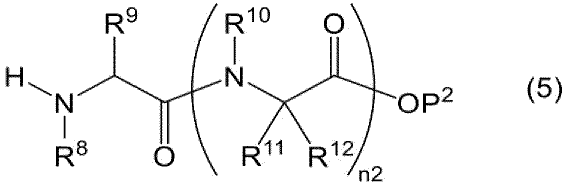
wherein R⁸, R⁹, R¹⁰, R¹¹, R¹², P², and n2 are as defined above.

2. The production method according to claim 1, wherein the reaction step comprises:
(A) a reaction step of reacting the first amino acid or peptide represented by formula (1), the halogenating agent, the tertiary amine represented by formula (3), and the amino acid N-carboxy anhydride (NCA) represented by formula (4); and
(B) a reaction step of reacting a reaction mixture obtained in step (A) and the second amino acid or peptide represented by formula (5).

3. The production method according to claim 2, wherein step (A) comprises:
(A1) a reaction step of reacting the first amino acid or peptide represented by formula (1), the halogenating agent, and the tertiary amine represented by formula (3); and
(A2) a reaction step of reacting a reaction mixture obtained in step (A1) and the amino acid N-carboxy anhydride (NCA) represented by formula (4).

4. The production method according to claim 1, wherein the halogenating agent is thionyl represented by formula (2): 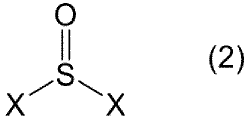 wherein Xs are the same or different and each represents a halogen atom.

5. The production method according to claim 1, wherein the tertiary amine comprises an aliphatic amine and/or a heterocyclic aromatic amine.

6. The production method according to claim 1, wherein the reaction temperature of the reaction step is -80 to 100°C.

7. The production method according to claim 1, wherein the reaction time of the reaction step is less than 60 minutes.

8. The production method according to any one of claims 1 to 7, wherein the reaction step is performed by a flow method.

9. The production method according to claim 8, wherein the reaction step is performed by a micro-flow method.

10. An acylated NCA represented by formula (8): 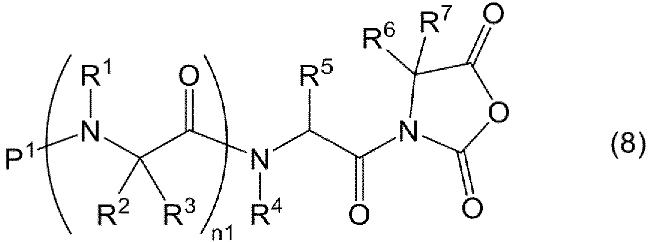 wherein R¹ represents a hydrogen atom or an optionally substituted monovalent organic group; R⁴ represents a hydrogen atom or a methyl group; R², R⁵, and R⁶ are the same or different and each represents a side chain of an amino acid residue; R², R⁵, and R⁶ each optionally form a ring together with the adjacent nitrogen atom through the carbon atom to which each is attached; R³ and R⁷ each represent a hydrogen atom or an optionally substituted monovalent organic group; P¹ represents a hydrogen atom, a protecting group for amino groups, a tag, or a solid-phase; and n1 represents an integer of 0 or more, and when n1 is an integer of 2 or more, n1 R¹s to R³s are optionally the same or different.

11. A peptide represented by formula (9): 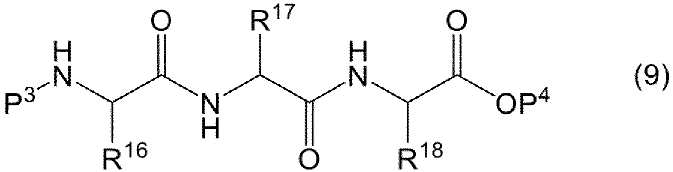 wherein R¹⁶, R¹⁷, and R¹⁸ are the same or different and each represents a side chain of an amino acid residue; R¹⁶, R¹⁷, and R¹⁸ each optionally form a ring together with the adjacent nitrogen atom through the carbon atom to which each is attached; P³ represents a hydrogen atom or a protecting group for amino groups; and P⁴ represents a hydrogen atom or a protecting group for carboxy groups.

12. A peptide consisting of an amino acid sequence represented by SEQ ID NO: 1.
